(19) Europäisches Patentamt
European Patent Office
Office européen des brevets

(11) EP 4 081 655 B1

(12) EUROPEAN PATENT SPECIFICATION

(45) Date of publication and mention of the grant of the patent:
**18.03.2026 Bulletin 2026/12**

(21) Application number: **20841720.4**

(22) Date of filing: **24.12.2020**

(51) International Patent Classification (IPC):
***C12Q 1/6806*** *(2018.01)*

(52) Cooperative Patent Classification (CPC):
(C-Sets available)
**C12Q 1/6806** (Cont.)

(86) International application number:
**PCT/EP2020/087851**

(87) International publication number:
**WO 2021/130356 (01.07.2021 Gazette 2021/26)**

# (54) DISEASE DETECTION IN LIQUID BIOPSIES

KRANKHEITSNACHWEIS IN FLÜSSIGEN BIOPSIEN

DÉTECTION DE MALADIES DANS DES BIOPSIES LIQUIDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.12.2019 EP 19219656**

(43) Date of publication of application:
**02.11.2022 Bulletin 2022/44**

(73) Proprietors:
- **VIB VZW**
  **9052 Gent (BE)**
- **Katholieke Universiteit Leuven, K.U.Leuven R&D**
  **3000 Leuven (BE)**

(72) Inventors:
- **LAMBRECHTS, Diether**
  **3010 Kessel-Lo (BE)**
- **VENKEN, Tom**
  **3201 Langdorp (BE)**
- **TIMMERMAN, Dirk**
  **3000 Leuven (BE)**
- **COOSEMANS, An**
  **3000 Leuven (BE)**

(56) References cited:
**WO-A1-2017/181146**
**WO-A1-2018/009723**
**US-A1- 2018 149 636**
**US-A1- 2019 287 645**
**US-A1- 2019 352 695**

- **CHAN LANDON L ET AL: "Bioinformatics analysis of circulating cell-free DNA sequencing data", CLINICAL BIOCHEMISTRY, ELSEVIER, AMSTERDAM, NL, vol. 48, no. 15, 9 May 2015 (2015-05-09), pages 962 - 975, XP029291458, ISSN: 0009-9120, DOI: 10.1016/J.CLINBIOCHEM.2015.04.022**
- **CHIANG-CHING HUANG ET AL: "Bioinformatics Analysis for Circulating Cell-Free DNA in Cancer", CANCERS, vol. 11, no. 6, 11 June 2019 (2019-06-11), pages 805, XP055697060, DOI: 10.3390/cancers11060805**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

(Cont. next page)

(52) Cooperative Patent Classification (CPC): (Cont.)

C-Sets
**C12Q 1/6806, C12Q 2522/10, C12Q 2523/125, C12Q 2537/16, C12Q 2537/164, C12Q 2537/165**

## Description

### FIELD OF THE INVENTION

Described are methods of analyzing cell free DNA based on combining analysis of cfDNA methylation with analysis of the cfDNA nucleosome footprint and methods of analyzing cell free DNA based on combining analysis of cfDNA methylation with analysis of the cfDNA nucleosome footprint and with analysis of cfDNA copy number alteration. The diagnostic performance of these methods, in particular relating to early or earlier stage diseases or disorders, is increased compared to the diagnostic performance of the individual cfDNA analysis methods.

### BACKGROUND

It is clear that early detection of a disease or disorder is beneficial as it reduces treatment complexity and improves treatment success and survival rates of the subjects having the disease or disorder. This is for instance the case when the disease or disorder is cancer (e.g. https://ascopost.com/News/59711 about "World Cancer Day 2019: Emphasis on Early Detection"). Specifically relating to ovarian cancer: over 75% of ovarian cancers are diagnosed only at stage III (51%) and IV (29%), resulting in 5-year survival rate of less than 30%. Five-year survival rates increase to 70% when ovarian cancer is detected at stage II, and to 90% when ovarian cancer is detected at stage I (Nebgen et al. 2019, Curr Oncol Rep 21:75). An ongoing trend in the cancer diagnostics field thus is to push the detection to earlier time points, even to time points where a subject or its general practitioner or physician are not even aware of the onset of a tumorous event in the subject's body. Sampling tumor tissue in such cases is not done, and may even not be possible due to the small size of the tumorous event. Research in this field is now starting to focus on analysis of liquid biopsies. Liquid biopsies contain minute amounts of cell-free or circulating free DNA (cfDNA), a fraction of which originating from a tumor (circulating tumor DNA or ctDNA) when present in a subject's body. The advantages of liquid biopsies versus tissue biopsies are listed in e.g. Table 1 of Tuaeva et al. 2019 (Cells 8:1251). Detection methods, and diagnostic kits based on such methods, thus must technically provide for very low detection limits in order to be able to catch a tumorous event at an early or very early stage. Such early detection methods, and diagnostic kits based on such methods, are also being applied to detection of other conditions (e.g. acute or chronic tissue damage, inflammatory disorders, autoimmune disorders), are being applied to monitor the fate or function of transplanted organs, or could be implemented in order to improve sensitivity of non-invasive prenatal tests (NIPTs).

A further problem with biopsies is that these may not provide sufficient information on the spatial and temporal heterogeneity present in a disease or disorder. Furthermore, due to comorbidities associated with obtaining biopsies, sequential biopsy sampling is difficult to implement in clinical practice. Also in these settings, liquid biopsies/cfDNA/ctDNA analysis offers a non-invasive alternative for monitoring the course of a disease or disorder.

Low concentrations of cfDNA are present in plasma of healthy individuals in the form of short double-stranded DNA fragments; 70-90% of this cfDNA is derived from leukocytes, while the remaining amounts originate from several other organs, such as the liver (Sun et al. 2015, PNAS 112:E5503-E5512). In cancer patients, a highly variable percentage of cfDNA originates from the tumor. Previous efforts to characterize this tumor-specific fraction (ctDNA) have focused on the detection of somatic mutations and copy number alterations (CNAs). However, this approach often requires *a priori* knowledge of the mutation spectrum of the tumor or is limited to the detection of tumors characterized by a certain degree of chromosomal instability (e.g. WO2017009372; WO2018204657; Vanderstichele et al. 2016, Clin Cancer Res 23:2223-2231). Other efforts of detecting ctDNA have focused on epigenetic features of cfDNA. Tumor-specific patterns of DNA methylation have, for instance, been used to identify which tissues or cell types are contributing to the cfDNA fraction (Sun et al. 2015, PNAS 112:E5503-E5512; Guo et al. 2017, Nat Genet 49:635-642; Li et al. 2018, Nucleic Acids Res 46:e89; Kang et al. 2017, Genome Biol 18:53; WO2019006269; Widschwendter et al. 2017, Genome Medicine 9:116; WO2018109212; WO2018109217; WO2017048932; WO2019068082; WO2019071161). Other approaches used whole-genome sequencing (WGS) of cfDNA to locate nucleosome positions, their occupancy and spacing in the cfDNA. It is hypothesized that the DNA at the sites of the nucleosomes in apoptotic cells is protected at least to some extent against degradation by nucleases and that by analysing WGS data the location of the nucleosomes can be determined. As a result of these nucleases, the average size of cfDNA is 167 bps, which corresponds to the length of a DNA fragment wrapped around a histone core (the nucleosome, ±147 bps) and its H1 linker histone (±20 bps). Further, since the genomic distribution of nucleosomes is considered to be cell-type specific, mapping of cell type-specific nucleosome positions can be used to assess which tissues are contributing to cfDNA. The first evidence for this came from studies focusing on the size distribution of cfDNA fragments using WGS (Lo et al. 2010, Sci Transl Med 2: 61ra91; Jiang et al. 2016, Trends Genet 32:360-371). Building on these findings, Snyder et al. 2016 (Cell 164:57-68; WO2016015058; WO2017012592) demonstrated how spacing between nucleosomes can be leveraged to identify the tissue-of-origin of cfDNA. Cristiano et al. 2019 (Nature 570:385-389) used counts of short and long fragments in 5 Mbp windows to estimate the tissue of origin. Ulz et al. 2016 (Nat Genet 48: 1273-1278) analyzed the sequencing depth at transcription start sites in cfDNA to infer tumor-specific gene expression (nucleosome promoter analysis). Straver et al. 2016 (Prenat Diagn 36:614-621) used genome-wide deviations from expected nucleosome positions to quantify the percentage of fetal DNA (independent of fetal sex) in plasma of pregnant women, without referring to any further diagnostic application. In the latter, it is noted that the nucleosome lengths in fetal and maternal cfDNA differ (147 vs 167 bp). In general, it is perceived that determining plasma fetal DNA size profiles is less challenging than determining ctDNA size profiles as the latter is less readily distinguishable from background cfDNA size profiles (Jiang & Lo 2016, Trends Genet 32:360-371). WO2017/181146 describes a method of analyzing cfDNA, the method relying on detection of a tumor marker present in at least 80% of subjects having cancer and wherein such tumor marker is represented in a gene sequencing panel no greater than 50k nucleotides, and the method further adapted to be able to detect the tumor marker when present at a frequency of as low as 0.01% in the cfDNA. This method thus is focusing on tumor markers present in a limited set of genes, and is biased towards known tumor markers (present in at least 80% of subjects with a cancer).

US2018149636 discloses a method for detection of cancer by analyzing methylation levels of cfDNA from urinary samples and in some instances of cfDNA from blood, possibly in combination with copy number analysis.

Although a field moving forward on a fast pace, there is still the need to increase the power of liquid biopsy testing.

## SUMMARY OF THE INVENTION

The invention is set out in the appended set of claims.

In one aspect, the invention relates to a method or methods for analyzing cell free DNA (cfDNA), the method comprising:

- obtaining cell-free DNA (cfDNA) from a biological sample obtained from a subject;
- analyzing the presence of DNA methylation in the obtained cfDNA;
  and:
  - analyzing a nucleosome footprint of the obtained cfDNA;
    and, in some embodiments:
    - analyzing copy number alteration (CNA) in the obtained cfDNA.

Such method or methods more in particular may be a method or methods for diagnosing a disease or disorder in a subject, for detecting the presence of a disease or disorder in a subject, for early detection of a disease or disorder in a subject, for early diagnosis of a disease or disorder in a subject, for screening for the presence of a disease or disorder in a subject, for determining an increased likelihood for a disease or disorder to be present in a subject, for monitoring a disease or disorder in a subject, for determining a response of a disease or disorder to therapy, for monitoring a disease or disorder after therapy, or for predicting a response of a disease or disorder to therapy.

## DESCRIPTION TO THE FIGURES

**FIGURE 1. Nucleosome footprint in paired-end and single-end cfDNA sequencing data.**
**A,** The insert size distributions of 3 plasma samples sequenced at high coverage using paired-end sequencing data shows fragment lengths centered on the size of nucleosome-bound DNA. **B,** Coverage and large window protection score (L-WPS) score (as defined by Snyder et al.2016, Cell 164:57-68); same genomic region is displayed) based on paired-end sequencing data of one plasma sample, illustrating specific positioning of nucleosomes and their footprint in plasma cfDNA. **C,** In single-end sequencing data, it is expected that mapped reads will tend to start (dots) at the boundaries of nucleosomes. **D,** When constructing a genome-wide distribution of the distances between all read start positions and the centers of the nearest expected nucleosomes as derived from a reference experiment in healthy individuals, the result is an M-shaped distribution with an enrichment of read starts at the edges of nucleosomes and a depletion at the centers of nucleosomes. The distributions shown here are derived from cfDNA samples of 125 healthy individuals and 43 patients with relapsed HGSOC. Compared to healthy individuals (thick line), plasma samples of relapsed HGSOC patients (thin lines deviating from the thick line) show a reduced enrichment of read starts at the nucleosome edges and a reduced depletion at nucleosome centers.

**FIGURE 2. Distribution of nucleosome scores (A) and genome-wide z-scores (B), according to FIGO stage and histology.** Scores are shown for 130 patients with benign ovarian disease, 41 patients with borderline ovarian tumors (BOT), 92 patients with invasive ovarian carcinoma according to FIGO stage (Invasive, I-IIa, IIb-IV) and 8 patients with adnexal metastases of other primary cancers. HGSOC cases are highlighted in red. The axis of the genome-wide z-scores was truncated for visualization purposes. Further descriptive statistics are detailed in Figures 5 and 6.

**FIGURE 3. ROC analysis nucleosome scores and genome-wide z-scores.**

ROC curves for nucleosome scores ("nucl.") and genome-wide z-scores ("gw-z") to discriminate patients with benign ovarian disease (n=130) from patients with borderline (BOT) and invasive carcinoma (n=141, including 8 metastases; first row); patients with invasive carcinoma (n=100; second row); patients with HGSOC disease (n=62; third row); patients with non-HGSOC disease (n=30; fourth row). ROC curves for nucleosome and genome-wide z-scores were then combined in a single predictor and the optimism-corrected AUC value was calculated (second column).

**FIGURE 4. Characteristics of non-HGSOC cases, nucleosome and genome-wide z-scores.**

**A,** Correlation between nucleosome and genome-wide z-scores for all invasive tumor samples (including 8 metastasis samples), HGSOC and non-HGSOC samples. **B,** Fraction of the genome that is not copy-neutral for a HGSOC26 and non-HGSOC cohort. **C, D,** Illustrations of genomic representation profiles obtained from baseline fresh-frozen tumor tissue for 3 non-HGSOC samples (LGSOC, MUCOC and NEOC) and for 3 HGSOC samples.

**FIGURE 5. Table depicting genome-wide z-scores according to stage and histology.**

**FIGURE 6. Table depicting nucleosome scores according to stage and histology.**

**FIGURE 7. Overview of training set and test set in the determination of DNA methylation sites in cfDNA of ovarian cancer samples.**

**FIGURE 8. Overview of the methylation bioinformatics pipeline to calculate a methylation score of a plasma sample of interest, based on the methylation patterns found in sequencing reads.**

**FIGURE 9. Distribution of the methylation scores according to sample type.**

**FIGURE 10. ROC curves based on cfDNA analysis from benign versus invasive ovarian cancer plasma samples.** Left panel: Individual curves for each metric: nucleosome score (nucl.), genome-wide z-score (gw-z) and methylation score (meth.). Right panel: combination of the three scores with an optimism-corrected AUC value using logistic regression.

**FIGURE 11.** Overview of expanded sample set.

**FIGURE 12.** Overview of expanded training set and test set.

**FIGURE 13.** Distribution of the methylation scores according to sample type (expanded sample set).

**FIGURE 14.** ROC curves of benign versus invasive plasma samples (expanded sample set). Left panel: Individual curves for each metric: nucleosome score (nucl.), genome-wide z-score (gw-z) and methylation score (meth.). Right panel: combination of the three scores with an optimism-corrected AUC value using logistic regression.

## DETAILED DESCRIPTION

The experimental work described hereinafter provided the foundation for early detection of diseases or disorders independent of specific markers. In particular, different analyses of cfDNA obtained from a biological sample of a subject were demonstrated to provide non-overlapping predictive value for the disease. Combination of these different analyses lead to increased accuracy in predicting the presence of the disease compared to the accuracy in predicting the presence of the disease by the individual analysis methods.

Two of the cfDNA analysis methods are based on a single/same set of low-coverage sequencing data of a subject's test cfDNA and comparison with a (healthy subject) reference, germline or control set of low-coverage genome-wide sequencing data. Knowledge of specific markers for any disease or disorder is not required as a deviation between the analyses of the two data sets (test vs control or reference) is indicative of the presence of a disorder or disease independent of such specific markers for any disease or disorder.

A third cfDNA analysis is based on methylation of a subset of cfDNA sequences obtained from a subject's test cfDNA (cell free methylome analysis) wherein the subset of cfDNA sequences covers a set of cfDNA sequences that are not methylated in healthy subjects; when thus analyzing the methylation, in a subject's test cfDNA sample, of the set of cfDNA sequences that are not methylated in healthy subjects, the presence of methylation is indicative of the presence of a disorder or disease, and knowledge of specific markers for any disease or disorder is not required.

The predictive power of different combinations of the three types of cfDNA analyses, including combinations of two out of the three, was determined. This revealed that, in order to increase the predictive power of any of the herein considered combinations of cfDNA analyses compared to the individual single types of cfDNA analysis, inclusion of cfDNA methylation analysis in the combination is required.

Although the experimental work described hereinafter focused on ovarian cancer, the above explanation plausibly supports general application of the combined cfDNA analyses as described, as can be gleaned from the analysis of cfDNA samples obtained from subjects with ovarian cancer originating from a non-ovarian primary tumor (metastasized to the ovaries). The analysis based on methylation of cfDNA obtained from ovarian cancer patients furthermore allowed expansion of the genome-wide set of cfDNA sequences that are not methylated in healthy subjects and selection of an improved set of ovarian cancer-specific cfDNA methylation markers. The identification of such a disease-specific set of cfDNA methylation markers allows the development of a detection method specific for a disease, or to define the origin (to define the diseased tissue or organ) of deviating methylation when analyzing, in a subject's test cfDNA sample, methylation of the genome-wide set of cfDNA sequences that are not methylated in healthy subjects.

The inventive concept is described in detail hereinafter, only the embodiments subject of the appended claims being part of the invention.

One general aspect of the invention therefore relates to a method or to methods for analyzing cell free DNA (cfDNA), the method comprising:

- obtaining, extracting, isolating or purifying cell-free DNA (cfDNA) from a biological sample obtained from a subject;
- analyzing, assaying, assessing, measuring or determining the presence of DNA methylation in the obtained, extracted, isolated or purified cfDNA;
  and:
  - analyzing, assaying, assessing, measuring or determining a nucleosome footprint of the obtained, extracted, isolated or purified cfDNA;

and, in some embodiments:

- analyzing, assaying, assessing, measuring or determining copy number alteration (CNA) in the obtained, extracted, isolated or purified cfDNA.

In one embodiment, such methods thus include analysis of DNA methylation and of the nucleosome footprint. In another embodiment, all three analysis methods (analysis of DNA methylation, nucleosome footprint and copy number alteration) are combined.

Concerning the step of analyzing, assaying, assessing, measuring or determining a nucleosome footprint and/or copy number alteration in such method or such methods, the nucleosome footprint can be a genome-wide nucleosome footprint and/or the copy number alteration can be a genome-wide copy number alteration.

As indicated above, analyzing, assaying, assessing, measuring or determining the presence of DNA methylation in the cfDNA can in one embodiment be based on a genome-wide set of cfDNA sequences that are lowly methylated, nearly unmethylated or not methylated in (cfDNA of) healthy subjects, such as on a genome-wide set of CpGs or CpG clusters with a mean average methylation $\beta$-value of less than 0.03 in (cfDNA of) healthy individuals (as outlined in Example 2.2 herein).

In another embodiment, the cfDNA methylation is not analyzed, assayed, assessed, measured or determined on a genome-wide scale or is not an analysis on a genome-wide scale or is not a genome-wide analysis. As exemplified herein, cfDNA methylation analysis on a set of CpGs or on a set of CpG clusters is sufficient for increased accuracy in predicting the presence of a disease when combined with nucleosome footprint analysis and/or copy number alteration analysis, in particular when combined with genome-wide nucleosome footprint analysis and genome-wide copy number alteration analysis. In particular, as described in Example 2.2 herein, the set of CpGs or set of CpG clusters comprises CpGs or CpG clusters that are lowly methylated or nearly unmethylated in healthy individuals, more particularly in cfDNA of healthy individuals. Furthermore in particular, as described in Example 2.2 herein, the set of CpGs or set of CpG clusters comprises CpGs or CpG clusters with a mean average methylation $\beta$-value of less than 0.03 in healthy individuals, more particularly in cfDNA of healthy individuals. In very specific cases, the set of CpGs or CpG clusters may be limited further such as to arrive at a minimal set of CpGs or CpG clusters being specific to a certain disease. In particular, the number of CpGs in a set of CpGs (or in a minimal set of disease-specific CpGs) may be from 2 to 45000 CpGs, from 2 to 40000 CpGs, from 2 to 30000 cpGs, from 2 to 20000 CpGs, from 2 to 10000 CpGs, from 2 to 7500 CpGs, from 2 to 5000 CpGs, from 2 to 2500 CpGs, from 2 to 2000 CpGs, from 2 to 1500 CpGs, from 2 to 1000 CpGs, from 2 to 500 CpGs, from 2 to 250 CpGs, at most 45000 CpGs, at most 40000 CpGs, at most 30000 CpGs, at most 20000 CpGs, at most 10000 CpGs, at most 5000 CpGs, at most 4000 CpGs, at most 3000 CpGs, at most 2000 CpGs, at most 1500 CpGs, at most 1000 CpGs, at most 900 CpGs, at most 800 CpGs, at most 700 CpGs, at most 600 CpGs, at most 500 CpGs, at most 400 CpGs, at most 300 CpGs, at most 200 CpGs, at most 190 CpGs, at most 180 CpGs, at most 170 CpGs, at most 160 CpGs, at most 150 CpGs, at most 140 CpGs, at most 130 CpGs, at most 120 CpGs, at most 110 CpGs, at most 100 CpGs, at most 90 CpGs, at most 80 CpGs, at most 70 CpGs, at most 60 CpGs, at most 50 CpGs, at most 40 CpGs, at most 30 CpGs, at most 20 CpGs, or at most 10 CpGs.

In particular, the number of CpG clusters in a set of CpG clusters (or in a minimal set of disease-specific CpG clusters) may be from 1 to 30000 CpG clusters, from 1 to 25000 CpG clusters, from 1 to 20000 CpG clusters, from 1 to 15000 CpG clusters, from 1 to 10000 CpG clusters, from 1 to 5000 CpG clusters, from 1 to 2500 CpG clusters, from 1 to 1000 CpG clusters, from 1 to 500 CpG clusters, from 1 to 400 CpG clusters, from 1 to 300 CpG clusters, from 1 to 200 CpG clusters, from 1 to 150 CpG clusters, from 1 to 100 CpG clusters, from 1 to 75 CpG clusters, from 1 to 50 CpG clusters, from 1 to 25 CpG clusters, from 1 to 20 CpG clusters, from 1 to 15 CpG clusters, from 1 to 10 CpG clusters, from 1 to 5 CpG clusters, at most 30000 CpG clusters, at most 25000 CpG clusters, at most 20000 CpG clusters, at most 15000 CpG clusters, at most 10000 CpG clusters, at most 10000 CpG clusters, at most 5000 CpG clusters, at most 2500 CpG clusters, at most 1000 CpG clusters, at most 500 CpG clusters , at most 400 CpG clusters, at most 300 CpG clusters, at most 200 CpG clusters, at most 190 CpG clusters, at most 180 CpG clusters, at most 170 CpG clusters, at most 160 CpG clusters, at most 150 CpG clusters, at most 140 CpG clusters, at most 130 CpG clusters, at most 120 CpG clusters, at most 110 CpG clusters , at most 100 CpG clusters, at most 90 CpG clusters, at most 80 CpG clusters , at most 70 CpG clusters , at most 60 CpG clusters, at most 50 CpG clusters, at most 40 CpG clusters, at most 30 CpG clusters, at most 20 CpG clusters, at most 10 CpG clusters, or at most 5 CpG clusters.

As indicated above, some methods include analysis of DNA methylation of the cfDNA and of the nucleosome footprint of the cfDNA.

Herein, in one embodiment, the analysis of the nucleosome footprint of the cfDNA may be genome-wide analysis, in particular such as genome-wide analysis based on low-coverage whole genome sequencing. Herein, in a further embodiment, the analysis of DNA methylation of the cfDNA in particular may be non-genome-wide analysis (such as on a set of CpGs or CpG clusters with a mean average methylation β-value of less than 0.03 in healthy individuals or subjects as described above; or such as on a subset of such CpGs or CpG clusters).

In a further embodiment, the analysis of DNA methylation of the cfDNA in particular may be non-genome-wide analysis (such as on a set of CpGs or CpG clusters with a mean average methylation β-value of less than 0.03 in healthy individuals or subjects as described above; or such as on a subset of such CpGs or CpG clusters) and the analysis of the nucleosome footprint of the cfDNA may be genome-wide analysis (such as analysis based on low-coverage whole genome sequencing data).

As indicated above, some methods include analysis of DNA methylation of the cfDNA and of the copy number alteration in the cfDNA.

Herein, in one embodiment, the analysis of the copy number alteration in the cfDNA may be genome-wide analysis, in particular such as genome-wide analysis based on low-coverage whole genome sequencing.

Herein, in a further embodiment, the analysis of DNA methylation of the cfDNA in particular may be non-genome-wide analysis (such as on a set of CpGs or CpG clusters with a mean average methylation β-value of less than 0.03 in healthy individuals or subjects as described above; or such as on a subset of such CpGs or CpG clusters).

In a further embodiment, the analysis of DNA methylation of the cfDNA in particular may be non-genome-wide analysis (such as on a set of CpGs or CpG clusters with a mean average methylation β-value of less than 0.03 in healthy individuals or subjects as described above; or such as on a subset of such CpGs or CpG clusters) and the analysis of the copy number alteration in the cfDNA may be genome-wide analysis (such as analysis based on low-coverage whole genome sequencing data).

As indicated above, some methods combine all three cfDNA analysis methods (analysis of DNA methylation, nucleosome footprint and copy number alteration).

Herein, in one embodiment, the analysis of the nucleosome footprint of the cfDNA may be genome-wide analysis, in particular such as genome-wide analysis based on low-coverage whole genome sequencing. Herein, in a further embodiment, the analysis of the copy number alteration in the cfDNA may be genome-wide analysis, in particular such as genome-wide analysis based on low-coverage whole genome sequencing.

Herein, in a further embodiment, the analysis of DNA methylation of the cfDNA in particular may be non-genome-wide analysis (such as on a set of CpGs or CpG clusters with a mean average methylation β-value of less than 0.03 in healthy individuals or subjects as described above; or such as on a subset of such CpGs or CpG clusters).

In a further embodiment, the analysis of the nucleosome footprint of the cfDNA and of the copy number alteration in the cfDNA may be genome-wide analysis, in particular such as genome-wide analysis based on low-coverage whole genome sequencing. More in particular the same genome-wide analysis data may be used both to analyse the nucleosome footprint of the cfDNA and to analyze the copy number alteration in the cfDNA.

In a further embodiment, the analysis of DNA methylation of the cfDNA in particular may be non-genome-wide analysis (such as on a set of CpGs or CpG clusters with a mean average methylation β-value of less than 0.03 in healthy individuals or subjects as described above; or such as on a subset of such CpGs or CpG clusters) and the analysis of the nucleosome footprint of the cfDNA may be genome-wide analysis (such as analysis based on low-coverage whole genome sequencing data).

In a further embodiment, the analysis of DNA methylation of the cfDNA in particular may be non-genome-wide analysis (such as on a set of CpGs or CpG clusters with a mean average methylation β-value of less than 0.03 in healthy individuals or subjects as described above; or such as on a subset of such CpGs or CpG clusters) and the analysis of the copy number alteration in the cfDNA may be genome-wide analysis (such as analysis based on low-coverage whole genome sequencing data).

In a further embodiment, the analysis of DNA methylation of the cfDNA in particular may be non-genome-wide analysis (such as on a set of CpGs or CpG clusters with a mean average methylation β-value of less than 0.03 in healthy individuals or subjects as described above; or such as on a subset of such CpGs or CpG clusters), and the analysis of the nucleosome footprint of and copy number alteration in the cfDNA may be genome-wide analysis (such as analysis based on low-coverage whole genome sequencing data). More in particular the same genome-wide analysis data may be used both to analyse the nucleosome footprint of the cfDNA and to analyze the copy number alteration in the cfDNA.

In any of the above methods, the cfDNA is in one embodiment obtained, extracted, isolated or purified from a blood, plasma, or serum sample as obtained from a test subject, such as a mammal (such as a human) suspected of having cancer.

Thus, in one embodiment, methods including analysis of DNA methylation of the cfDNA and of the nucleosome footprint of the cfDNA rely on cfDNA obtained, extracted, isolated or purified from a blood, plasma, or serum sample as obtained from a test subject such as a mammal (such as a human) suspected of having cancer. Herein,
analysis of DNA methylation of the cfDNA in particular may be non-genome-wide analysis (such as on a set of CpGs or CpG clusters with a mean average methylation β-value of less than 0.03 in healthy individuals or subjects as described above; or such as on a subset of such CpGs or CpG clusters) and the analysis of the nucleosome footprint of the cfDNA may be genome-wide analysis (such as analysis based on low-coverage whole genome sequencing data).

In a further embodiment, methods including analysis of DNA methylation of the cfDNA and of the copy number alteration in the cfDNA rely on cfDNA obtained, extracted, isolated or purified from a blood, plasma, or serum sample as obtained from a test subject such as a mammal (such as a human) suspected of having cancer. Herein, analysis of DNA methylation of the cfDNA in particular may be non-genome-wide analysis (such as on a set of CpGs or CpG clusters with a mean average methylation β-value of less than 0.03 in healthy individuals or subjects as described above; or such as on a subset of such CpGs or CpG clusters) and the analysis of the copy number alteration in the cfDNA may be genome-wide analysis (such as analysis based on low-coverage whole genome sequencing data).

In a further embodiment, all three cfDNA analysis methods (analysis of DNA methylation, nucleosome footprint and copy number alteration) are combined and rely on cfDNA obtained, extracted, isolated or purified from a blood, plasma, or serum sample as obtained from a test subject such as a mammal (such as a human) suspected of having cancer. Herein, analysis of DNA methylation of the cfDNA in particular may be non-genome-wide analysis (such as on a set of CpGs or CpG clusters with a mean average methylation β-value of less than 0.03 in healthy individuals or subjects as described above; or such as on a subset of such CpGs or CpG clusters), and the analysis of the nucleosome footprint of and copy number alteration in the cfDNA may be genome-wide analysis (such as analysis based on low-coverage whole genome sequencing data). More in particular the same genome-wide analysis data may be used both to analyse the nucleosome footprint of the cfDNA and to analyze the copy number alteration in the cfDNA.

In any of the above methods and their embodiments, a further step can be included wherein a sample cfDNA methylation score is calculated; the cfDNA methylation score is the DNA methylation score for the cfDNA on which the presence of DNA methylation has been analyzed, assayed, assessed, measured or determined.

In any of the above methods and their embodiments, a further step can be included wherein the sample cfDNA methylation score is compared with a reference cfDNA methylation score which is the DNA methylation score for reference cfDNA.

In one embodiment, such cfDNA methylation score is not determined, calculated or based on a genome-wide scale or is not an analysis on a genome-wide scale or is not a genome-wide analysis. As exemplified herein, a cfDNA methylation score calculated for a set of CpGs or for a set of CpG clusters is sufficient for increased accuracy in predicting the presence of a disease when combined with nucleosome footprint analysis and/or copy number alteration analysis, in particular when combined with genome-wide nucleosome footprint analysis and genome-wide copy number alteration analysis.

In any of the above methods and their embodiments, a further step can be included wherein a sample cfDNA nucleosome score is calculated which is the nucleosome score for the obtained, extracted, isolated or purified cfDNA; and/or wherein a sample cfDNA CNA score is calculated, which is the CNA score for the obtained, extracted, isolated or purified cfDNA.

In any of the above methods and their embodiments, a further step can be included wherein the sample cfDNA nucleosome score is compared with a reference cfDNA nucleosome score which is the corresponding nucleosome score for reference cfDNA;
and/or
wherein the sample cfDNA CNA score is compared with a reference cfDNA CNA score which is the corresponding CNA score for reference cfDNA.

In any of the above methods and their embodiments, a further step can be included wherein of a disease or disorder is determined likely to be present in the subject when the DNA methylation in the sample cfDNA is deviating or different from the DNA methylation of reference cfDNA or when the sample cfDNA methylation score is deviating or different from the reference cfDNA methylation score; and

when the sample cfDNA nucleosome score is deviating or different from the reference cfDNA nucleosome score;

and/or
when the sample cfDNA CNA score is deviating or different from the reference cfDNA CNA score.

In any of the above methods and their embodiments, a further step can be included wherein the sample cfDNA methylation score, the sample cfDNA nucleosome score and the sample cfDNA CNA score are combined in a sample cfDNA single score.
In any of the above methods and their embodiments, a further step can be included wherein a disease or disorder is determined likely to be present in the subject when the sample cfDNA single score is deviating or different from the reference cfDNA single score.
In any of the above methods and their embodiments, a further step can be included wherein, within the total amount of cfDNA obtained from the biological sample, the proportional amount of cell-free DNA associated with the disease or disorder is quantified, measured or determined. The quantification can be based on the DNA methylation analysis, nucleosome footprint analysis and/or the copy number alteration analysis of the sample cfDNA.
In any of the above methods and their embodiments, the obtained, extracted, isolated or purified cfDNA may not need to be amplified prior to or before any analysis step, or the obtained, extracted, isolated or purified cfDNA may be amplified prior to or before any analysis step. The obtained, extracted, isolated or purified cfDNA may likewise by aliquoted after which e.g. one aliquot is subjected to amplification prior to or before any analysis step.
Steps and terms relating to the above aspect and embodiments of the invention are explained in detail hereafter.

## DNA Methylation / CpGs / CpG clusters / DNA methylation score

DNA methylation as described herein is usually analyzed, assessed or determined on defined CpGs or on defined CpGs clusters.
The annotation "CpG" is an abbreviation for 5'-cytosine-phosphate-guanine-3'. Although the frequency of occurrence of CpGs in the human genome is less than 25% of the expected frequency, CpGs tend to cluster in "CpG islands". One possible definition of a CpG island refers to a region of at least 200 bp in length with a GC-content of more than 50%, and with an observed-to-expected CpG ratio of more than 60%. Herein the observed CpG obviously is the actual number of CpG occurrences within the delineated CpG island. The expected number of CpGs can be calculated as ([C]x [G])/sequence length (Gardiner-Garden et al. 1987, J Mol Biol 196:261-282) or as $(([C]+[G])/2)^2$/sequence length (Saxonov et al. 2006, PNAS 103:1412-1417), wherein [C] and [G] are the number of cytosines and guanines, respectively, in the delineated CpG island. As synonym for CpG island, especially if prone to changes in DNA methylation of one or more CpGs in the CpG island, reference is sometimes made to differentially methylated region or DMR.
"DNA methylation", in particular methylation on a (set of) CpG(s) or methylation of a (set of) CpGs, such as comprised in cfDNA, is the attachment of a methyl group to the cytosine located in a (set of) CpG dinucleotide(s), creating a (set of) 5-methylcytosine(s) (5mC). CpG dinucleotides (CpGs) tend to cluster in so-called CpG islands, and when they are methylated this often correlates with transcriptional silencing of the affected gene. DNA methylation represents a relatively stable but reversible epigenetic mark (Bachman et al. 2014, Nat Chem 6:1049-1055). Its removal can be initiated by ten-eleven translocation (TET) enzymes, which convert 5mC to 5-hydroxymethylcytosine (5hmC) in an oxygen-dependent manner (Williams et al. 2011, Nature 473:343-348). Recently, it was demonstrated that tumor hypoxia reduces TET activity, leading to the accumulation of 5mC and loss of 5hmC (Thienpont et al. 2016, Nature 537:63-68). Assays for determining, detecting, measuring, assessing or assaying DNA methylation as well as methodologies for scoring DNA methylation levels (and changes therein) will be discussed in more detail further herein.
The methylation status of DNA or cfDNA can be determined for a series of adjacent CpG sites, together forming a CpG block or CpG cluster; these blocks or clusters of adjacent CpG sites present in DNA or cfDNA can thus be the targets for determining the methylation status, and are sometimes referred to herein as (DNA) methylation blocks or (DNA) methylation clusters. When the series of adjacent CpGs are jointly methylated (all methylated, all adjacent CpGs are methylated), then the methylation block or methylation cluster can be referred to as methylated CpG block or methylated CpG cluster. Alternatively, the average methylation of the CpGs in a CpG block or CpG cluster in test cfDNA can be higher than the average methylation of the CpGs in a CpG block or CpG cluster in control or reference cfDNA. Hypermethylation of a (cf)DNA region or of a CpG or CpG cluster can refer either to an increased frequency of methylation of the analyzed DNA or to an increased average methylation of the analyzed DNA, compared to a reference DNA. It can alternatively refer to an increased frequency of occurrence of the methylated analyzed DNA. The latter can be relevant in view of the CpG clusters of the type that are selected to be fully methylated; hypermethylation of such CpG cluster then can refer to the increased proportion of such fully methylated CpG cluster versus the same not fully (hypo-) or non-methylated CpG cluster in the same sample, this compared to the proportion of such fully methylated CpG cluster versus the same not fully (hypo-) or non-methylated CpG cluster in a reference sample.
A DNA methylation score is a numerical value providing information on the DNA methylation status of the CpG or CpGs on which DNA methylation was determined; the numerical value is resulting from a numerical calculation, such as by a computerized numerical calculation or numerical calculation performed by a computer system. Alternative numerical calculation methods are possible, of importance is that the same numerical calculation method is used when comparing DNA methylation scores of a test sample and a reference sample.

CpGs or CpG clusters as referred to herein are defined by their chromosomal location. Retrieving the actual nucleic acid sequence context of the indicated CpGs or CpG clusters on the indicated chromosome is known to the skilled person, and such actual nucleic acid sequence context can be retrieved e.g. by using a genome browser (e.g. https://genome.ucsc.edu/ or https://www.ncbi.nlm.nih.gov/genome/) for searching a reference genome (sequence of all of the chromosomes of a species), in particular a human reference genome. In the work described herein, the February 2009 human reference sequence GRCh37 (also referred to as GRCh37/hg19 or as GRCh37.p13) produced by the Genome Reference Consortium) was relied on to localize or delineate the CpGs of interest as indicated in Tables 3, 4, 6, 7 and 8 herein (see Examples). Table 4 herein is providing an overview of CpG clusters of interest. When taking as arbitrary example CpG cluster #5 listed therein, it is annotated therein as residing on chromosome 1 with a start and end-1 position of 16488977 and 16489215, respectively, on that chromosome. When searching GRCh37/hg19 via https://www.ncbi.nlm.nih.gov/genome/ for the sequence on chromosome 1 starting on position chr1: 16488971 and ending on position 16489220, in short "chr1:16488971-16489220", this yields the below-depicted nucleotide sequence in which the individual CpGs are indicated in highlighted characters:
chr1:16488971 (referred to as "8971" in the sequence below) - 16489220 (referred to as "9220" in the sequence below); CpGs are shadowed:

```
8971 AGGCTGCGCC ACTTCCTTTG GGGCTCGCTC GCGCTTCCTG GGAGCCTTTG ATGTGGGTTC
9031 TTGGTAGATC CTGCCCGCGG GGCCTGGATG CGGGCGGAGC CTCCGGGCAG GGCGGGGCCT
9091 CAGCCTGGGT CCGCACTGGG GAGGCGGCCA CCCGGTAGCT TGGGCGGGTT CCGCCCACCT
9151 CCCGCCTGTA TTTACAGCCC CCTTCCAACA TCCCCGACTC CCTGAGGGCG CCCCTCATTC
9211 TGCTCGGCCT 9220      (SEQ ID NO:1)
```

The above thus illustrates how all individual CpGs and CpG clusters referred to herein are defined and how their sequence context can be retrieved.

## Nucleosome footprinting / nucleosome score

The terms nucleosome map, nucleosome distribution, nucleosome occupancy or nucleosome footprint are interchangeable herein.

The nucleosome footprinting approach as relied on herein is starting from the premise of differential positioning of DNA in nucleosomes (resulting in differential DNA fragmentation when shed from a cell) depending on the tissue of origin and/or depending on the presence of a disease, disorder or (pre- )pathological condition in a subject. The presence of differential nucleosome footprints in cfDNA is likely to be proportional to the amount of diseased tissue giving rise to the differential nucleosome footprints. The nucleosome footprinting approach as relied on herein is starting from sequencing reads obtained from cfDNA isolated from a liquid biopsy sample of a subject. The same sequencing reads as obtained for copy number alteration analysis on the cfDNA can be used for nucleosome footprinting analysis described hereinabove, i.e. sequencing reads obtained by low-coverage whole genome sequencing (although higher coverage whole genome sequencing, up to very deep whole genome sequencing is not excluded). In general terms, sequencing reads are positioned on a reference or control genome-wide map of nucleosome-protected cfDNA. More in particular, an essentially saturated genome-wide nucleosome map as determined by Snyder et al. 2016 (Cell 164:57-68)/WO2016015058 was used herein as reference or control genome-wide nucleosome map (comprising nearly 13 million in vivo nucleosomes spread over the whole human genome). The obtained sequencing reads were mapped to their corresponding reference or control nucleosomes and the distances between the ends of the obtained sequencing reads and the center of the nearest corresponding reference or control nucleosomes were calculated. Based on the frequency of occurrence of each end of each obtained sequencing read mapped to a corresponding reference or control nucleosome, a genome-wide distribution of distances (nucleosome end versus nucleosome center) can be constructed or calculated. The genome-wide distribution of such distances determined for cfDNA obtained from a subject (sample, subject's sample or test sample nucleosome footprint) can be compared to the genome-wide reference or control distribution of such distances (reference or control nucleosome footprint). Any deviation of the sample nucleosome footprint from the reference or control nucleosome footprint is indicative of the presence of cfDNA normally not occurring in a liquid biopsy sample of a healthy subject, and therewith indicative for the presence of a disease, disorder or (pre-)pathological condition in the subject.

The reference or control cfDNA nucleosome footprint is a cfDNA nucleosome footprint representative for a healthy or control subject. It can be based on cfDNA samples from one or more healthy or control subjects, or on cfDNA samples from germline material from one or more healthy subjects or subjects having the disease, disorder or (pre-) pathological condition of interest. Ideally, the reference or control cfDNA is of the same source as the source of the test cfDNA (e.g. blood, serum or plasma). Alternatively, the reference or control cfDNA is obtained from blood, serum or plasma, and the source of the test cfDNA is different.

Catalogs or databases of cfDNA nucleosome footprints may be compiled or constructed in a similar manner as for cfDNA chromosomal instability patterns.

In layman's terms the probability is determined whether or not a nucleosome footprint of a sample is more likely to be one associated with the presence of a disease or disorder or is more likely to be one associated with a normal background or with absence of a disease or disorder. In case of a likelihood of a nucleosome footprint to be associated with the presence of a disease or disorder (i.e. a likelihood substantially higher than the likelihood of association with a normal background or with (a background in the) absence of the disease or disorder), the subject is predicted to have the disease or disorder. In case of a likelihood of a nucleosome footprint to be associated with a normal background or with (a background in) the absence of a disease or disorder (i.e. a likelihood substantially lower than the likelihood of association with the presence of the disease or disorder), the subject is predicted to not have the disease or disorder.

A nucleosome score for a DNA is a numerical value providing information on the nucleosome distribution (footprint) within the analyzed DNA; the numerical value is resulting from a numerical calculation, such as by a computerized numerical calculation or numerical calculation performed by a computer system. Alternative numerical calculation methods are possible, of importance is that the same numerical calculation method is used when comparing nucleosome scores of a test sample and a reference sample. A genome-wide nucleosome score as used herein is perfectly suited to compress the complexity of large amounts of molecular data relating to genome-wide alterations into a biologically and clinically meaningful parameter highly increasing interpretability. When nucleosome catalogs as described above become available, it is expected that genome-wide nucleosome analysis will not be required and will optionally be replaced by a more focused sequencing effort (low coverage or higher coverage).

**Copy number alteration (CNA) / CNA score**

Generally, three distinct forms of genome instability are discerned: microsatellite instability (MSI: alterations in relatively short repetitive DNA sequences), CpG island methylator phenotype (CIMP: changes in methylation of gene promoter regions leading to modulation of gene transcription), and structural variation (SV) or chromosome instability (CIN).

Besides DNA inversions and translocations, chromosomal instability also relates to the rate or frequency in gains or losses of chromosomes or of parts of chromosomes. The latter can be detected in a sample by determining copy number alteration (CNA) or copy number variability (CNV). Chromosomal instability frequently occurs in several types of cancer (the so-called "C-class" cancer types including at least HGSOC, BLCA (bladder urothelial carcinoma), BRCA (breast carcinoma), HNSCC (head and neck squamous cell carcinoma), LUSC (lung squamous cell carcinoma) and UCEC (uterine carcinoma); Ciriello et al. 2013, Nat Genet 45:1127-1133; see also e.g. Fig 1e in Bowtell et al. 2015, Nat Rev Cancer 15:668-679). Traditionally, chromosomal instability is determined in cancer tissue or individual cancer cells (e.g. Lepage et al. 2019, Cancers 11: 226). Whereas Leary et al. 2012 (Sci Transl Med 4:162ra154) reported detection of chromosomal alterations in cfDNA from cancer patients by whole genome sequencing at a depth of 1-10x, a landmark study by Vanderstichele et al. 2017 (Clin Cancer Res 23:2223-2231), demonstrated that chromosomal instability can be determined reliably in cell-free DNA (cfDNA) by low-coverage (median read depth 0.12x) whole genome sequencing, this for ovarian cancer. The chromosomal instability patterns as can be determined in cfDNA correlated with the chromosomal instability patterns of tumor cells obtained from the same patients for which the cfDNA chromosomal instability pattern was determined. Furthermore, chromosomal instability in cell-free DNA has been evaluated in an early testing setting for lung cancer (Xia et al. 2015, Lung Cancer 90:78-84) and prostate cancer (Schutz et al. 2015, Clin Chem 61:239-248).

A particular strength of assessing chromosomal instability at the whole-genome level as performed herein on cfDNA is that this unbiased approach can be applied successfully to tumors or cancers without prior knowledge about the genetic architecture of the tumor or cancer. In other words, this approach is not limited to assessing pre-defined chromosomal instability events identified to occur frequently in a given tumor or cancer, or, alternatively is not relying on genotypic differences, more in particular not relying on genotypic differences specific for a given tumor or cancer type. It is also not limited to analysis of a panel of regions (such as taken from a plurality of different genes), i.e., no tumor- or cancer-specific marker panels have to be designed. It is further not limited by heterogeneity within a tumor or cancer type or by heterogeneity within a subject's tumor or cancer or a biopsy thereof. The lack of these limitations is of especial importance in an early detection setting in which neither the type of cancer nor the type of chromosomal instability is known. It is surprising that low-coverage whole genome sequencing is sufficient to identify chromosomal instability in cfDNA as it is generally accepted that sequencing depth is increasing with decreasing frequency of a marker of interest. Although low-coverage whole genome sequencing is sufficient in the above context, higher coverage whole genome sequencing, up to very deep whole genome sequencing is not excluded.

One disadvantage may be that the tissue of origin is more difficult to determine or cannot be determined by applying whole-genome sequencing-based chromosomal instability analysis. It can thus also not be excluded that a chromosomal instability pattern determined for a given sample that is deviating from the reference or control chromosomal instability pattern is not caused by a tumor or cancer or pre-tumorous or pre-cancerous condition, but rather is caused by another pathological condition or pre-pathological condition. One solution may come from catalogs or databases comprising information on chromosomal instability patterns determined for a population of sequenced samples from which the origin and/or (pre-)pathological condition is known. While such catalogs or databases may still need to be compiled, it is plausible that it is only a matter of time for them (as well as for reference sets or control sets or patterns of chromosomal instability) to be constructed. Such catalog may also comprise, for a given disease, disorder or (pre-) pathological condition, time-dependent or stage-dependent chromosomal instability patterns, or chromosomal instability patterns representative for response to therapy, for relapse, or for metastasis (in case of a tumor or cancer). An alternative solution is to search in the whole-genome sequencing-based chromosomal instability results for the absence or presence of a set of markers known to be specific for a given tissue or organ (providing information on the origin) and/or for a set of markers known to be associated with a (pre-)pathological condition. Pre-pathological conditions in general refer to the presence of markers of a pathological condition before standard clinical signs of the pathological condition become recognizable.

The reference or control cfDNA chromosomal instability pattern is a cfDNA chromosomal instability pattern representative for a healthy or control subject. It can be based on cfDNA samples from one or more healthy or control subjects, or on cfDNA samples from germline material from one or more healthy subjects or subjects having the disease, disorder or (pre-)pathological condition of interest. Ideally, the reference or control cfDNA is of the same source as the source of the test cfDNA (e.g. blood, serum or plasma). Alternatively, the reference or control cfDNA is obtained from blood, serum or plasma, and the source of the test cfDNA is different.

A CNA score for a DNA is a numerical value providing information on the level of copy number alteration present within the analyzed DNA; the numerical value is resulting from a numerical calculation, such as by a computerized numerical calculation or numerical calculation performed by a computer system. Alternative numerical calculation methods are possible, of importance is that the same numerical calculation method is used when comparing CNA scores of a test sample and a reference sample.

A genome-wide CNA score as used herein is perfectly suited to compress the complexity of large amounts of molecular data relating to genome-wide alterations into a biologically and clinically meaningful parameter highly increasing interpretability. When chromosomal instability catalogs as described above become available, it is expected that genome-wide CNA analysis will not be required and will optionally be replaced by a more focused sequencing effort (low coverage or higher coverage). Combining genome-wide CNA scores as used herein and calling for mutations furthermore is, for a certain cancer type, not mutually exclusive. Mutations in TP53, for instance, are known to be the most common mutations in C-class HGSOC (Bowtell et al. 2015, Nat Rev Cancer 15:668-679).

**Sample / biological sample**

A sample can be any biological sample isolated from or obtained from a subject. For example, a sample can comprise, without limitation, bodily fluid, whole blood, serum, plasma, synovial fluid, lymphatic fluid, ascites fluid, interstitial or extracellular fluid, the fluid in spaces between cells, including gingival crevicular fluid, cerebrospinal fluid, saliva, mucous, sputum, phlegm, smegma, seminal fluid, ejaculate, sweat, tears, urine, fluid from nasal brushings, colonic washing fluid, fluid from a pap smear, vaginal fluid, vaginal flushing fluid, fluid from a hydrocele, pleural fluid, bronchoalveolar lavage fluid, discharge fluid from the nipple, aspiration fluid from a part of the body, colostrum, breast milk, ventricular fluid, any other bodily fluids. A bodily fluid can include saliva, blood, or serum. A sample can comprise a volume of plasma containing cell free DNA molecules. A sample may comprise a volume of plasma sufficient to achieve a given read depth. A volume of sampled plasma may be at least 0.5 milliliters (mL), 1 mL, 5 mL 10 mL, 20 mL, 30 mL, or 40 mL, or a volume of at most 0.5 mL, 1 mL, 5 mL 10 mL, 20 mL, 30 mL, or 40 mL. A volume of sampled plasma may be 0.5 to 20 mL, or may be 5 to 20 mL. A volume of sampled plasma may be 10 ml to 20 mL.

Isolation or extraction of polynucleotides may be performed through collection of bodily fluids using a variety of techniques. In some cases, collection may comprise aspiration of a bodily fluid from a subject using a syringe. In other cases collection may comprise pipetting or direct collection of fluid into a collecting vessel. In yet further cases, it may comprise collecting fluid with a small brush or brush-like device, and washing the brush or brush-like device in a suitable buffer to release polynucleotides contained in the collected fluid. A sample may be comprising cells. For the isolation of cfDNA, cells can be removed from the sample e.g. by precipitation, centrifugation or filtration.

After collection of bodily fluid, and, when required, after removal of cells, polynucleotides such as cfDNA may be isolated or extracted using a variety of techniques utilized in the art. In some cases, cell-free DNA may be isolated, extracted and prepared using commercially available kits such as the Qiagen® Qiamp® Circulating Nucleic Acid Kit protocol. In other examples, Qiagen® Qubit™ dsDNA HS Assay kit protocol, Agilent™ DNA 1000 kit, or TruSeq™ Sequencing Library Preparation; Low-Throughput (LT) protocol may be used. Further DNA extraction products include the DNeasy Blood and Tissue extraction kit (Qiagen®) and automated systems for DNA extraction such as the QiaSymphony® (Qiagen®) and Chemagen 360 (PerkinElmer).

In some instances, a plasma sample is treated with proteinase K (to degrade proteins/proteinaceous compounds present in the sample) and DNA is precipitated (e.g. with isopropanol) and subsequently captured on e.g. a Qiagen® column. The DNA then can be eluted by an eluent such as water or Tris-EDTA (TE) elution buffer. In some embodiments, a portion of the DNA can be selected based on size (e.g., DNA of 500 nucleotides or fewer in length), for example, using Solid Phase Reversible Immobilization (SPRI) beads, such as AgenCourt®AMPure® beads. In some embodiments, the DNA can be resuspended in a smaller volume. Approximately 5 ng of DNA may be equivalent to about 1500 haploid genome equivalents ("HGE").

In any of the above method or methods, the DNA methylation may be analyzed by means of high-throughput sequencing, pyrosequencing, sequencing-by-synthesis, single- molecule sequencing, nanopore sequencing, semiconductor sequencing, sequencing-by-ligation, sequencing-by-hybridization, bisulfite sequencing, methylation-sensitive single-strand conformation analysis, high resolution melting analysis, methylation-sensitive single nucleotide primer extension, base-specific cleavage/MALDI-TOF, methylation-specific PCR, a microarray-based method, or reduced representation bisulfite sequencing. In any of the above method or methods, in particular an agent measuring methylation in the cfDNA is used for analyzing the presence of DNA methylation. Such agents include for example/ such agent can be selected from (the group consisting of)/ such agent can be chosen from: a compound modifying an unmethylated cytosine base, a methylation-sensitive restriction enzyme, an oligonucleotide specific to a methylated cfDNA site, and/or an oligonucleotide specific to the unmethylated cfDNA site. Herein, the compound modifying an unmethylated cytosine base is more in particular a compound modifying the unmethylated cytosine into an uracil base. An oligonucleotide specific to a methylated CpG site is an oligonucleotide that hybridizes specifically to the methylated CpG and not to the unmethylated CpG; specificity can be obtained by e.g. converting the unmethylated cytosine base into an uracil base. An oligonucleotide specific to an unmethylated CpG site is an oligonucleotide that hybridizes specifically to the unmethylated CpG and not to the methylated CpG.

In any of the above method or methods, and for purpose of analyzing the presence of DNA methylation, the unmethylated cytosine bases in the obtained cfDNA can be converted to uracil bases prior to analyzing the presence of DNA methylation. Furthermore in particular, such conversion may be performed before, preceding, or prior to amplification of the cfDNA.

In any of the above method or methods, a portion of the cfDNA may optionally be isolated, extracted or purified from the biological sample or partly processed biological sample using oligonucleotides. Such oligonucleotides can be attached to a solid support (the solid support may optionally further be part of e.g. a cartridge or container e.g. fitting or fitted in e.g. an automated and/or computerized analysis device) or can be comprised in a microfluidic drop or microdrop (e.g. for purposes of droplet PCR or droplet digital PCR).

In any of the above method or methods, the nucleosome footprint of the cfDNA and/or CNA in the cfDNA may be analyzed via sequencing, such as via low-coverage sequencing, ultralow-pas sequencing, shallow sequencing, or by random non-targeted sequencing.

Terms as used in the above further embodiments are explained in detail hereafter.

**Determination of DNA methylation**

Assays for DNA methylation analysis have been reviewed by e.g. Laird 2010 (Nat Rev Genet 11:191-203). The main principles of possible sample pre-treatment involve enzyme digestion (relying on restriction enzymes sensitive or insensitive to methylated nucleotides), affinity enrichment (involving e.g. chromatin immunoprecipitation, antibodies specific for 5MeC, methyl-binding proteins), sodium bisulfite treatment (converting an epigenetic difference into a genetic difference) followed by analytical steps (locus-specific analysis, gel-based analysis, array-based analysis, next-generation sequencing-based analysis) optionally combined in a comprehensible matrix of assays. Laird 2010 is providing a plethora of bioinformatic resources useful in DNA methylation analysis which can be applied by the skilled person as guiding principles, when wishing to analyze the methylation status of up to about 100 CpGs in a sample, with assays such as MethyLight, EpiTYPER®, MSP, COBRA, Pyrosequencing, Southern blot and Sanger BS appearing to be the most suitable assays. This guidance does, however, not take into account that assays with higher coverage can be adapted towards lower coverage. For example, design of custom DNA methylation profiling assays covering up to 96 or up to 384 individual regions is possible e.g. by using the VeraCode® technology provided by Illumina® (compared to the 450K DNA methylation array covering approximately 480000 individual CpGs). Another such adaptation for instance is enrichment of genome fractions comprising methylation regions of interest which is possible by e.g. hybridization with bait sequences or capture probes. Such enrichment may occur before bisulfite conversion (e.g. customized version of the SureSelect® Human Methyl-Seq from Agilent®) or after bisulfite conversion (e.g. customized version of the SeqCap® Epi CpGiant Enrichment Kit from Roche®). Such targeted enrichment can be considered as a further modification/simplification of RRBS (Reduced Representation Bisulfite Sequencing).

The MethyLight assay is a high-throughput quantitative or semi-quantitative methylation assay that utilizes fluorescence-based real-time PCR (e.g., TaqMan®) that requires no further manipulations after the PCR step (Eads et al. 2000, Nucleic Acids Res 28:e32). Briefly, the MethyLight process begins with a mixed sample of genomic DNA that is converted, in a sodium bisulfite reaction, to a mixed pool of methylation- dependent sequence differences according to standard procedures (the bisulfite process converts unmethylated cytosine residues to uracil). Fluorescence-based PCR is then performed in a "biased" reaction, e.g., with PCR primers that overlap known CpG dinucleotides. Sequence discrimination occurs at the level of the amplification process, at the level of the probe detection process, or at both levels. An unbiased control for the amount of input DNA is provided by a reaction in which neither the primers, nor the probe, overlie any CpG dinucleotides. Alternatively, a qualitative test for genomic methylation is achieved by probing the biased PCR pool with either control oligonucleotides that do not cover known methylation sites or with oligonucleotides covering potential methylation sites.

The EpiTYPER® assay involves many steps including gene-specific amplification of bisulfite-converted genomic DNA, *in vitro* transcription of the amplified DNA, uranil-specific cleavage of transcribed RNA, and MALDI-TOF analysis of the RNA fragments. The EpiTYPER software finally distinguishes between methylated and non-methylated cytosine in the genomic DNA.

Methylation-specific PCR (MSP) refers to the methylation assay as described by Herman et al. 1996 (Proc Natl Acad Sci USA 93:9821-9826), and by US 5,786,146. MSP (methylation-specific PCR) allows for assessing the methylation status of virtually any group of CpG sites within a CpG island, independent of the use of methylation-sensitive restriction enzymes. Briefly, DNA is modified by sodium bisulfite, which converts unmethylated, but not methylated cytosines, to uracil, and the products are subsequently amplified with primers specific for methylated versus unmethylated DNA. MSP requires only small quantities of DNA, is sensitive to 0.1% methylated alleles of a given CpG island locus, and can be performed on DNA extracted from paraffin-embedded samples. MSP primer pairs contain at least one primer that hybridizes to a bisulfite treated CpG dinucleotide. Therefore, the sequence of said primers comprises at least one CpG dinucleotide. MSP primers specific for non- methylated DNA contain a "T" at the position of the C position in the CpG. Variations of MSP include Methylation-sensitive Single Nucleotide Primer Extension (Ms-SNuPE; Gonzalgo & Jones 1997, Nucleic Acids Res 25:2529-2531). Another variation, however including restriction enzyme digestion instead of bisulfite modification as sample pretreatment, is Methylation- Sensitive Arbitrarily-Primed Polymerase Chain Reaction (MS AP-PCR; Gonzalgo et al. 1997, Cancer Research 57:594-599).

Combined Bisulfite Restriction Analysis (COBRA) refers to the methylation assay described by Xiong & Laird 1997 (Nucleic Acids Res 25:2532-2534). COBRA analysis is a quantitative methylation assay useful for determining DNA methylation levels at specific loci in small amounts of genomic DNA. Briefly, restriction enzyme digestion is used to reveal methylation- dependent sequence differences in PCR products of sodium bisulfite-treated DNA. Methylation-dependent sequence differences are first introduced into the genomic DNA by bisulfite treatment. PCR amplification of the bisulfite converted DNA is then performed using primers specific for the CpG islands of interest, followed by restriction endonuclease digestion, gel electrophoresis, and detection using specific, labeled hybridization probes. Methylation levels in the original DNA sample are represented by the relative amounts of digested and undigested PCR product in a linearly quantitative fashion across a wide spectrum of DNA methylation levels. In addition, this technique can be reliably applied to DNA obtained from microdissected paraffin-embedded tissue samples.

Sanger BS is the original way of analysis of bisulfite-treated DNA: gel electrophoresis-based Sanger sequencing of cloned PCR products from single loci (Frommer et al. 1992, Proc Natl Acad Sci USA 89:1827-1831). A technique such as pyrosequencing is similar to Sanger BS and obviates the need of gel electrophoresis; it, however, requires other specialized equipment (e.g. Pyromark instrument). Sequencing approaches are still applied, especially with the emergence of next-generation sequencing (NGS) platforms. Southern blot analysis of DNA methylation depends on methyl-sensitive restriction enzymes (e.g. Moore 2001, Methods Mol Biol 181:193-201).

Other assays to determine CpG methylation include the HeavyMethyl® (HM) assay (Cottrell et al. 2004, Nucleic Acids Res 32, e10; WO2004113567), Methylated CpG Island Amplification (MCA; Toyota et al. 1999, Cancer Res 59:2307-12; WO 00/26401), Reduced Representation Bisulfite Sequencing (RRBS; e.g. Meissner et al. 2005, Nucleic Acids Res 33: 5868-5877), Quantitative Allele-specific Real-time Target and Signal amplification (QuARTS®; e.g. WO2012067830), and assays described in Laird et al. 2010 (Nat Rev Genet 11:191-203) and in Kurdyukov & Bullock 2016 (Biology 5(1), pii: E3). Tailored to determine CpG methylation in cfDNA are for instance the cf-RRBS method (De Koker et al. 2019, bioRxiv:663195, doi: http://dx.doi.org/10.1101/663195; WO 2017/162754; Van Paemel et al. 2019, bioRxiv:795047, doi: https://doi.org/10.1101/795047). RRBS methods provide an acceptable balance between genome-wide coverage and accurate quantification of the methylation status and this at an affordable cost. Other methods tailored to analysis of methylation in cfDNA are described in WO2019006269 and US20100240549A1.

## Polynucleotide sequencing / sequencing depth / low coverage whole genome sequencing / sequence read

Polynucleotide sequencing is a common step in the methods as applied in the current invention. In general, sequencing methods may include, but are not limited to: high-throughput sequencing, pyrosequencing, sequencing-by-synthesis, single- molecule sequencing, nanopore sequencing, semiconductor sequencing, sequencing-by-ligation, sequencing-by-hybridization, RNA-Seq (Illumina®), Digital Gene Expression (Helicos), Next generation sequencing, Single Molecule Sequencing by Synthesis (SMSS) (Helicos), massively- parallel sequencing, Clonal Single Molecule Array (Solexa®), shotgun sequencing, Maxam- Gilbert or Sanger sequencing, primer walking, sequencing using PacBio®, SOLiD®, Ion Torrent®, or Nanopore platforms and any other sequencing methods known in the art. The sequencing method can be massively parallel sequencing, that is, simultaneously (or in rapid succession) sequencing any of at least 100, 1000, 10,000, 100,000, 1 million, 10 million, 100 million, 1 billion, or 10 billion polynucleotide molecules.

Certain DNA sequencing methods may rely on the capture of polynucleotides of interest such as to enrich for these sequences of interest. Polynucleotide or sequence capture typically involves the use of oligonucleotide probes that hybridize to the polynucleotide or sequence of interest. A probe set strategy can involve tiling the probes across a region of interest (complete or partial tiling of the target sequence with probes). Such probes can be, e.g., 10 to 400 or about 400 bases long, 10 to 300 or about 300 bases long, 10 to 200 or about 200 bases long, 10 to 100 or about 100 bases long, 10 to 80 or about 80 bases long, 10 to 60 or about 60 bases long, Such probes may comprise at least one or a set of oligonucleotides of 10 to 60 bases or nucleotides long and/or comprise at least one or a set of oligonucleotides of 15 to 120 bases or nucleotides long. Any set of such oligonucleotide probes can have a depth of about 0.1x, 0.2x, 0.3x, 0.4 x, 0.5x, 0.1x to 0.5x, 1x 2x, 3x, 4x, 5x, 6x, 8x, 9x, 10x, 15x, 20x, 50x or more. Enriched nucleic acid molecules can be representative of a nucleic acid features of interest such as, but not necessarily limited to copy number variants (CNVs), insertions or deletions (e.g., indels), nucleosome regions, specific DNA methylation sites.

Sequencing depth refers to the number of times a locus is covered by a sequence read aligned to the locus. A locus can be as small as a nucleotide, as large as a chromosome arm, or as large as the entire genome. Sequencing depth can be expressed as e.g. 10x, 50x, 100x, where "x" refers to the number of times a locus is covered by a sequence read. Sequencing depth can also be applied to multiple loci, or to the whole genome, in which case "x" can refer to the mean number of times the loci, or whole genome, is sequenced. Ultra-deep sequencing refers to a sequencing depth of at least 100x.

Shallow whole genome sequencing, low coverage whole genome sequencing, or ultra-low pass whole genome sequencing in general refers to short-read sequencing of genomes at low coverage, typically less than 3x coverage, less than 2x coverage, less than 1x coverage, such as 0.1x to 1x coverage, such as 0.1x to 0.8x coverage, such as 0.1x to 0.6x coverage, such as 0.1x to 0.5x coverage, such as 0.1x to 0.4x coverage, such as 0.1x to 0.3x coverage, such as 0.9x coverage, 0.8x coverage, 0.7x coverage, 0.6x coverage, 0.5x coverage, 0.4x coverage, 0.3x coverage, 0.2x coverage or 0.1x coverage, such as 0.1x coverage or less. Sequencing coverage can also be expressed as average sequencing coverage. Low coverage in the context of sequencing thus can also refer to typically on average less than 3x coverage, on average less than 2x coverage, on average less than 1x coverage, such as on average 0.1x to 1x coverage, such as on average 0.1x to 0.8x coverage, such as on average 0.1x to 0.6x coverage, such as on average 0.1x to 0.5x coverage, such as on average 0.1x to 0.4x coverage, such as on average 0.1x to 0.3x coverage, such as on average 0.9x coverage, on average 0.8x coverage, on average 0.7x coverage, on average 0.6x coverage, on average 0.5x coverage, on average 0.4x coverage, on average 0.3x coverage, on average 0.2x coverage or on average 0.1x coverage, such as on average 0.1x coverage or less.

By performing shallow whole genome sequencing, low coverage whole genome sequencing, or ultra-low pass whole genome sequencing, each sample is subjected to a small amount of sequencing, allowing application of whole genome sequencing to many samples at low cost per sample.

A sequence read is a string of nucleotides sequenced from a part or all of a nucleic acid molecule. A sequence read may be a short string of nucleotides (e.g. 20 to 150 nucleotides, around 50 nucleotides) sequenced from a nucleic acid (fragment). Sequence reads may be obtained at one end of a nucleic acid (fragment) or from both ends of a nucleic acid (fragment). Sequence reads may be obtained by e.g. applying a sequencing technique to the nucleic acid (fragment), by hybridization arrays or capture probes, by amplification techniques (e.g. PCR, linear amplification, isothermal amplification) such as amplification techniques using a single primer.

Thus, obtaining information from the nucleic acid molecules (e.g. cfDNA molecules) present in a biological sample may include a step of preparing a sequencing library using the nucleic acid molecules (e.g. cfDNA molecules) isolated from the biological sample. The preparation of such sequencing library may include a step of DNA amplification, or may, alternatively, not include a step of DNA amplification. Obtaining information from the nucleic acid molecules (e.g. cfDNA molecules) present in a biological sample may DNA or cfDNA sequence reads. Obtaining information from the nucleic acid molecules (e.g. cfDNA molecules) present in a biological sample may include the step of aligning the plurality of sequence reads to a reference genome to determine the genomic positions of each (individual) sequence read of the plurality of sequence reads. In view of the size of the reference genome and the number of sequence reads in the plurality of sequence reads, the sequence reads are usually received at a computer system.

In any of the above method or methods, at least one analysis step may be performed computationally, by a computer system or via a computer program product. As such, any of the above method or methods can be defined as computer-implemented method or computer-implemented methods. Alternatively, but not mutually exclusive, in any of the above method or methods, the calculation of the methylation score, the calculation of the nucleosome score, the calculation of the CNA score, and/or the calculation of the cfDNA single score is performed computationally, by a computer system or via a computer program product.

In one aspect thereto, the invention relates to a computer product comprising a computer readable medium storing instructions for operating a computer system to perform at least one analysis and/or calculation as outlined hereinabove.

**Computer / computer system / computer-implemented methods**

A computer or computer system as mentioned herein may utilize one or more subsystems. A computer or computer system may be a single computer apparatus comprising the one or more subsystems (e.g internal components), or may be multiple computers or multiple computer apparatuses each being a subsystem, and optionally, each comprising one or more own subsystems. Desktops, laptops, mainframe servers, tablets, mobile phones etc. all are computers or computer systems. The subsystems are usually interconnected and include a (central) processor (single-core processor, multi-core processor on a same integrated chip, or multiple processing units on a single circuit board or networked) capable of executing instructions, an input/output (I/O) controller, and a storage device (external, internal, peripheral, cloud, any medium readable by a computer or computer system). Input devices include keyboards, scanners, a computer mouse, camera, microphone, etc. In particular, the input device is a data collection or data generating device (which by itself may comprise a computer or computer system), such as a polynucleotide sequencing device (whether automated or not). Collected or generated data are fed to a computer or computer system designed to analyze the collected or generated data; this may be an ordinary computer system on which data analyzing software is installed (on a storage device) or which is capable of accessing data analyzing software (e.g. installed in or transmitted from a network) and whereby the processor of the computer system is instructed by the data analysis software on how to process the collected or generated data fed to the computer system, and how to display these via a display adapter to an output device. Output devices are further subsystems and comprise printers, monitors, computer readable medium. Input and output devices are usually connected to a computer or computer system via input/output ports to one another or via a network.

The specific combination of hardware and software allows implementation of e.g. analysis of data generated by a polynucleotide sequencing device. Different software packages (proprietary or open source) can be run on a computer or computer system to achieve the desired degree of data analysis. Output of one computerized data analysis can be the input of a subsequent computerized data analysis step, hence creating an analysis pipeline. Software components can be written in different codes (e.g. Java®, C, C++, Perl, Python) as long as the computer processor is able to execute the functions of the software component.

The methods of the invention may be computer-implemented methods, or methods that are assisted or supported by a computer or by a computer system. For instance, information required for the analysis, determination, detection, presence or absence of DNA methylation, the analysis of a nucleosome footprint and/or the analysis of copy number alteration in the cfDNA obtained from a sample is received by at least one first processor, and/or information required for the analysis, determination, detection, presence or absence of DNA methylation, the analysis of a nucleosome footprint and/or the analysis of copy number alteration in the cfDNA obtained from a sample is provided in user readable format by at least one/another processor. The same or a further processor may be calculating a cfDNA methylation score, a cfDNA nucleosome score and/or a cfDNA CNA score; or a cfDNA single score from the information received. The one or more processors may be coupled to random access memory operating under control of or in conjunction with a computer operating system. The processors may be included in one or more servers, clusters, or other computers or hardware resources, or may be implemented using cloud-based resources. The operating system may be, for example, a distribution of the LinuxTM operating system, the UnixTM operating system, or other open- source or proprietary operating system or platform. Processors may communicate with data storage devices, such as a database stored on a hard drive or drive array, to access or store program instructions other data. Processors may further communicate via a network interface, which in turn may communicate via the one or more networks, such as the Internet or other public or private networks, such that a query or other request may be received from a client, or other device or service. Such computer-implemented methods (or such methods that are assisted or supported by a computer) may be provided as a kit or as part of a kit. The bioinformatics software required to perform (part of) the computer-implemented methods, i.e. a computer program product, may also be part of a kit, or may be provided as an individual product. A computer product may also consist of a computer readable medium which is storing any of the instructions, computer program, or bioinformatics software enabling a computer system to perform at least one of the analysis of the herein described methods and/or to perform at least one calculation (of cfDNA scores) as described herein.

In any of the above method or methods and all embodiments related thereto, the subject may have been receiving or may be receiving a treatment for the disease or disorder.

Furthermore, any of the above method or methods and all embodiments related thereto may more in particular be a method or methods for diagnosing a disease or disorder in a subject, for detecting the presence of a disease or disorder in a subject, for early detection of a disease or disorder in a subject, for early diagnosis of a disease or disorder in a subject, for screening for the presence of a disease or disorder in a subject, for determining an increased likelihood for a disease or disorder to be present in a subject, for monitoring a disease or disorder in a subject, for determining a response of a disease or disorder to therapy, for monitoring a disease or disorder after therapy, or for predicting a response of a disease or disorder to therapy.

In any of the above method or methods and all embodiments related thereto, the disease or disorder may be cancer or a tumor, acute or chronic tissue damage, an inflammatory disorder or an autoimmune disease.

The invention therefore extends to a method or methods for diagnosing a disease or disorder in a subject, for detecting the presence of a disease or disorder in a subject, for early detection of a disease or disorder in a subject, for early diagnosis of a disease or disorder in a subject, for screening for the presence of a disease or disorder in a subject, for determining an increased likelihood for a disease or disorder to be present in a subject, for monitoring a disease or disorder in a subject, for determining a response of a disease or disorder to therapy, for monitoring a disease or disorder after therapy, or for predicting a response of a disease or disorder to therapy, the method comprising:

- obtaining, extracting, isolating or purifying cell-free DNA (cfDNA) from a biological sample obtained from the subject;
- analyzing, assaying, assessing, measuring or determining the presence of DNA methylation of the obtained, extracted, isolated or purified cfDNA;
  and:
    - analyzing, assaying, assessing, measuring or determining a nucleosome footprint of the obtained, extracted, isolated or purified cfDNA;
      and/or
    - analyzing, assaying, assessing, measuring or determining copy number alteration (CNA) in the obtained, extracted, isolated or purified cfDNA.

All embodiments outlined in relation to the higher-described methods of the invention likewise apply to a method or methods for diagnosing a disease or disorder in a subject, for detecting the presence of a disease or disorder in a subject, for early detection of a disease or disorder in a subject, for early diagnosis of a disease or disorder in a subject, for screening for the presence of a disease or disorder in a subject, for determining an increased likelihood for a disease or disorder to be present in a subject, for monitoring a disease or disorder in a subject, for determining a response of a disease or disorder to therapy, for monitoring a disease or disorder after therapy, or for predicting a response of a disease or disorder to therapy.

Some further terms are explained in detail hereafter.

Diagnosis of a disease or condition in a subject in general refers to any act which determines the disease or condition to be present in a subject, irrespective of the stage of the disease or disorder; detection of the presence of a disease or disorder in a subject can be considered as an interchangeable term for diagnosis of a disease or condition in a subject. The "classical definition" of diagnosis is in principle broader than the diagnosis or detection methods described herein. However, the diagnosis or detection methods described herein perfectly fit within and are part of a classical diagnosis (i.e. identification of a condition, disease, disorder, or problem by systematic analysis of the background or history, examination of the signs or symptoms, evaluation of the research or test results, and investigation of the assumed or probable causes). The subject may be suspected of having the disease or disorder or may not be suspected of having the disease or disorder. The subject may have experienced symptoms consistent with a diagnosis of the disease or disorder; or the subject may not have experienced any symptoms, or may have exhibited symptoms not consistent with the disease or disorder.

Early detection of a disease or condition in a subject in general refers to detection of the disease or condition in the subject even before common clinical signs or symptoms of the disease or condition are recognized by the subject or by a general practitioner or physician; or before the subject, general practitioner or physician are even aware of the onset of the disease or condition in the subject's body. Upon early detection of a disease or condition, examination of the subject by e.g. imaging methods (such as one or more of positron emission tomography scan, magnetic resonance imaging, X-ray, computerized axial tomography scan, endoscopy, ultrasound) or serum protein tests may not reveal the presence of the disease or condition. At this stage, the disease or disorder can be referred to as or be qualified as premalignant or pre-pathological. Early diagnosis can be considered as an interchangeable term for early detection

Screening for the presence of a disease or condition in a subject in general refers to testing an individual or a subset of a population for the presence of any signs of a disease or condition. In particular, screening for the presence of a disease or disorder can be applied to a subset of a population known to have an increased risk to develop the disease or disorder (e.g. due to age or gender, due to unhealthy habits, due to comorbidities known to put an individual at an increased risk for developing or for having the disease or disorder, due to familial history) and is aiming at identifying at early stage those individuals within the otherwise healthy (with respect to the disease or disorder screened for) population that are in need of closer medical follow-up with respect to the disease or disorder. The latter can also be referred to as targeted screening for the presence of a disease or condition in a subject. Depending on the disease or condition, the methods and systems described herein may detect the disease or disorder before it becoming detectable (or re-detectable in case of relapse) using conventional or clinically established methods, e.g., at least 1 year, 6 months, 3 months, or 1 month before the disease or disorder may be detectable by using conventional or clinically established methods. In case of the disease or condition being a cancer or tumor, the methods and systems described herein may detect the cancer or tumor before it becoming detectable (or re-detectable in case of relapse) using conventional or clinically established methods, e.g. at least 1 year, 6 months, 3 months, or 1 month before the cancer or tumor may be diagnosed using conventional or clinically established methods at stage I, stage II, stage III, or stage IV, or at least 1 year, 6 months, 3 months, or 1 month before the cancer may recur. Determination of an increased likelihood for a disease or condition to be present in a subject in general either refers to early detection of a disease or condition, or to screening for the presence of a disease or disorder, more particularly to targeted screening.

Methods for monitoring a disease or condition in a subject in general refers to the above-described detection or diagnosis methods applied to a monitoring setting. Monitoring a disease or condition is of importance to follow up the course of the disease or condition (is it progressing, progressive or in progression; is it regressing, regressive or in regression; is it stable) with or without the disease or condition being treated. If the disease or condition is being treated, then the monitoring is the monitoring or determination of a response of the disease or disorder to therapy (is the subject a responder or responsive to the therapy, a non-responder or non-responsive to the therapy, or a partial responder or partially responsive to the therapy). If the disease or condition has been successfully treated (such as by pharmacological treatment or by surgery), then continued monitoring of the disease or condition in the subject (monitoring the disease or condition post-therapy or after therapy) may be required in order to follow-up whether the disease or condition is not re-occurring or relapsing, or has not spread to other tissues or organs (e.g. metastasis of a cancer or tumor) - in this setting early detection of diagnosis is of primordial importance. In case the disease or disorder is a cancer, the subject may have received surgical treatment, radiation treatment, chemotherapy, targeted cancer therapeutics, a cancer immunotherapy, a cancer vaccine, on oncolytic virus or any experimental cancer treatment.

Predicting a response of a disease or condition to therapy can also be within the ambit of the methods of the current invention as such method can provide valuable genetic background information of the disease or condition. Targeted therapies of a disease or disorder often are successful only in a subset of the subjects having the disease or disorder, often dependent on the e.g. the genetic constellation underlying the disease or disorder. Positively correlating disease- or disorder-specific information with outcome of a certain therapy is at the basis of personalized medicine, and is contributing to increased therapeutic efficiency (the right therapy can be chosen for the specific disease or disorder as specifically present in a subject) and to decreased non-efficient treatment (therewith also decreasing the financial burden on the national healthcare systems). Predicting a response of a disease or condition to therapy can also contribute to selection of an efficient treatment regimen of the disease or condition. Indeed, a disease or condition can be responsive to an initial treatment, but upon subsequent monitoring of the disease or disorder, it is possible that relapse is detected, often as a consequence of changes in the genetic background of the cells having caused the initially diagnosed disease or disorder.

Sensitive methods for monitoring a disease or condition in a subject are furthermore of interest during clinical phase testing of new experimental drugs, compounds or medicaments for treating the disease or condition.

Thus, in the foregoing, screening can check if the disease or disorder is present in someone not previously known to have the disease or disorder. Monitoring can follow the progress of the disease or disorder over time, can study the effectiveness of therapies (whether approved or whether in clinical trial testing), and can assist in prognosis. In case of the disease or disorder being e.g. ovarian cancer, the prognosis of a subject diagnosed to have ovarian cancer can be expressed as, e.g., the chance of the subject dying of the ovarian cancer, the chance of the ovarian cancer progressing after a specific duration or time (e.g. with or without treatment), or the chance of the ovarian cancer to metastasize.

The disease or condition in one aspect is a cancer or tumor. In further aspects, the disease or condition is either one of e.g. acute or chronic tissue damage (of autologous tissue or allogenic tissue, such as an allograft; the tissue can be an organ), an inflammatory disorder, an autoimmune disorder.

**Other Definitions**

The present invention is described with respect to particular embodiments and with reference to certain drawings but the invention is not limited thereto but only by the claims. Any reference signs in the claims shall not be construed as limiting the scope. The drawings described are only schematic and are nonlimiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated. Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. Unless specifically defined herein, all terms used herein have the same meaning as they would to one skilled in the art of the present invention. Practitioners are particularly directed to Sambrook et al., Molecular Cloning: A Laboratory Manual, 4th ed., Cold Spring Harbor Press, Plainsview, New York (2012); and Ausubel et al., current Protocols in Molecular Biology (Supplement 100), John Wiley & Sons, New York (2012), for definitions and terms of the art. The definitions provided herein should not be construed to have a scope less than understood by a person of ordinary skill in the art.

The term "defined by SEQ ID NO:X" as used herein refers to a biological sequence consisting of the sequence of amino acids or nucleotides given in the SEQ ID NO:X. For instance, a nucleotide sequence defined in/by SEQ ID NO:X consists of the sequence of nucleotides given in SEQ ID NO:X. A further example is a nucleotide sequence comprising SEQ ID NO:X, which refers to a nucleotide sequence longer than the nucleotide sequence given in SEQ ID NO:X but entirely comprising the nucleotide sequence given in SEQ ID NO:X, or to a nucleotide sequence consisting of nucleotide sequence given in SEQ ID NO:X.

The subject in particular is a mammal. The group of mammals includes, besides humans, mammals such as primates, cattle, horses, sheep, goats, pigs, rabbits, mice, rats, guinea pigs, llama's, dromedaries and camels.

### AUC / ROC

AUC is referred to as area under the curve, in particular area under the ROC curve (ROC: receiver operating characteristic curve). A ROC curve plots true-positive rate (TPR) versus false-positive rate (FPR) at different classification thresholds; it is a graphical representation of the diagnostic ability of a classifier system upon variation of its discrimination threshold. AUC values range from 0 to 1. A model whose predictions are 100% wrong has an AUC of 0.0; one whose predictions are 100% correct has an AUC of 1.0.

## EXAMPLES

### EXAMPLE 1. Genome-wide copy number alteration and nucleosome footprints of cfDNA of patients with different stages of ovarian cancer

### 1.1. MATERIALS AND METHODS

#### 1.1.1. Patients and samples

Pre-treatment blood samples were obtained from 271 patients with an adnexal mass, undergoing surgical treatment. Patients were enrolled in the TRANS-IOTA study after diagnosis with transvaginal ultrasound at the University Hospitals Leuven (Belgium) between June 2015 and February 2017 (approved by the local Ethics Committee, s51375 and s59207). Age, BMI, final histology and FIGO stage were collected from the electronic patient files. Exclusion criteria were presence of or active therapy for non-ovarian cancer at the moment of inclusion, presence of immune disease, treatment with immunomodulators, pregnancy, age below 18 years, surgery of the suspected mass elsewhere prior to inclusion and positive infectious serology (HIV, HepB, HepC).

An additional 125 samples were collected as negative controls (referred to herein as healthy individuals), as approved by the local Ethics Committee, s50887. This group consisted of healthy donors and of patients consulting the hospital for non-ovarian related gynaecological complaints; the latter were only included after transvaginal ultrasound demonstrating two normal ovaries. Their median age was 52 years (IQR=17 years). Additionally, we included plasma samples from 43 patients with relapsed high-grade serous ovarian cancer (HGSOC). These patients all participated in the phase 2 GANNET53 trial (Concin et al. 2018, J Clin Oncol 36(15 Suppl):5567-5567) (NCT02012192). This trial included female patients with platinum-resistant relapsed ovarian cancer, treated with paclitaxel with or without the Hsp90-inhibitor ganetespib. Prospective collection of baseline blood samples for cfDNA extraction before treatment was included in the study protocol. Herein, the first batch of available baseline blood samples (n=43) were used for cfDNA extraction and development of the model.

Plasma was prepared and cfDNA was extracted as previously described (Vanderstichele et al. 2017, Clin Cancer Res 23:2223-2231). DNA sequencing libraries were prepared using the KAPA DNA Library Preparation Kit (KAPA Biosystems®, Wilmington, MA, USA). All samples were subjected to low-coverage whole-genome sequencing (LC-WGS) on a HiSeq platform (Illumina®, San Diego, CA, USA) using a V4 flow cell generating $1 \times 51$ bp reads, with a median read count of $10.4*10^6$ reads per sample (Table 1). For 19 of the non-HGSOC plasma samples, a matching formalin-fixed paraffin-embedded (FFPE) tumor biopsy sample was available. These were sequenced using LC-WGS similarly as to the plasma samples. In addition, 3 plasma samples with high tumoral load were selected for genome-wide paired-end sequencing on a NovaSeq 6000 platform (Illumina®, San Diego, CA, USA), generating $2 \times 151$ bp reads at coverage $7.4\times$,

18.8× and 30.6×.

**Table 1. Number of raw sequencing reads and sequencing coverage.**

| | Number of sequencing reads Median (IQR) [million] | Coverage Median |
|---|---|---|
| **Reference samples** | | |
| Healthy individuals (n=125) | 10.8 (5.91-14.8) | 0.17× |
| Relapsed HGSOC patients (n=43) | 12.8 (11.2-15.4) | 0.20× |
| **Patients with adnexal mass (n=271)** | 9.8 (7.8-13.8) | 0.16× |
| Benign mass (n=130) | 9.5 (7.4-12.4) | 0.15× |
| Borderline carcinoma (n=41) | 9.9 (8.5-13.4) | 0.16× |
| Invasive carcinoma (n=92) | 10.3 (8.5-15.4) | 0.16× |
| Metastasis (n=8) | 13.0 (10.5-27.8) | 0.21× |

### 1.1.2. Bio-informatics pipeline

Raw sequencing reads were mapped to the human reference genome Hg19 using BWA v0.7.1 (Li & Durbin 2009, Bioinformatics 25:1754-1760). Duplicate and low-quality reads were removed by Picard Tools v1.11 and Samtools v0.1.18 respectively (Li et al. 2009, Bioinformatics 25:2078-2079). *Genome-wide z-score*: chromosomal instability was assessed using genome-wide z-score calculation, as described previously (Vanderstichele et al. 2017, Clin Cancer Res 23:2223-2231). Briefly, the genome was divided in 1000 kbp bins, excluding sex chromosomes. Reads were counted in each bin and adjusted for total number of reads, GC-content and mappability. The bin values were smoothened by taking moving window averages of 50 adjacent bins, and then z-scores were calculated for each window using the distribution of healthy individuals as a reference. Subsequently, a single genome wide z-score was calculated for each sample as the z-score (again using healthy individuals as a reference) of the sum of squares of all window z-values.

For each sample i, we calculated the sum of squares Si of window z-values. To quantify chromosomal instability, we calculated the genome-wide z-score (Heitzer et al. 2013, Genome Med 5:30) as the Si normalized based on the healthy individuals (HI):

$$z_i = \frac{S_i - \overline{S_{HI}}}{SD(S_{HI})}$$

*Nucleosome score:* genome-wide deviation of nucleosome footprints was quantified in cfDNA using a nucleosome score. To this end, we compared the start positions of 51 bp Illumina® reads-representing the boundaries of circulating cfDNA fragments-to a map of nucleosome positions found in plasma of healthy individuals. We used a previously published list of $13*10^6$ nucleosome positions as a reference (Snyder et al. 2016, Cell 164:57-86). We calculated distances on autosomes between each read start and the nearest nucleosome center from the reference list. We only focused at distances i within a [-300, +300] bp range, and counted their frequencies *yi.* The distribution of distances displays a typical M-shaped profile: read starts are enriched at the edges of nucleosomes and are depleted at the centres of nucleosomes (Straver et al. 2016, Prenat Diagn 36:614-621).

To quantify deviations of this profile, we trained a model using plasma samples of a training set of 125 healthy individuals and 43 relapsed HGSOC patients. Given these reference samples *j*, the frequencies of distances i within the [-300, +300] range are modelled as a multinomial stochastic variable:

$$y_j \sim \text{Multinomial}(\theta = \theta_k),$$

in which *yj* is a vector for sample *j* containing the observed number of read starts at distances i from - 300 to +300, and θ*k* represents a probability simplex containing the probabilities for all distances i given class *kj* of the sample (either healthy or HGSOC, depending on training sample *j*). As such, θ*HGSOC* and θ*healthy* represent how read starts are positioned around expected nucleosome centers for samples of both classes.

After this training step, we quantified the nucleosome score of an unknown sample using a mixture parameter λ which optimizes the probability simplex θ*mixt* as a weighted average of θ*HGSOC* and θ*healthy* given the observed read counts *yobs*:

$$\theta_{mixt} = \lambda\ \theta_{HGSOC} + (1 - \lambda)\ \theta_{healthy},$$

$$y_{obs} \sim \text{Multinomial}(\theta = \theta_{mixt}).$$

If the M-shaped profile of a sample corresponds closely to those of the samples in the HGSOC reference set, $\lambda$ will have an estimated value near 1; if the M-shaped profile corresponds closely to the healthy reference samples, the value of $\lambda$ will be estimated to be near 0.

We implemented this analysis as a Bayesian hierarchical model with uninformative uniform priors in Stan (using the interface from R with package rstan v2.18.1 (Stan Development Team. Rstan 2018; <http://mc-stan-org/>). Four parallel Markov chains of 300 iterations were run after a warm-up of 300 iterations to estimate $\lambda$. Convergence was obtained for each sample according to the Rhat statistic and a visual check of the 4 Markov chains. The nucleosome score is determined as the median of the posterior sample of $\lambda$, which is constrained within 0 and 1.

*Non-HGSOC tumor tissue*: 19 FFPE tumor tissues, matched to a non-HGSOC plasma sample, were mapped to the human reference genome and reads were counted in bins in the same way as described above for the plasma samples. ASCAT (Van Loo et al. 2010, PNAS 107:16910-16915) was then used to estimate copy number segments for these tumors. The lengths of segments with non-neutral copy number was summed and expressed as a fraction of the total segment lengths. As a comparison, this was plotted against the same fractions in a published cohort of HGSOC tumor samples (Despierre et al. 2014, Gynecol Oncol 135:415-422).

### 1.1.3. Statistical analysis

Receiver operation characteristic (ROC) curves were constructed and the corresponding area under the curve (AUC) values were calculated using the pROC package in R (Robin et al. 2011, BMC Bioinformatics 12:77). To combine genome-wide z-scores and nucleosome scores into a single predictor and corresponding ROC curve, a logistic regression model with ranks of both scores was fitted. Optimism of the AUC value of the combined predictor was estimated using 500 non-parametric bootstrap iterations and subtracted to obtain an unbiased estimate of performance (Harrell 2015, Regression modeling strategies, Springer International Publishing). All data was processed in R version 3.1.3 (R Core Team, R:A language and environment for statistical computing; https://www.r-project.org/). GNU parallel was used for running scripts in parallel (Tange 2011, ;login USENIX Mag 36:42-47).

## 1.2. RESULTS

### 1.2.1. Plasma cfDNA fragments display a nucleosome footprint

First, we confirmed that LC-WGS of cfDNA can be used to retrieve information about nucleosome positions. For this, 3 cfDNA samples from high-grade serous ovarian carcinoma (HGSOC) patients were selected for paired-end sequencing at high coverage. As expected, the size of cfDNA fragments corresponded to the length of DNA wrapped around histones, with a peak occurring at 167 bps (Figure 1A). We also detected additional peaks with a length of 10 bps higher or lower, which reflects the helical pitch of the DNA molecule wrapped around the nucleosome, as previously reported (Snyder et al. 2016, Cell 164:57-86). To further illustrate the position of nucleosomes in specific chromosomal regions, we used the large window protection score (L-WPS score), which reflects the number of fragments spanning a 120 bps moving window minus the number of fragments with a fragment end within the 120 bps moving window (Snyder et al. 2016, Cell 164:57-86). When plotting L-WPS in function of chromosomal coordinates, we were indeed able to detect where nucleosomes were positioned in specific regions of the genome. Moreover, these positions closely corresponded to the nucleosome reference positions identified by Snyder et al. 2016 (Cell 164:57-86) in the plasma of healthy individuals (indicated by vertical lines in Figure 1B). We were thus able to leverage plasma cfDNA fragmentation and reveal information about the nucleosome positions, as described by Snyder et al. 2016 (Cell 164:57-86).

### 1.2.2. HGSOC patients display a global deviation in nucleosome footprints

We next focused on deviations of cfDNA fragmentation between healthy individuals and patients diagnosed with HGSOC. Particularly, 168 cfDNA samples were obtained from 125 healthy individuals and 43 patients with relapsed HGSOC disease. The latter group of patients was selected because we detected high allelic frequencies of TP53 mutations in the cfDNA of each patient, indicating that these patients had high amounts of ctDNA in their plasma and were therefore well suited as a training set to detect malignancy. Rather than performing WGS at full depth, we conducted LC-WGS with a median of $11.3*10^6$ single-end reads per sample, corresponding to a median coverage of 0.18$\times$ (see Table 1).

For every sample, reads were mapped and distances were calculated between the start of a sequencing read (i.e., a cfDNA fragment boundary) and the center of the nearest nucleosome from a reference list of 13*$10^6$ nucleosomes (Figure 1C). This reference list was generated from plasma of healthy individuals (Snyder et al. 2016, Cell 164:57-86). The distribution of these distances displayed an M-shaped curve, as shown in Figure 1D, with proportionally more cfDNA fragments starting at the edges compared to the centers of expected nucleosome positions.

When plotting these distributions separately, either for the 125 healthy individuals or 43 cancer patients, we noticed that edges of nucleosomes were relatively enriched and centers depleted for start positions of sequencing reads from healthy individuals relative to cancer patients (Figure 1D). This observation led us to hypothesize that nucleosome footprints in cfDNA from cancer patients deviate from the reference list of nucleosome positions constructed in plasma from healthy individuals. This reflects a shift in the distribution of cell types contributing to the circulating cfDNA pool, suggesting that it can be used as a biomarker to detect invasive disease in women with an adnexal mass.

**1.2.3. Calculating nucleosome and genome-wide z-scores based on cfDNA**

Next, we explored whether based on fragmentation patterns in cfDNA, we were able to predict malignancy in a clinical cohort of patients with adnexal masses. Particularly, this cohort consisted of baseline cfDNA samples collected from 271 new patients, of which 130 exhibited on pathological examination a benign adnexal mass, 41 had a borderline ovarian tumor (BOT), 92 exhibited invasive ovarian disease and 8 cases presented with adnexal metastases of a non-ovarian malignancy (Table 2). We performed LC-WGS on each cfDNA sample with a median of 9.8*$10^6$ single-end reads per sample, corresponding to a median read depth of 0.16× (see Table 1). We quantified the degree of overall deviation in cfDNA fragments using the above-described 168 LC-WGS samples as positive and negative training sets to predict malignancy. Particularly, sequencing reads were mapped and M-shaped distributions of distances between start positions of sequencing reads and nucleosome centers of a reference set were constructed (see Methods). Nucleosome scores between 0 and 1 were calculated for each of the 271 plasma samples as described in the Methods section, where values around 0 correspond to reference healthy profiles and values around 1 correspond to reference HGSOC profiles.

The distribution of all nucleosome scores for patients with benign, borderline and invasive disease stratified for FIGO stage is shown in Figure 2A (see also Figure 6). Additionally, we reconstructed whole-genome copy number alteration (CNA) profiles and calculated the corresponding genome-wide z-scores (Figure 2B and Figure 5) (Vanderstichele et al. 2017, Clin Cancer Res 23:2223-2231). We did not observe a substantial association of sequencing depth with either nucleosome scores or genome-wide z-scores. In the 130 samples from patients with benign tumors, the nucleosome score and genome-wide z-score decreased on average with 0.00 and 0.03 for every million sequenced reads, an effect we consider negligible. Patients with an increased body mass index (BMI) have an increased turnover of adipocytes, which may decrease the fraction of ctDNA (Wang et al. 2013, Clin Cancer Res 23:2223-2231). However, we could not find a significant association between baseline BMI values and either nucleosome scores or genome-wide z-scores; for every unit increase of BMI within the benign tumor patients the scores decreased on average with 000 and 0.11 respectively. Thus, we assume that both sequencing depth and BMI do not substantially affect the results of our cfDNA analyses.

The FIGO staging system is determined by the International Federation of Gynecology and Obstetrics (Fédération Internationale de Gynécologie et d'Obstétrique) and comprises 5 main stages and multiple substages for stages I to IV (e.g. Bhatla et al. 2019, Int J Gynecol Obstet 145: 129-135).

**Table 2. Clinical characteristics of the 271 patients with adnexal masses.**

| | Patients with an adnexal mass (n=271) | | | |
|---|---|---|---|---|
| | **Benign mass (n=130)** | **Borderline carcinoma (n=41)** | **Invasive carcinoma (n=92)** | **Metastatic tumour (n=8)** |
| Age (in years) | | | | |
| Median | 53 | 52 | 64 | 55 |
| IQR | 43-64 | 37-63 | 57-73 | 52-69 |
| Adnexal histology | | | | |
| [BENIGN] | | | | |
| Cystadenoma | 21 | - | - | - |
| Cystadenofibroma | 52 | - | - | - |
| Fibrothecoma | 1 | - | - | - |
| Teratoma | 25 | - | - | - |
| Leiomyoma | 13 | - | - | - |

(continued)

| | | | | |
|---|---|---|---|---|
| Adnexal histology | | | | |
| Other | 18 | - | - | - |
| [BORDERLINE] | | | | |
| Serous | - | 22 | - | - |
| Mucinous | - | 15 | - | - |
| Other | - | 4 | - | - |
| [INVASIVE] | | | | |
| High-grade serous | - | - | 62 | - |
| Low-grade serous | - | - | 6 | - |
| Mucinous | - | - | 8 | - |
| Endametrioid | - | - | 9 | - |
| Clear-cell | - | - | 3 | - |
| Non-epithelial | - | - | 4 | - |
| [METASTASIS] | | | | |
| Gastric cancer | - | | | 3 |
| Other | - | | | 5 |
| FIGO stage | | | | |
| IA | - | 30 | 15 | - |
| IB | - | 3 | | - |
| IC | - | 3 | 8 | - |
| IIA | - | 1 | 1 | - |
| IIB | - | 1 | 2 | - |
| IIIA | - | 2 | 4 | - |
| IIIB | - | 1 | 8 | - |
| IIIC | - | - | 22 | - |
| IVB | - | - | 32 | - |
| CA-1.25 (in kU/L) | | | | |
| Median | 20 | 30 | 206 | 37 |
| IQR | 12-34 | 18-109 | 63-643 | 23-91 |

### 1.2.4. Clinical correlations of nucleosome scores and genome-wide z-scores

Next, we explored how these nucleosome and genome-wide z-scores correlated with clinical characteristics of the 271 patients. Overall, we observed low values for nucleosome and genome-wide z-scores in patients with benign disease (Figure 2A-B). As with genome-wide z-scores, nucleosome scores of borderline carcinomas did not differ from patients with benign disease. On the contrary, advanced-stage (FIGO IV) HGSOC cases displayed a very high median genome-wide z-score of 16.5 (n=32; Figure 5). This also applied to the nucleosome scores, which with a median score of 0.65 was highest in advanced-stage FIGO IV patients. Overall, the median nucleosome score for all patients with invasive disease was 0.35, while for BOTs and benign tumors the median score was respectively 0.06 and 0.00.

We previously reported how genome-wide z-scores were not elevated in patients with invasive ovarian cancer that did not present with a high-grade serous histology (hereafter referred to as non-HGSOC patients), including non-epithelial histology (Vanderstichele et al. 2017, Clin Cancer Res 23:2223-2231). In the current study, we again observed an increased genome-wide z-score in only 5 out of 30 non-HGSOC patients. Probably, lack of genomic instability in non-HGSOC patients explains why, compared to HGSOC patients, these exhibit a weaker performance for the genome-wide z-score. Remarkably, however, we did observe a substantial increase in nucleosome scores in these 30 non-HGSOC tumors: the median nucleosome scores were 0.07, 0.33, 0.09, 0.19 and 0.07 for patients with clear cell, endometrioid, low-grade serous, mucinous and non-epithelial non-HGSOC disease (Figure 6). Overall, this suggests that nucleosome footprinting may be useful for the detection of tumors not characterized by CNAs. As such, nucleosome and genome-wide z-scores, which can both be derived from the same LC-WGS data, provide independent diagnostic information.

### 1.2.5. Performance of nucleosome-based prediction of malignancy

In order to further evaluate whether nucleosome or genome-wide z-scores can be used to predict malignancy in women with adnexal masses, we generated ROC curves and calculated AUC values to determine specificities and sensitivities of both scores (Figure 3). Nucleosome and genome-wide z-scores could distinguish 130 benign cases from a combined group of 141 patients with BOT, invasive carcinoma and ovarian metastasis, displaying an AUC value of 0.71 (95% CI: 0.65-0.77) and 0.72 (95% CI: 0.66-0.78) for both scores, respectively (Figure 3). When combining both metrics in a single ROC curve (see Methods), AUC values improved to 0.74 (95% CI: 0.68-0.80). When only invasive carcinoma was considered relative to benign cases (i.e., excluding BOTs), AUC values increased to 0.76 (95% CI: 0.70-0.82) and 0.81 (95% CI: 0.75-0.87) for nucleosome and genome-wide z-scores, respectively (Figure 3) and to 0.81 (95% CI: 0.76-0.87) when both scores were combined. Additionally, AUC values of both metrics to discriminate HGSOC cases (n=62; all FIGO stages) from benign cases (n=130) were respectively, 0.78 (95% CI: 0.70-0.86) and 0.90 (95% CI: 0.84-0.95), respectively, or 0.89 (95% CI: 0.84-0.95) when both scores were combined (Figure 3). The latter results confirm the value of assessing chromosomal instability in cfDNA for the detection of tumors with large-scale CNAs. Indeed, to detect HGSOC in cfDNA the genome-wide z-score exhibited superior values compared to the nucleosome score (Vanderstichele et al. 2017, Clin Cancer Res 23:2223-2231; Ciriello et al. 2013, Nat Genet 45:1127-1133). As we previously observed that a significant number of non-HGSOC cases, which typically are characterized by low genome-wide z-scores (see below), exhibit elevated nucleosome scores, we also assessed how both tests performed when comparing non-HGSOC cases (n=30; all FIGO stages) to benign cases (n=130). Nucleosome scores performed better than genome-wide z-scores (AUC 0.74 (95% CI: 0.65-0.84) versus 0.63 (95% CI: 0.51-0.75) respectively), illustrating that a subset of cases with a low genome-wide z-score (typically non-HGSOC cases) might be detectable through an independent nucleosome-based analysis of LC-WGS data (Figure 3). Notably, by comparing AUC values of both HGSOC and non- HGSOC cases to patients with benign disease using the nucleosome (0.78 versus 0.74, respectively) and genome-wide z-score (0.90 versus 0.63, respectively), the sensitivity of the nucleosome score appeared stable across both HGSOC and non-HGSOC subgroups, indicating it to be a more generic test to detect tumor-derived cfDNA.

Next, we correlated genome-wide z-scores and nucleosome scores for all invasive cases (n=100, including 8 patients with a non-ovarian primary tumor with a metastasis to the ovary) and for both subgroups of HGSOC (n=62) and non-HGSOC cases (n=30). Although both scores were significantly correlated in general (Spearman's rho = 0.58; p-value < 0.05), this correlation was less pronounced in the non-HGSOC subgroup (Figure 4A; Spearman's rho = 0.64 for HGSOC and 0.47 for non-HGSOC). By visually inspecting the plots, we noticed a number of patients (n=11) with an elevated nucleosome score (>0.25) but a baseline genome-wide z-score in the non-HGSOC subgroups (Figure 4A). *Vice versa,* only one patient presented with a low nucleosome (<0.25) but high (>2.5) genome-wide z-score. One patient had a high nucleosome and genome-wide z-score, while 17 patients had both a low nucleosome and genome-wide z-score, respectively. When performing LC-WGS on 19 DNA samples that were available from matching non-HGSOC tumors, we could indeed observe low levels of genome-wide aneuploidy compared to HGSOC tumors (Figure 4B). Figure 4C illustrates this observation for a low-grade serous (LGSOC), mucinous (MUCOC) and non-epithelial (NEOC) ovarian carcinoma sample. These profiles were different from HGSOC tumors, which generally show very high instability (Figure 4D). The low chromosomal instability of non-HGSOC tumors was similarly reflected in a low genome-wide z-score in cfDNA; nevertheless, a higher proportion of these patients showed an increased nucleosome score (Figure 4A).Particularly, for the 3 non-HGSOC examples, the nucleosome score exceeded 0.25 (Figure 4D).

Interestingly, although we only assessed 8 cfDNA samples from invasive cancer patients with a metastasis to the ovary, 4 of these exhibited a high nucleosome score and only 2 presented with a high genome-wide z-score. The sample size is insufficient for inference; however, given the fact that many cancer types display less chromosomal instability than HGSOC, these results may be indicative of the nucleosome score being a more generic method to detect tumor lesions based on cfDNA.

### 1.3. DISCUSSION

Here, we performed LC-WGS of plasma cfDNA and generated a nucleosome footprinting score, which for each cfDNA sample measures the overall deviation in nucleosome footprints compared to those observed in healthy individuals. As nucleosome patterns are cell-type specific, a high nucleosome score in a cfDNA sample likely reflects a change in the contribution of cell types to the cfDNA fraction in a patient. In cancer patients, where highly variable levels of tumor-derived DNA contribute to the cfDNA fraction, elevated nucleosome score could therefore predict the presence of a malignant tumor. In 271 cfDNA samples from patients presenting with an adnexal mass, we indeed observed higher nucleosome scores for patients with invasive disease relative to those presenting with benign or borderline disease. Adnexal masses are very frequent, with some studies reporting a lifetime risk of 5-10% for women to undergo surgery for a suspected ovarian malignancy (Curtin 1994, Gynecol Oncol 55:S42-S46). Typically, during follow-up of these adnexal masses, gynaecologists are confronted with a diagnostic dilemma, as they need to carefully balance the disadvantage of undergoing surgery (i.e., risk of complications, loss of fertility and health-economic considerations) against the risk of missing the diagnosis of an invasive tumor. Since sequentially and invasively obtaining tumor biopsies from adnexal masses to assess potential malignancy is not a clinical option, there is a need to develop non-invasive biomarkers that could differentiate between benign versus malignant adnexal masses. Numerous efforts to develop such biomarkers have already been made. For instance, the ADNEX risk model developed by the International Ovarian Tumor Analysis (IOTA) group estimates the probability that an adnexal mass is benign, borderline, stage I cancer, stage II-IV cancer, or secondary metastatic cancer based on clinical and ultrasound data (Van Calster et al. 2014, BMJ 349:1-14). This model currently represents a clinical standard to predict ovarian malignancy, but as cfDNA-based tests are gaining momentum in clinical practice, an emerging question is whether existing predictive models could be further improved by implementing additional cfDNA-based tests.

Deep-sequencing of cfDNA and subsequent size distribution analyses have provided new insights in the biology of cfDNA (Snyder et al. 2016, Cell 164:57-68; Jiang et al. 2015, PNAS 112:E1317-E1325; Mouliere et al. 2017, BioRxiv doi:10.1101/134437). For instance, it was shown that cfDNA fragments originate from nucleosome-bound DNA, which is protected from degradation by nucleases. Although genomic nucleosome positions are highly dynamic, it appears that the overall nucleosome landscape is specific for each cell type, cell state and tissue (Corces et al. 2018, Science 362:eaav1898; Scott-Browne et al. 2016, Immunity 45:1327-1340). Consequently, we can use nucleosome footprints in cfDNA to quantify the contribution of each tissue to the cfDNA. For instance, using 76 expression sets of human cell lines and tissues as a reference, Snyder et al. were able to demonstrate that tumor tissue contributes to cfDNA in 5 selected patients with advanced-stage cancer (Snyder et al. 2016, Cell 164:57-68). A similar approach was used in the context of prenatal diagnosis, where a different cfDNA fragmentation pattern between maternal and fetal-derived cfDNA was leveraged to calculate the fraction of fetal DNA in cfDNA from pregnant women (Straver et al. 2016, Prenat Diagn 36:614-621).

In this study, we centered single-end sequencing reads derived from LC-WGS on a map of reference nucleosome positions and we observed that the distribution of the start positions of each read differed between a reference set of healthy individuals and a cohort of relapsed HGSOC patients with high ctDNA load. This suggests that a deviation in nucleosome footprints, associated with the presence of an invasive carcinoma, can be inferred from cfDNA-based LC-WGS data. When assigning nucleosome scores, which reflect a numeric read-out of this deviation, to each sample from a large cohort of 271 cfDNA samples obtained from patients with adnexal masses, we indeed found that the nucleosome score was elevated in patients with a malignancy compared to those with a benign lesion. Interestingly, we previously reported how chromosomal instability distinguishes HGSOC from women with benign adnexal masses using LC-WGS (Vanderstichele et al. 2014, Clin Cancer Res 23:2223-2231). Compared to the genome-wide z-score, which was similarly increased in patients with a malignancy, the nucleosome score had a weaker performance. However, we previously also demonstrated that the genome-wide z-score fails to reliably detect other ovarian cancer histologies characterized by less chromosomal instability. Indeed, in non-HGSOC patients, the performance of the genome-wide z-score dropped considerably. The nucleosome score, however, performed better to identify non-HGSOC patients. This is a quite interesting observation as both the nucleosome and genome-wide z-score can be derived from the same LC-WGS data. As such, LC-WGS of cfDNA represents a single diagnostic test that generates 2 independent and complementary diagnostic read-outs.

As mentioned, the nucleosome score quantifies a shift from the average cfDNA patterns of healthy individuals. These shifts most likely reflect the contribution of other tissues to the cfDNA pool in plasma. However, it is agnostic to which cell types are causing the deviation. As such, we are unable to investigate whether the deviation in nucleosome footprints is caused by tumor-derived cfDNA or whether the deviation is possibly also caused by other non-tumoral cells contributing cfDNA to the plasma. Indeed, in cancer patients there is also a major shift in the abundance and type of circulating immune cells. Changes in the levels of various circulating leukocytes have for instance been observed in ovarian cancer patients, while moreover, these changes are of important prognostic relevance (Baert et al. 2017, Gynecol Oncol Reports 19:57-58). Additionally, patients with other disease, such as autoimmune disease patients (e.g., lupus or multiple sclerosis) or patients with liver disease, a myocardial infarction or a kidney transplantation may also be characterized by a different composition of cell types contributing to the cfDNA (Sun et al. 2015, PNAS 112:E5503-E5512), which may be reflected in the nucleosome footprint because of differences in chromatin landscapes between these cell types (Snyder et al. 2016, Cell 164:57-68).

Based on our observations, several questions emerge. A first interesting question is how to integrate the genome-wide z-score and nucleosome score in a potential clinical setting. Ultrasonography is capable of correctly distinguishing most HGSOC tumors from benign cysts, but often additional confirmation is needed. Hence, there could be a diagnostic opportunity for both scores in combination with ultrasonography. As such, prediction models such as the ADNEX risk model, which combines ultrasound and clinical variables, could be extended with cfDNA-based scores. Additional research is needed to determine how these scores have to be integrated in the current ADNEX model and how this will add to the predictive power of the ADNEX model. In addition, it remains to be investigated how different sets of control samples will affect the scores and their performances. Indeed, when using different control sets, scores may deviate, possibly leading to different risk estimates. Such heterogeneity is undesirable, and efforts may be required to control for this. A second question is related to increasing the signal-to-noise ratio of the nucleosome score that we developed. Indeed, we pooled genomic regions and assessed the average deviation of nucleosome patterns across the entire genome. We anticipate, however, that focusing the score on genomic regions specifically altered in HGSOC or non-HGSOC could still improve the performance. Additional datasets and more in-depth bio-informatics analyses are needed to explore this in future work. Technical improvements such as higher sequencing coverage or paired-end sequencing, could also still contribute to an overall improved performance.

In conclusion, we here show that LC-WGS generates 2 biomarker read-outs that yield complementary diagnostic information. Particularly, we confirm that the genome-wide z-score efficiently detects chromosomal instability of HGSOC tumors in plasma cfDNA, while additionally, we show that non-HGSOC patients are often missed when using the genome wide z-score. The latter patients are, however, more effectively detected using nucleosome footprinting of cfDNA.

**EXAMPLE 2. Methylation of cfDNA of patients with different stages of ovarian cancer**

**2.1. Samples**

Samples include control samples obtained from healthy subjects, and samples obtained from subjects exhibiting a benign ovarian tumor, a borderline ovarian tumor (or BOT), or an invasive ovarian tumor. Samples obtained from subjects exhibiting invasive ovarian tumors were further subdivided in a group corresponding to high-grade serous ovarian cancer (HGSOC) and non-high-grade serous ovarian cancer (non-HGSOC).

**2.2. Target MethylSeq of cfDNA.** DNA methylation of plasma-derived cfDNA and tumor tissue is profiled by targeted bisulfite sequencing (Target Methyl-Seq), using an in-house developed protocol to reliably assess the methylation status of low concentrations of heavily fragmented cfDNA in body fluids. Briefly, rather than subjecting dsDNA to bisulfite conversion after DNA library preparation, low amounts of input ctDNA (2-40 ng DNA) are first subjected to bisulfite conversion. Subsequently, the Accel-NGS® kit (Swift BioSciences®) is used to generate functional double-stranded, bisulfite-converted, indexed libraries. Subsequently, a subset of the genome is captured by a pool of 25,399 customized capture probes (SeqCap® Epi, Roche®), which were designed to specifically target CpGs that are lowly methylated in blood from healthy individuals. Particularly, 44,341 target CpGs were initially selected that are unmethylated or nearly unmethylated (mean average methylation β-value < 0.03 across > 600 healthy individuals). Target regions surrounding the 44,341 CpGs resulted in a design of 25,399 SeqCap® Epi capture probes with a median size of 149 bps (range 59-1,037 bps).

Captured libraries are then sequenced on an Illumina HiSeq4000 (paired-end 2*150 bp reads) with a median coverage >150x. The sequencing reads generated by the Accel-NGS® kit were trimmed using TrimGalore. The trimmed FASTQ files were mapped on a bisulfite-converted human genome (GRCh37) using Bismark. Coverage files have been extracted using Bismark's methylation extractor which quantify for each CpG position the degree of methylation.

**2.3. Selection of ovarian cancer specific CpGs**

To obtain ovarian cancer-specific probes, a HGSOC tissue dataset and a healthy control plasma sample dataset were compared, yielding 19,217 CpGs that were significantly hypermethylated in HGSOC compared to healthy samples using a minimum absolute beta value difference of 0.25 and a false discovery rate (FDR)- adjusted P-value smaller than 0.01. In this step, no information is obtained on which of the CpGs hypermethylated in tumor tissue are detectable in a liquid biopsy sample (cfDNA) of an ovarian cancer patient (not all DNA methylation markers identified as suitable for determining the presence of a tumor in tissue biopsies are suitable for determining the presence of a tumor in a liquid biopsy, e.g. Melnikov et al. 2009, J Mol Diagnostics 11:60-65).

Of these 19,217 identified hypermethylated CpGs, 616 are targeted by the 25,399 designed capture probes. As outlined above, these 616 CpGs are present in but are unmethylated or nearly unmethylated in plasma of healthy individuals (mean average methylation β-value < 0.03 across > 600 healthy individuals), and therewith constitute ideal markers for determining methylation as basis for diagnosis of ovarian cancer: detection of one or more of these 616 CpGs in methylated form in cfDNA of a subject is indicative of the likelihood of that subject to have ovarian cancer. Alternatively, or in addition, the higher the frequency of occurrence of one (or more) of these 616 CpGs in a sample when compared to the frequency of occurrence of the corresponding non-methylated CpG, the higher the likelihood of the subject having ovarian cancer. Alternatively, or in addition, such frequency of occurrence can provide information on the tumor load or on the stage of the tumor. These 616 CpGs are listed in Table 3, and are covered by 400 of the designed capture probes.

**Table 3.** List of 616 ovarian cancer specific CpGs not methylated in cfDNA of healthy subjects defined by their start position ("start") on the indicated chromosome number ("chr#"). A general numbering ("#") is included in the first column.

| # | chr# | start |
|---|---|---|
| 1 | chr1 | 969254 |
| 2 | chr1 | 969257 |
| 3 | chr1 | 1566687 |
| 4 | chr1 | 1566699 |
| 5 | chr1 | 2222419 |
| 6 | chr1 | 2222440 |
| 7 | chr1 | 2232469 |
| 8 | chr1 | 2232481 |
| 9 | chr1 | 6086254 |
| 10 | chr1 | 6086275 |
| 11 | chr1 | 6520354 |
| 12 | chr1 | 6526129 |
| 13 | chr1 | 9714397 |
| 14 | chr1 | 16489115 |
| 15 | chr1 | 25349006 |
| 16 | chr1 | 27687232 |
| 17 | chr1 | 35351340 |
| 18 | chr1 | 36042986 |
| 19 | chr1 | 36043002 |
| 20 | chr1 | 36043014 |
| 21 | chr1 | 36043084 |
| 22 | chr1 | 42217077 |
| 23 | chr1 | 47701206 |
| 24 | chr1 | 47911449 |
| 25 | chr1 | 50513766 |
| 26 | chr1 | 50513870 |
| 27 | chr1 | 63790044 |
| 28 | chr1 | 67217950 |
| 29 | chr1 | 67217984 |
| 30 | chr1 | 86081680 |
| 31 | chr1 | 90309410 |
| 32 | chr1 | 91182856 |
| 33 | chr1 | 91301461 |
| 34 | chr1 | 91301484 |
| 35 | chr1 | 91301651 |
| 36 | chr1 | 91301731 |
| 37 | chr1 | 110186027 |
| 38 | chr1 | 110186044 |

(continued)

| # | chr# | start |
|---|---|---|
| 39 | chr1 | 110611465 |
| 40 | chr1 | 113051846 |
| 41 | chr1 | 113051925 |
| 42 | chr1 | 119535619 |
| 43 | chr1 | 119535693 |
| 44 | chr1 | 151811364 |
| 45 | chr1 | 160983767 |
| 46 | chr1 | 192544716 |
| 47 | chr1 | 197888880 |
| 48 | chr1 | 200842890 |
| 49 | chr1 | 202129865 |
| 50 | chr1 | 219347279 |
| 51 | chr1 | 219347340 |
| 52 | chr1 | 219347410 |
| 53 | chr1 | 219347458 |
| 54 | chr1 | 219834537 |
| 55 | chr1 | 219834581 |
| 56 | chr1 | 221050459 |
| 57 | chr1 | 221050491 |
| 58 | chr1 | 224805753 |
| 59 | chr1 | 228463634 |
| 60 | chr1 | 231115871 |
| 61 | chr1 | 235099151 |
| 62 | chr2 | 19551750 |
| 63 | chr2 | 19551789 |
| 64 | chr2 | 25439110 |
| 65 | chr2 | 25496390 |
| 66 | chr2 | 25500046 |
| 67 | chr2 | 38302230 |
| 68 | chr2 | 43019854 |
| 69 | chr2 | 45231382 |
| 70 | chr2 | 63281069 |
| 71 | chr2 | 63281139 |
| 72 | chr2 | 63283939 |
| 73 | chr2 | 63283967 |
| 74 | chr2 | 63284066 |
| 75 | chr2 | 63284132 |
| 76 | chr2 | 71192445 |
| 77 | chr2 | 85804732 |

(continued)

| # | chr# | start |
|---|---|---|
| 78 | chr2 | 86263224 |
| 79 | chr2 | 86263270 |
| 80 | chr2 | 96990861 |
| 81 | chr2 | 99439533 |
| 82 | chr2 | 99439883 |
| 83 | chr2 | 105459097 |
| 84 | chr2 | 105459164 |
| 85 | chr2 | 127413505 |
| 86 | chr2 | 127414108 |
| 87 | chr2 | 127414381 |
| 88 | chr2 | 127414455 |
| 89 | chr2 | 127839539 |
| 90 | chr2 | 157177008 |
| 91 | chr2 | 162279964 |
| 92 | chr2 | 162281111 |
| 93 | chr2 | 171573419 |
| 94 | chr2 | 200329872 |
| 95 | chr2 | 200334001 |
| 96 | chr2 | 202125212 |
| 97 | chr2 | 219736250 |
| 98 | chr2 | 219736549 |
| 99 | chr2 | 223154140 |
| 100 | chr2 | 228736253 |
| 101 | chr2 | 228736258 |
| 102 | chr2 | 228736449 |
| 103 | chr2 | 233925001 |
| 104 | chr2 | 238600061 |
| 105 | chr2 | 242743214 |
| 106 | chr3 | 4910524 |
| 107 | chr3 | 9904411 |
| 108 | chr3 | 9904557 |
| 109 | chr3 | 43431343 |
| 110 | chr3 | 50310766 |
| 111 | chr3 | 50312913 |
| 112 | chr3 | 50378529 |
| 113 | chr3 | 51990575 |
| 114 | chr3 | 61550379 |
| 115 | chr3 | 62861055 |
| 116 | chr3 | 113252289 |

(continued)

| # | chr# | start |
|---|---|---|
| 117 | chr3 | 121379745 |
| 118 | chr3 | 121379777 |
| 119 | chr3 | 121379791 |
| 120 | chr3 | 129693489 |
| 121 | chr3 | 141516232 |
| 122 | chr3 | 147126753 |
| 123 | chr3 | 147126961 |
| 124 | chr3 | 147127010 |
| 125 | chr3 | 147127012 |
| 126 | chr3 | 147127662 |
| 127 | chr3 | 147128123 |
| 128 | chr3 | 147128157 |
| 129 | chr3 | 147130477 |
| 130 | chr3 | 147130536 |
| 131 | chr3 | 147140847 |
| 132 | chr3 | 147140880 |
| 133 | chr3 | 147140930 |
| 134 | chr3 | 150996297 |
| 135 | chr3 | 157261086 |
| 136 | chr3 | 157812475 |
| 137 | chr3 | 160167990 |
| 138 | chr3 | 171175950 |
| 139 | chr3 | 171176016 |
| 140 | chr3 | 181444870 |
| 141 | chr3 | 183273557 |
| 142 | chr3 | 184099432 |
| 143 | chr3 | 187458630 |
| 144 | chr3 | 194408901 |
| 145 | chr4 | 996175 |
| 146 | chr4 | 4867164 |
| 147 | chr4 | 13524143 |
| 148 | chr4 | 25656865 |
| 149 | chr4 | 38807337 |
| 150 | chr4 | 38807382 |
| 151 | chr4 | 40198392 |
| 152 | chr4 | 40858965 |
| 153 | chr4 | 56686117 |
| 154 | chr4 | 79642491 |
| 155 | chr4 | 85402497 |

(continued)

| # | chr# | start |
|---|---|---|
| 156 | chr4 | 90757351 |
| 157 | chr4 | 90757452 |
| 158 | chr4 | 120061211 |
| 159 | chr4 | 140656926 |
| 160 | chr4 | 153601329 |
| 161 | chr4 | 154713748 |
| 162 | chr4 | 155411806 |
| 163 | chr4 | 169799087 |
| 164 | chr4 | 174430487 |
| 165 | chr4 | 174452835 |
| 166 | chr4 | 174459412 |
| 167 | chr4 | 175133103 |
| 168 | chr4 | 175133151 |
| 169 | chr4 | 183062460 |
| 170 | chr5 | 2038528 |
| 171 | chr5 | 14810180 |
| 172 | chr5 | 40681137 |
| 173 | chr5 | 40681893 |
| 174 | chr5 | 42952369 |
| 175 | chr5 | 42994123 |
| 176 | chr5 | 42994709 |
| 177 | chr5 | 42994776 |
| 178 | chr5 | 43019660 |
| 179 | chr5 | 54398555 |
| 180 | chr5 | 94982330 |
| 181 | chr5 | 115152413 |
| 182 | chr5 | 115152420 |
| 183 | chr5 | 115152431 |
| 184 | chr5 | 115152485 |
| 185 | chr5 | 115152492 |
| 186 | chr5 | 115152494 |
| 187 | chr5 | 122430235 |
| 188 | chr5 | 132150117 |
| 189 | chr5 | 134363516 |
| 190 | chr5 | 134363562 |
| 191 | chr5 | 138677027 |
| 192 | chr5 | 140797234 |
| 193 | chr5 | 142814934 |
| 194 | chr5 | 159894937 |

(continued)

| # | chr# | start |
|---|---|---|
| 195 | chr5 | 169724768 |
| 196 | chr5 | 169724791 |
| 197 | chr5 | 169724803 |
| 198 | chr5 | 169724831 |
| 199 | chr5 | 172672959 |
| 200 | chr5 | 176302913 |
| 201 | chr5 | 176302976 |
| 202 | chr5 | 176784688 |
| 203 | chr5 | 176784715 |
| 204 | chr5 | 176784719 |
| 205 | chr6 | 475074 |
| 206 | chr6 | 7541066 |
| 207 | chr6 | 10422322 |
| 208 | chr6 | 13274180 |
| 209 | chr6 | 17281015 |
| 210 | chr6 | 26044405 |
| 211 | chr6 | 27107097 |
| 212 | chr6 | 27107145 |
| 213 | chr6 | 28457210 |
| 214 | chr6 | 29617549 |
| 215 | chr6 | 29894315 |
| 216 | chr6 | 29894619 |
| 217 | chr6 | 29894679 |
| 218 | chr6 | 29895116 |
| 219 | chr6 | 29943408 |
| 220 | chr6 | 29943414 |
| 221 | chr6 | 29943425 |
| 222 | chr6 | 29943455 |
| 223 | chr6 | 29943480 |
| 224 | chr6 | 30458161 |
| 225 | chr6 | 30646949 |
| 226 | chr6 | 30652347 |
| 227 | chr6 | 30652376 |
| 228 | chr6 | 30652396 |
| 229 | chr6 | 30652399 |
| 230 | chr6 | 30653659 |
| 231 | chr6 | 30653732 |
| 232 | chr6 | 30653736 |
| 233 | chr6 | 30653755 |

(continued)

| # | chr# | start |
|---|---|---|
| 234 | chr6 | 30653799 |
| 235 | chr6 | 30711580 |
| 236 | chr6 | 31765567 |
| 237 | chr6 | 31765590 |
| 238 | chr6 | 31765614 |
| 239 | chr6 | 31765619 |
| 240 | chr6 | 31765634 |
| 241 | chr6 | 31783468 |
| 242 | chr6 | 31866072 |
| 243 | chr6 | 33245488 |
| 244 | chr6 | 33245490 |
| 245 | chr6 | 33245537 |
| 246 | chr6 | 33245541 |
| 247 | chr6 | 34203153 |
| 248 | chr6 | 35108921 |
| 249 | chr6 | 35109121 |
| 250 | chr6 | 41528623 |
| 251 | chr6 | 41528750 |
| 252 | chr6 | 41528785 |
| 253 | chr6 | 42738967 |
| 254 | chr6 | 42739021 |
| 255 | chr6 | 42739049 |
| 256 | chr6 | 43337775 |
| 257 | chr6 | 85482570 |
| 258 | chr6 | 85482612 |
| 259 | chr6 | 105388668 |
| 260 | chr6 | 105388694 |
| 261 | chr6 | 105388731 |
| 262 | chr6 | 105400985 |
| 263 | chr6 | 105400993 |
| 264 | chr6 | 106429316 |
| 265 | chr6 | 106960491 |
| 266 | chr6 | 108495678 |
| 267 | chr6 | 134210946 |
| 268 | chr6 | 170403264 |
| 269 | chr6 | 170463397 |
| 270 | chr7 | 26193032 |
| 271 | chr7 | 27205224 |
| 272 | chr7 | 27205230 |

(continued)

| # | chr# | start |
|---|---|---|
| 273 | chr7 | 27205262 |
| 274 | chr7 | 27213971 |
| 275 | chr7 | 39649290 |
| 276 | chr7 | 42267719 |
| 277 | chr7 | 42267747 |
| 278 | chr7 | 45018757 |
| 279 | chr7 | 45018789 |
| 280 | chr7 | 45018849 |
| 281 | chr7 | 45961537 |
| 282 | chr7 | 50348255 |
| 283 | chr7 | 73021662 |
| 284 | chr7 | 96652153 |
| 285 | chr7 | 96652245 |
| 286 | chr7 | 102067162 |
| 287 | chr7 | 121939827 |
| 288 | chr7 | 150211761 |
| 289 | chr7 | 150211811 |
| 290 | chr7 | 150211820 |
| 291 | chr7 | 150211855 |
| 292 | chr8 | 22961088 |
| 293 | chr8 | 23563970 |
| 294 | chr8 | 23564025 |
| 295 | chr8 | 70982285 |
| 296 | chr8 | 72754469 |
| 297 | chr8 | 97157856 |
| 298 | chr8 | 97157878 |
| 299 | chr8 | 120651398 |
| 300 | chr8 | 145106246 |
| 301 | chr8 | 145106299 |
| 302 | chr9 | 100616469 |
| 303 | chr9 | 100616607 |
| 304 | chr9 | 122132103 |
| 305 | chr9 | 139428006 |
| 306 | chr10 | 8097331 |
| 307 | chr10 | 8097354 |
| 308 | chr10 | 11207527 |
| 309 | chr10 | 11207969 |
| 310 | chr10 | 21807252 |
| 311 | chr10 | 22541995 |

(continued)

| # | chr# | start |
|---|---|---|
| 312 | chr10 | 22542024 |
| 313 | chr10 | 22634142 |
| 314 | chr10 | 22634199 |
| 315 | chr10 | 22634218 |
| 316 | chr10 | 22634224 |
| 317 | chr10 | 22634226 |
| 318 | chr10 | 22634432 |
| 319 | chr10 | 22634439 |
| 320 | chr10 | 30316432 |
| 321 | chr10 | 43893015 |
| 322 | chr10 | 70847350 |
| 323 | chr10 | 70847399 |
| 324 | chr10 | 70847430 |
| 325 | chr10 | 100993553 |
| 326 | chr10 | 102587110 |
| 327 | chr10 | 102998662 |
| 328 | chr10 | 102998762 |
| 329 | chr10 | 103536348 |
| 330 | chr10 | 105036645 |
| 331 | chr10 | 118891601 |
| 332 | chr10 | 118891670 |
| 333 | chr10 | 118891706 |
| 334 | chr11 | 268950 |
| 335 | chr11 | 415080 |
| 336 | chr11 | 415088 |
| 337 | chr11 | 415111 |
| 338 | chr11 | 627152 |
| 339 | chr11 | 627175 |
| 340 | chr11 | 637170 |
| 341 | chr11 | 637173 |
| 342 | chr11 | 637175 |
| 343 | chr11 | 720859 |
| 344 | chr11 | 2292691 |
| 345 | chr11 | 2292751 |
| 346 | chr11 | 2322729 |
| 347 | chr11 | 2322741 |
| 348 | chr11 | 2322781 |
| 349 | chr11 | 2322802 |
| 350 | chr11 | 2322808 |

(continued)

| # | chr# | start |
|---|---|---|
| 351 | chr11 | 2889809 |
| 352 | chr11 | 2889875 |
| 353 | chr11 | 31825226 |
| 354 | chr11 | 31846844 |
| 355 | chr11 | 31846849 |
| 356 | chr11 | 44325823 |
| 357 | chr11 | 47416487 |
| 358 | chr11 | 47416535 |
| 359 | chr11 | 61062962 |
| 360 | chr11 | 63974123 |
| 361 | chr11 | 63974131 |
| 362 | chr11 | 63974153 |
| 363 | chr11 | 63974162 |
| 364 | chr11 | 65647270 |
| 365 | chr11 | 65661516 |
| 366 | chr11 | 66885276 |
| 367 | chr11 | 71952131 |
| 368 | chr11 | 76382128 |
| 369 | chr11 | 76382149 |
| 370 | chr11 | 78673073 |
| 371 | chr11 | 78673100 |
| 372 | chr11 | 82443614 |
| 373 | chr11 | 94884121 |
| 374 | chr11 | 119293861 |
| 375 | chr11 | 119293863 |
| 376 | chr11 | 119293869 |
| 377 | chr11 | 128565168 |
| 378 | chr11 | 129243219 |
| 379 | chr12 | 3861567 |
| 380 | chr12 | 6184355 |
| 381 | chr12 | 6665288 |
| 382 | chr12 | 6665330 |
| 383 | chr12 | 6665335 |
| 384 | chr12 | 6665370 |
| 385 | chr12 | 6665424 |
| 386 | chr12 | 6665447 |
| 387 | chr12 | 6881590 |
| 388 | chr12 | 6881595 |
| 389 | chr12 | 6881601 |

(continued)

| # | chr# | start |
|---|---|---|
| 390 | chr12 | 6881624 |
| 391 | chr12 | 6881629 |
| 392 | chr12 | 12867753 |
| 393 | chr12 | 14135228 |
| 394 | chr12 | 25055967 |
| 395 | chr12 | 46767665 |
| 396 | chr12 | 46767683 |
| 397 | chr12 | 46767747 |
| 398 | chr12 | 51640305 |
| 399 | chr12 | 51717865 |
| 400 | chr12 | 51718088 |
| 401 | chr12 | 51718112 |
| 402 | chr12 | 52652220 |
| 403 | chr12 | 52652462 |
| 404 | chr12 | 53718427 |
| 405 | chr12 | 54088934 |
| 406 | chr12 | 54389934 |
| 407 | chr12 | 54398765 |
| 408 | chr12 | 54398809 |
| 409 | chr12 | 54812000 |
| 410 | chr12 | 54812085 |
| 411 | chr12 | 57618943 |
| 412 | chr12 | 57618965 |
| 413 | chr12 | 58013414 |
| 414 | chr12 | 58013458 |
| 415 | chr12 | 58013475 |
| 416 | chr12 | 58013487 |
| 417 | chr12 | 58013517 |
| 418 | chr12 | 58013539 |
| 419 | chr12 | 58013569 |
| 420 | chr12 | 58013636 |
| 421 | chr12 | 58013645 |
| 422 | chr12 | 58013651 |
| 423 | chr12 | 58013687 |
| 424 | chr12 | 58021569 |
| 425 | chr12 | 58135973 |
| 426 | chr12 | 58135981 |
| 427 | chr12 | 63207179 |
| 428 | chr12 | 66582780 |

(continued)

| # | chr# | start |
|---|---|---|
| 429 | chr12 | 85667616 |
| 430 | chr12 | 85672623 |
| 431 | chr12 | 85673200 |
| 432 | chr12 | 85673221 |
| 433 | chr12 | 105114327 |
| 434 | chr12 | 105114399 |
| 435 | chr12 | 114847578 |
| 436 | chr12 | 128850550 |
| 437 | chr12 | 128850557 |
| 438 | chr12 | 128850696 |
| 439 | chr13 | 20806730 |
| 440 | chr13 | 20875915 |
| 441 | chr13 | 24844846 |
| 442 | chr13 | 24844852 |
| 443 | chr13 | 24844880 |
| 444 | chr13 | 24844896 |
| 445 | chr13 | 28527255 |
| 446 | chr13 | 30945876 |
| 447 | chr13 | 41556559 |
| 448 | chr13 | 53313174 |
| 449 | chr13 | 95360278 |
| 450 | chr13 | 98794470 |
| 451 | chr13 | 112722719 |
| 452 | chr14 | 22362410 |
| 453 | chr14 | 24045549 |
| 454 | chr14 | 29254853 |
| 455 | chr14 | 38724648 |
| 456 | chr14 | 65565498 |
| 457 | chr14 | 70653964 |
| 458 | chr14 | 102027797 |
| 459 | chr14 | 102172296 |
| 460 | chr14 | 105512071 |
| 461 | chr14 | 105512213 |
| 462 | chr14 | 105512268 |
| 463 | chr14 | 105512306 |
| 464 | chr15 | 40600265 |
| 465 | chr15 | 40600279 |
| 466 | chr15 | 40600284 |
| 467 | chr15 | 40600635 |

(continued)

| # | chr# | start |
|---|---|---|
| 468 | chr15 | 41787780 |
| 469 | chr15 | 44092606 |
| 470 | chr15 | 51385891 |
| 471 | chr15 | 57592222 |
| 472 | chr15 | 65689152 |
| 473 | chr15 | 75081572 |
| 474 | chr15 | 81589248 |
| 475 | chr15 | 82555336 |
| 476 | chr15 | 85360319 |
| 477 | chr16 | 3265396 |
| 478 | chr16 | 3265467 |
| 479 | chr16 | 11327141 |
| 480 | chr16 | 28996329 |
| 481 | chr16 | 28996358 |
| 482 | chr16 | 28996362 |
| 483 | chr16 | 30103978 |
| 484 | chr16 | 50699167 |
| 485 | chr16 | 50699197 |
| 486 | chr16 | 54970523 |
| 487 | chr16 | 55363058 |
| 488 | chr16 | 55544136 |
| 489 | chr16 | 56669681 |
| 490 | chr16 | 56669726 |
| 491 | chr16 | 67197150 |
| 492 | chr16 | 67197186 |
| 493 | chr16 | 67199463 |
| 494 | chr16 | 67199830 |
| 495 | chr16 | 72821498 |
| 496 | chr16 | 89006335 |
| 497 | chr16 | 89034778 |
| 498 | chr16 | 89034797 |
| 499 | chr16 | 89778247 |
| 500 | chr17 | 2699505 |
| 501 | chr17 | 2699542 |
| 502 | chr17 | 2699553 |
| 503 | chr17 | 2699689 |
| 504 | chr17 | 2699706 |
| 505 | chr17 | 2699718 |
| 506 | chr17 | 4648566 |

(continued)

| # | chr# | start |
|---|---|---|
| 507 | chr17 | 4648580 |
| 508 | chr17 | 6358928 |
| 509 | chr17 | 7341641 |
| 510 | chr17 | 7906098 |
| 511 | chr17 | 8770954 |
| 512 | chr17 | 8771003 |
| 513 | chr17 | 8771055 |
| 514 | chr17 | 8771114 |
| 515 | chr17 | 9143754 |
| 516 | chr17 | 16284307 |
| 517 | chr17 | 27940509 |
| 518 | chr17 | 27942115 |
| 519 | chr17 | 32964596 |
| 520 | chr17 | 32964615 |
| 521 | chr17 | 35303330 |
| 522 | chr17 | 40440904 |
| 523 | chr17 | 40440916 |
| 524 | chr17 | 42287971 |
| 525 | chr17 | 53341243 |
| 526 | chr17 | 56408197 |
| 527 | chr17 | 56408804 |
| 528 | chr17 | 56409518 |
| 529 | chr17 | 56409534 |
| 530 | chr17 | 58498977 |
| 531 | chr17 | 59480674 |
| 532 | chr17 | 59480730 |
| 533 | chr17 | 59530104 |
| 534 | chr17 | 61778515 |
| 535 | chr17 | 61778550 |
| 536 | chr17 | 73749654 |
| 537 | chr17 | 73749694 |
| 538 | chr17 | 75462189 |
| 539 | chr17 | 76128481 |
| 540 | chr17 | 76128556 |
| 541 | chr17 | 80186266 |
| 542 | chr17 | 80186273 |
| 543 | chr17 | 80186275 |
| 544 | chr17 | 80186336 |
| 545 | chr17 | 80358829 |

(continued)

| # | chr# | start |
|---|---|---|
| 546 | chr17 | 80358850 |
| 547 | chr17 | 80358876 |
| 548 | chr17 | 80407590 |
| 549 | chr18 | 501137 |
| 550 | chr18 | 3499253 |
| 551 | chr18 | 29265840 |
| 552 | chr18 | 29265842 |
| 553 | chr18 | 46461202 |
| 554 | chr18 | 77277583 |
| 555 | chr19 | 1069320 |
| 556 | chr19 | 1074926 |
| 557 | chr19 | 1851882 |
| 558 | chr19 | 1851995 |
| 559 | chr19 | 2302995 |
| 560 | chr19 | 3178844 |
| 561 | chr19 | 6481819 |
| 562 | chr19 | 6481826 |
| 563 | chr19 | 6481857 |
| 564 | chr19 | 11354132 |
| 565 | chr19 | 13124215 |
| 566 | chr19 | 14550997 |
| 567 | chr19 | 14550999 |
| 568 | chr19 | 15342915 |
| 569 | chr19 | 17346507 |
| 570 | chr19 | 17462455 |
| 571 | chr19 | 18981378 |
| 572 | chr19 | 50004444 |
| 573 | chr19 | 51232007 |
| 574 | chr19 | 52104632 |
| 575 | chr19 | 55593580 |
| 576 | chr20 | 3052692 |
| 577 | chr20 | 25129126 |
| 578 | chr20 | 25129296 |
| 579 | chr20 | 30639305 |
| 580 | chr20 | 35274595 |
| 581 | chr20 | 35274627 |
| 582 | chr20 | 35274639 |
| 583 | chr20 | 35274655 |
| 584 | chr20 | 44879700 |

(continued)

| # | chr# | start |
|---|---|---|
| 585 | chr20 | 55200146 |
| 586 | chr20 | 55965245 |
| 587 | chr20 | 56025557 |
| 588 | chr20 | 57224919 |
| 589 | chr20 | 61560627 |
| 590 | chr20 | 62369462 |
| 591 | chr21 | 36399226 |
| 592 | chr21 | 36399258 |
| 593 | chr21 | 36421467 |
| 594 | chr21 | 36421472 |
| 595 | chr21 | 36421503 |
| 596 | chr22 | 19711051 |
| 597 | chr22 | 24823455 |
| 598 | chr22 | 24823509 |
| 599 | chr22 | 24823514 |
| 600 | chr22 | 24823519 |
| 601 | chr22 | 24823554 |
| 602 | chr22 | 30662972 |
| 603 | chr22 | 30662987 |
| 604 | chr22 | 30662994 |
| 605 | chr22 | 30663007 |
| 606 | chr22 | 30663034 |
| 607 | chr22 | 30663041 |
| 608 | chr22 | 30663316 |
| 609 | chr22 | 37813098 |
| 610 | chr22 | 38477085 |
| 611 | chr22 | 44577211 |
| 612 | chr22 | 44577222 |
| 613 | chr22 | 44577265 |
| 614 | chr22 | 50623687 |
| 615 | chr22 | 50623692 |
| 616 | chr22 | 50986962 |

**2.4. Estimation of tumor content using linkage disequilibrium methylation pipeline.**

In one step to down-size the number of markers required to reliably detect the presence of ovarian cancer in a subject, downstream analysis was performed to calculate the methylation score of cfDNA in each sample (performed in R), using an in-house developed linkage disequilibrium bio-informatic pipeline based on co-methylation patterns of CpGs located in the tumor specific probe reads. It has been shown that adjacent CpG sites on the same DNA molecules can share similar methylation status (Lövkvist et al. 2016, Nucleic Acids Res 44:5123-5132). Until now, however, methylation studies in cancer settings have mainly focused on assessing individual CpG sites. Only recently, it was shown that there are methylation haplotypes, consisting of co-methylated CpG sites, which appear to provide for more sensitive tumor signals compared to the average methylation rate of an individual CpG site (Li et al. 2018, Nucleic Acids Res 46:e89).

The sequencing reads of our studied cohort samples were filtered based on their full overlap with the 400 ovarian tumor specific probe regions. To generate a methylation score of a plasma sample of interest, we first trained a model using 25 ovarian tumor tissue samples and 43 healthy control plasma samples, representing a tumor and healthy condition (see Figure 7). We restricted our analysis to the 400 ovarian tumor specific regions defined above and considered only reads containing at least 4 CpGs. Within each read, we identified all possible combinations of methylated versus unmethylated CpGs when at least 4 CpGs were covered by the read, and we kept only the patterns that are smaller than 200 bp and that are fully methylated. In addition, we restricted our analysis to regions containing at least 10X average coverage across all samples in the training set. For each filtered pattern that we detected in a read, we next determined the probability that each pattern would occur in the training set of either the tumor or healthy tissue by fitting the occurrence of each pattern to a beta-distribution. Specifically, for each pattern two coverage distributions were generated, one for the tumor and one for the healthy tissue. We kept the top 15% patterns with the largest average difference in the mean beta distribution of tumor versus healthy samples, and removed the patterns with 5% largest variance in tumor and in healthy distribution.

After this training step, we quantified the methylation score in cfDNA collected from plasma samples in a test set. We summed the likelihoods of all patterns relative to a hypothetical tumor content (theta) using Monte Carlo simulations. By simulating all possible theta values, from 0 (no tumor content) to 1 (only tumor content) with a stepsize of 0.001, we can find out which theta value has the highest likelihood and therefore is the most likely tumor content in the studied test sample. In other words, if all patterns correspond closely to those of the samples in the tumor training set, then the theta value with a maximum likelihood will have an estimated value near 1. However, if more patterns are more likely to resemble the healthy pattern training set, then the theta value with a maximum likelihood will be closer to 0. Thus, the theta value represents the estimated cfDNA tumor load in a sample based on co-methylation patterns, and is further referred to as a methylation score. The principles of the methylation pipeline are outlined in Figure 8.

Next, we constructed a probabilistic model with a 4-fold cross validation on a training set of 25 HGSOC tissue samples and 32 (3/4) plasma control samples, and a test set of 11 (1/4) plasma control samples, 55 ovarian cancer plasma samples (invasive, i.e. combination of HGSOC and non-HGSOC), 26 benign samples and 23 borderline ("BOT") samples. For the 4-fold cross validation, we randomly partitioned the control samples into 4 folds, and the results were then averaged to obtain a single predictive estimate. Receiver operation characteristic (ROC) curve were constructed and the corresponding area under the curve (AUC) values was calculated using the pROC package in R.

Out of the 4-fold cross validation, a set of 3205 read patterns common to all folds of the validation were compiled. Overlapping reads were further compiled into 211 clusters (such compilation is exemplified for CpG cluster #5 hereinafter). These clusters were defined by a start position defined by the first occurring CpG in the cluster, and by an end position defined by the last occurring CpG in the cluster. These 211 clusters, referred to as 211 CpG clusters, are listed in Table 4. Where available, information of the gene in which each cluster is (partially) located is included.

Thus, starting from the sequencing reads filtered based on their full overlap with the 400 ovarian tumor specific probes (these probes themselves selected for covering the 616 CpGs selected as described in Example 2.3.), a series of 211 CpG clusters comprising additional (relative to the starting 616 CpGs) co-methylated CpGs were constructed. Table 4 lists for each CpG cluster which of the initial 616 CpGs (as listed in Table 3) are comprised in the CpG cluster. For those CpG clusters not comprising one of the initial 616 CpGs, Table 4 lists those CpG(s) of the initial 616 CpGs that are comprised in a probe (also listed in Table 4) underlying the construction of the CpG cluster (see heading of Table 4; and, as example, explanation for CpG clusters # 93-95 hereinafter). The individual CpGs comprised within a CpG cluster are listed in Table 6. The individual CpGs comprised within a probe but outside a CpG cluster are listed in Table 8.

It is noted that, in the herein described 4-fold validation relying on the herein analyzed number of ovarian cancer samples, and the associated filtering of read patterns common to all folds of the validation, a subset of the starting 616 CpGs is not retained in the 211 CpG clusters - this does not invalidate the applicability of the non-retained CpGs but rather illustrates that the starting 616 CpGs can be used to identify further CpGs of diagnostic value (due to their behavior of co-methylation together with methylation of a CpG from the starting 616 CpGs retained in the cluster). This is further corroborated by the fact that, with very few exceptions, the CpGs not present in the CpG clusters and not comprised by a probe (listed in Table 7) are more frequently methylated in ovarian cancer cfDNA samples compared to methylation of these CpGs in healthy subject reference cfDNA (comparison of 25 HGSOC samples vs 49 healthy reference samples; see Table 7). Furthermore, those CpGs indicated in Table 4 as comprised in a probe but not in the corresponding CpG cluster (compiled in Table 8) are more often co-methylated with the CpGs in the CpG cluster in the ovarian cancer cfDNA samples compared to healthy reference cfDNA samples, which is further corroborated by the data in Table 7 which also includes a number of the CpGs listed in Table 8 (CpGs present in probe, not in CpG cluster) and 5 randomly selected CpGs of Table 6 (CpGs present in CpG cluster).

As each of the 211 CpG clusters is the result of rigorous selection and validation, detection, in a test sample, of at least one hypermethylated or fully methylated CpG cluster, or part thereof, selected from the clusters of Table 4 is indicative of the test sample having been obtained from a subject having ovarian cancer. Alternatively, or in addition, the higher the frequency of occurrence of such at least one hypermethylated or fully methylated CpG cluster, or part thereof, in a sample when compared to the frequency of occurrence of the corresponding non-fully methylated CpG cluster, or part thereof, the higher the likelihood of the subject having ovarian cancer. Alternatively, or in addition, such frequency of occurrence can provide information on the tumor load or on the stage of the tumor.

**Table 4.** Overview of 211 CpG clusters identified in cfDNA, (hyper)methylation of which is indicative for ovarian cancer. "#": general number (1-211); "# in chr": number in chromosome; "chr#": chromosome number; "start": position of start of CpG cluster, location of cytosine of first CpG; "end-1": position of end of CpG cluster minus 1, location of cytosine of last CpG; "#CpGs": number of CpGs in the CpG cluster; "gene": indication of gene (partially) harboring the CpG cluster; "# CpG of Table 3": number of the CpG as listed in the column labeled with "#" in Table 3 and which is comprised in the CpG cluster defined in the same row - when in between brackets, the CpG is comprised in the Probe (and not in the CpG cluster); "Probe": the oligonucleotide (of the 400 ovarian tumor specific oligonucleotides) underlying the construction of the CpG cluster in the same row; the numbers indicated in this column refer to start- and end position of the probe, these numbers have to be completed relative to the CpG position: for example for CpG #1, the start position of the probe is 2222408 and the end position 2222544; for e.g. CpG #6, the start position of the probe is 25348926 and the end position 25349079; for e.g. CpG #124, the start position of the probe is 162279888 and the end position 162280097; for e.g. CpG # 84 two different probes are listed.

| # | # in chr | chr# | start | end-1 | # CpGs | gene | # CpG of Table 3 | Probe |
|---|---|---|---|---|---|---|---|---|
| 1 | 1 | chr1 | 2222419 | 2222568 | 13 | MLH | 5-6 | 408-544 |
| 2 | 2 | chr1 | 2232469 | 2232596 | 12 | SKI | 7-8 | 408-544 |
| 3 | 3 | chr1 | 6526073 | 6526267 | 26 | TNFRSF25 (PLEKHG5) | 12 | 105-249 |
| 4 | 4 | chr1 | 9714299 | 9714408 | 9 | PIK3CD | 13 | 318-458 |
| 5 | 5 | chr1 | 16488977 | 16489215 | 19 | | 14 | 037-172 |
| 6 | 6 | chr1 | 25348948 | 25349006 | 5 | | 15 | 8926-9079 |
| 7 | 7 | chr1 | 35351370 | 35351407 | 4 | DLGAP3 | (17) | 263-478 |
| 8 | 8 | chr1 | 50513718 | 50513762 | 6 | ELAVL4 | (25-26) | 3679-4002 |
| 9 | 9 | chr1 | 63790003 | 63790048 | 6 | FOXD3 | 27 | 9964-0116 |
| 10 | 10 | chr1 | 91301484 | 91301794 | 33 | | (33) 34-36 | 406-563; 651-793 |
| 11 | 11 | chr1 | 119535667 | 119535693 | 5 | | (42) 43 | 614-763 |
| 12 | 12 | chr1 | 151811268 | 151811342 | 12 | C2CD4D | (44) | 284-431 |
| 13 | 13 | chr1 | 151811375 | 151811497 | 12 | C2CD4D | (44) | 284-431 |
| 14 | 14 | chr1 | 200842853 | 200842912 | 11 | GPR25 | 48 | 676-963 |
| 15 | 15 | chr1 | 200842920 | 200842960 | 4 | GPR25 | (48) | 676-963 |
| 16 | 1 | chr10 | 8097256 | 8097279 | 4 | GATA3 | (306-307) | 275-409 |
| 17 | 2 | chr10 | 11207850 | 11207952 | 10 | CELF2 | (309) | 829-985 |
| 18 | 3 | chr10 | 22541945 | 22541972 | 4 | LOC100130992 | (311-312) | 1953-2091 |
| 19 | 4 | chr10 | 22542017 | 22542094 | 10 | | (311) 312 | 1953-2091 |
| 20 | 5 | chr10 | 22634107 | 22634125 | 4 | | (313-316) | 121-302 |
| 21 | 6 | chr10 | 22634135 | 22634199 | 8 | | (315-317) | 121-302 |
| 22 | 7 | chr10 | 22634525 | 22634537 | 4 | | (318-319) | 356-501 |
| 23 | 8 | chr10 | 30316276 | 30316485 | 12 | JCAD | 320 | 354-507 |
| 24 | 9 | chr10 | 100993520 | 100993558 | 5 | HPSE2 | 325 | 507-649 |
| 25 | 10 | chr10 | 103536381 | 103536416 | 5 | FGF8 | (329) | 270-410 |
| 26 | 11 | chr10 | 118891641 | 118891716 | 8 | VAX1 | (331) 332-333 | 590-729 |

(continued)

| # | # in chr | chr# | start | end-1 | # CpGs | gene | # CpG of Table 3 | Probe |
|---|---|---|---|---|---|---|---|---|
| 27 | 1 | chr11 | 720781 | 720875 | 15 | EPS8L2 | 343 | 779-930 |
| 28 | 2 | chr11 | 2322642 | 2322696 | 6 | C11orf2 | (346) | 708-839 |
| 29 | 3 | chr11 | 2322729 | 2322808 | 5 | C11orf2 | 346-350 | 708-839 |
| 30 | 4 | chr11 | 31846797 | 31846861 | 10 | PAX6/RCN1 | 354-355 | 747-888 |
| 31 | 5 | chr11 | 44325748 | 44325841 | 11 | ALX4 | 356 | 743-884 |
| 32 | 6 | chr11 | 47416442 | 47416635 | 15 | SLC39A13 | 357-358 | 409-614 |
| 33 | 7 | chr11 | 65647242 | 65647322 | 6 | CTSW | 364 | 194-349 |
| 34 | 8 | chr11 | 65661461 | 65661584 | 12 | FOSL1 | 365 | 441-581 |
| 35 | 9 | chr11 | 66885152 | 66885201 | 4 | | (366) | 160-307 |
| 36 | 10 | chr11 | 76382181 | 76382232 | 8 | | (368-369) | 072-219 |
| 37 | 11 | chr11 | 128565102 | 128565168 | 4 | SENCR/FLI1 | 377 | 089-235 |
| 38 | 1 | chr12 | 3861616 | 3861735 | 6 | CRACR2A | (379) | 542-680 |
| 39 | 2 | chr12 | 6184402 | 6184493 | 7 | VWF | (380) | 277-435 |
| 40 | 3 | chr12 | 14135166 | 14135201 | 4 | | (393) | 125-261 |
| 41 | 4 | chr12 | 46767629 | 46767747 | 9 | | 395-397 | 586-749 |
| 42 | 5 | chr12 | 51717740 | 51717947 | 17 | BIN2 | 399 | 786-940 |
| 43 | 6 | chr12 | 51718011 | 51718224 | 15 | BIN2 | 400-401 | 036-179 |
| 44 | 7 | chr12 | 54088890 | 54088972 | 8 | NXPH4 | 405 | 892-044 |
| 45 | 8 | chr12 | 57618899 | 57618965 | 7 | | 411-412 | 865-012 |
| 46 | 9 | chr12 | 58013324 | 58013592 | 22 | | 413-419 | 400-718 |
| 47 | 10 | chr12 | 58013627 | 58013741 | 7 | SLC26A10 | 420-423 | 400-718 |
| 48 | 11 | chr12 | 58021424 | 58021482 | 8 | B4GALNT1 | (424) | 490-636 |
| 49 | 12 | chr12 | 58021569 | 58021625 | 7 | B4GALNT1 | 424 | 490-636 |
| 50 | 13 | chr12 | 58135963 | 58135988 | 4 | | 425-426 | 895-041 |
| 51 | 14 | chr12 | 66582696 | 66582728 | 4 | | (428) | 731-870 |
| 52 | 15 | chr12 | 85672576 | 85672623 | 5 | | 430 | 544-686 |
| 53 | 16 | chr12 | 85672699 | 85672731 | 4 | | (430) | 544-686 |
| 54 | 17 | chr12 | 85673227 | 85673270 | 5 | | (431-432) | 194-345 |
| 55 | 18 | chr12 | 105114202 | 105114430 | 12 | CHST11 | 433-434 | 250-400 |
| 56 | 19 | chr12 | 114847574 | 114847641 | 8 | TBX5 | 435 | 563-696 |
| 57 | 20 | chr12 | 128850607 | 128850696 | 5 | TMEM132C | (436-437) 438 | 550-698 |
| 58 | 1 | chr13 | 24844769 | 24844978 | 10 | SPATA13 | 441-444 | 4774-5020 |
| 59 | 2 | chr13 | 28527100 | 28527172 | 4 | | (445) | 180-329 |
| 60 | 3 | chr13 | 98794353 | 98794573 | 15 | | 450 | 392-536 |
| 61 | 4 | chr13 | 112722628 | 112722649 | 7 | SOX1 | (451) | 639-788 |
| 62 | 5 | chr13 | 112722658 | 112722730 | 12 | SOX1 | 451 | 639-788 |
| 63 | 6 | chr13 | 112722773 | 112722806 | 7 | SOX1 | (451) | 639-788 |
| 64 | 1 | chr14 | 24045549 | 24045574 | 4 | JPH4 | 453 | 473-628 |

(continued)

| # | # in chr | chr# | start | end-1 | # CpGs | gene | # CpG of Table 3 | Probe |
|---|---|---|---|---|---|---|---|---|
| 65 | 2 | chr14 | 24045586 | 24045632 | 6 | JPH4 | (453) | 473-628 |
| 66 | 3 | chr14 | 38724534 | 38724546 | 4 | CLEC14A | (455) | 577-709 |
| 67 | 4 | chr14 | 38724680 | 38724732 | 5 | CLEC14A | (455) | 577-709 |
| 68 | 5 | chr14 | 102027713 | 102027760 | 5 | DIO3 | (458) | 658-798 |
| 69 | 1 | chr15 | 51385812 | 51385891 | 6 | TNFAIP8L3 | 470 | 5840-6043 |
| 70 | 2 | chr15 | 65689210 | 65689229 | 4 | IGDCC4 | (472) | 086-241 |
| 71 | 3 | chr15 | 75081516 | 75081651 | 8 | CSK | 473 | 492-629 |
| 72 | 4 | chr15 | 81589179 | 81589273 | 7 | IL16 | 474 | 171-334 |
| 73 | 1 | chr16 | 11327009 | 11327233 | 21 | | 479 | 063-206 |
| 74 | 2 | chr16 | 50699167 | 50699197 | 4 | | 484-485 | 118-261 |
| 75 | 3 | chr16 | 54970320 | 54970487 | 21 | | (486) | 393-534 |
| 76 | 4 | chr16 | 55362954 | 55363183 | 19 | IRX6 | 487 | 2978-3124 |
| 77 | 5 | chr16 | 56669661 | 56669726 | 9 | MT1JP | 489-490 | 612-766 |
| 78 | 6 | chr16 | 67197025 | 67197150 | 12 | | 491 | 110-252 |
| 79 | 7 | chr16 | 67197170 | 67197213 | 7 | FBXL8 | 492 | 110-252 |
| 80 | 8 | chr16 | 67197233 | 67197248 | 4 | FBXL8 | (492) | 110-252 |
| 81 | 9 | chr16 | 67199827 | 67199886 | 13 | HSF4 | (493) 494 | 385-537 |
| 82 | 10 | chr16 | 72821482 | 72821611 | 15 | ZFHX3 | 495 | 487-630 |
| 83 | 11 | chr16 | 89034749 | 89034778 | 4 | CBFA2T3 | 497 (498) | 702-844 |
| 84 | 1 | chr17 | 2699371 | 2699802 | 21 | RAP1GAP2 | 500-505 | 425-563; 610-792 |
| 85 | 2 | chr17 | 9143680 | 9143704 | 6 | NTN1 | (515) | 677-814 |
| 86 | 3 | chr17 | 27942025 | 27942155 | 8 | CORO6 | 518 | 036-181 |
| 87 | 4 | chr17 | 32964454 | 32964735 | 26 | TMEM132E | 519-520 | 526-662 |
| 88 | 5 | chr17 | 35303285 | 35303361 | 9 | | 521 | 254-414 |
| 89 | 6 | chr17 | 56408124 | 56408201 | 5 | TSPOAP1 | 526 | 129-255 |
| 90 | 7 | chr17 | 59480572 | 59480881 | 34 | TBX2 | 531-532 | 597-813 |
| 91 | 8 | chr17 | 59530081 | 59530139 | 9 | TBX4 | 533 | 024-175 |
| 92 | 9 | chr17 | 73749593 | 73749710 | 12 | ITGB4 | 536-537 | 615-755 |
| 93 | 10 | chr17 | 80186182 | 80186196 | 4 | | (541-544) | 190-405 |
| 94 | 11 | chr17 | 80186211 | 80186233 | 4 | SLC16A3 | (541-544) | 190-405 |
| 95 | 12 | chr17 | 80186266 | 80186310 | 5 | SLC16A3 | 541-543 (544) | 190-405 |
| 96 | 13 | chr17 | 80358752 | 80358932 | 15 | OGFOD3 | 545-547 | 797-951 |
| 97 | 14 | chr17 | 80407590 | 80407707 | 7 | CYBC1 | 548 | 517-655 |
| 98 | 1 | chr18 | 501137 | 501179 | 5 | | 549 | 030-187 |
| 99 | 2 | chr18 | 3499227 | 3499344 | 21 | DLGAP1 | 550 | 178-326 |
| 100 | 3 | chr18 | 29265882 | 29265925 | 4 | | (551-552) | 762-901 |
| 101 | 4 | chr18 | 77277473 | 77277583 | 9 | NFATC1 | 554 | 507-662 |

(continued)

| # | # in chr | chr# | start | end-1 | # CpGs | gene | # CpG of Table 3 | Probe |
|---|---|---|---|---|---|---|---|---|
| 102 | 1 | chr19 | 3178742 | 3178955 | 22 | S1PR4 | 560 | 766-907 |
| 103 | 2 | chr19 | 6481781 | 6481832 | 4 | DENND1C | 561-562 (563) | 740-924 |
| 104 | 3 | chr19 | 13124112 | 13124306 | 15 | NFIX | 565 | 137-279 |
| 105 | 4 | chr19 | 14550997 | 14551042 | 4 | PKN1 | 566-567 | 0922-1067 |
| 106 | 5 | chr19 | 15342959 | 15343001 | 9 | EPHX3 | (568) | 2910-3058 |
| 107 | 6 | chr19 | 15343017 | 15343030 | 4 | | (568) | 2910-3058 |
| 108 | 7 | chr19 | 17346347 | 17346567 | 31 | NR2F6 | 569 | 427-577 |
| 109 | 8 | chr19 | 50004414 | 50004448 | 4 | | 572 | 368-498 |
| 110 | 9 | chr19 | 51231938 | 51231990 | 6 | | (573) | 1892-2031 |
| 111 | 10 | chr19 | 52104675 | 52104694 | 5 | | (574) | 558-711 |
| 112 | 11 | chr19 | 55593512 | 55593614 | 13 | EPS8L1 | 575 | 501-638 |
| 113 | 1 | chr2 | 19551736 | 19551826 | 10 | OSR1 | 62-63 | 680-927 |
| 114 | 2 | chr2 | 19551868 | 19551894 | 5 | OSR1 | (62-63) | 680-927 |
| 115 | 3 | chr2 | 25496257 | 25496390 | 7 | DNMT3A | 65 | 319-464 |
| 116 | 4 | chr2 | 25499993 | 25500052 | 12 | DNMT3A | 66 | 499968-500109 |
| 117 | 5 | chr2 | 63283874 | 63284021 | 11 | OTX1 | 72-73 (74-75) | 3888-4144 |
| 118 | 6 | chr2 | 86263125 | 86263272 | 6 | POLR1A | 78 | 148-300 |
| 119 | 7 | chr2 | 105459097 | 105459219 | 14 | PANTR1 | 83-84 | 085-236 |
| 120 | 8 | chr2 | 105459233 | 105459253 | 4 | PANTR1 | (83-84) | 085-236 |
| 121 | 9 | chr2 | 127413505 | 127413553 | 4 | GYPC | 85 | 455-600 |
| 122 | 10 | chr2 | 127414059 | 127414189 | 18 | GYPC | 86 | 038-173 |
| 123 | 11 | chr2 | 127839449 | 127839653 | 12 | BIN1 | 89 | 468-603 |
| 124 | 12 | chr2 | 162280035 | 162280048 | 4 | TBR1 | (91) | 79888-80097 |
| 125 | 13 | chr2 | 162280074 | 162280098 | 4 | TBR1 | (91) | 79888-80097 |
| 126 | 14 | chr2 | 162280108 | 162280122 | 4 | TBR1 | (91) | 79888-80097 |
| 127 | 15 | chr2 | 219736469 | 219736591 | 16 | WNT6 | 98 | 470-626 |
| 128 | 16 | chr2 | 223154140 | 223154197 | 8 | PAX3 | 99 | 101-231 |
| 129 | 17 | chr2 | 228736258 | 228736310 | 7 | | (100) 101 | 183-337 |
| 130 | 18 | chr2 | 228736324 | 228736343 | 5 | DAW1 | (101) | 183-337 |
| 131 | 19 | chr2 | 233924852 | 233924930 | 7 | | (103) | 4859-5016 |
| 132 | 20 | chr2 | 238599945 | 238600002 | 7 | LRRFIP1 | (104) | 599984-600122 |
| 133 | 21 | chr2 | 238600017 | 238600049 | 6 | LRRFIP1 | (104) | 599984-600122 |
| 134 | 22 | chr2 | 238600058 | 238600083 | 5 | LRRFIP1 | 104 | 599984-600122 |
| 135 | 23 | chr2 | 242743157 | 242743288 | 23 | GAL3ST2 | 105 | 135-282 |
| 136 | 1 | chr20 | 25129223 | 25129350 | 11 | LOC284798 | (577) 578 | 113-377 |
| 137 | 2 | chr20 | 35274570 | 35274715 | 7 | SLA2 | 580-583 | 573-699 |
| 138 | 1 | chr21 | 36399099 | 36399258 | 19 | RUNX1 | 591-592 | 147-291 |

(continued)

| # | # in chr | chr# | start | end-1 | # CpGs | gene | # CpG of Table 3 | Probe |
|---|---|---|---|---|---|---|---|---|
| 139 | 2 | chr21 | 36421433 | 36421503 | 5 | RUNX1 | 593-595 | 418-561 |
| 140 | 1 | chr22 | 24823389 | 24823611 | 11 | ADORA2A/ SPECCL1 | 597-601 | 449-588 |
| 141 | 2 | chr22 | 30662972 | 30663076 | 11 | | 602-607 | 2927-3094 |
| 142 | 3 | chr22 | 30663228 | 30663434 | 12 | | 608 | 247-387 |
| 143 | 4 | chr22 | 38477033 | 38477098 | 5 | SLC16A8 | 610 | 008-158 |
| 144 | 5 | chr22 | 50623648 | 50623680 | 5 | | (614-615) | 607-764 |
| 145 | 1 | chr3 | 50312802 | 50312975 | 18 | SEMA3B | 111 | 835-973 |
| 146 | 2 | chr3 | 50312992 | 50313009 | 4 | SEMA3B | (111) | 835-973 |
| 147 | 3 | chr3 | 51990551 | 51990638 | 10 | GPR62 | 113 | 498-644 |
| 148 | 4 | chr3 | 121379695 | 121379791 | 8 | HCLS1 | 117-119 | 715-853 |
| 149 | 5 | chr3 | 147126697 | 147126741 | 5 | | (122) | 623-759 |
| 150 | 6 | chr3 | 147127043 | 147127080 | 5 | | (124-125) | 6883-7174 |
| 151 | 7 | chr3 | 147127581 | 147127669 | 12 | ZIC1 | 126 | 584-733 |
| 152 | 8 | chr3 | 147127676 | 147127721 | 8 | ZIC1 | (126) | 584-733 |
| 153 | 9 | chr3 | 147128015 | 147128283 | 33 | ZIC1 | 127-128 | 049-221 |
| 154 | 10 | chr3 | 147130423 | 147130576 | 15 | ZIC1 | 129-130 | 457-592 |
| 155 | 11 | chr3 | 147140820 | 147140847 | 4 | | 131 (132-133) | 803-944 |
| 156 | 12 | chr3 | 184099312 | 184099341 | 5 | CHRD | (142) | 353-500 |
| 157 | 13 | chr3 | 184099378 | 184099402 | 5 | CHRD | (142) | 353-500 |
| 158 | 14 | chr3 | 184099410 | 184099569 | 15 | CHRD | 142 | 353-500 |
| 159 | 1 | chr4 | 996164 | 996175 | 4 | IDUA | 145 | 105-248 |
| 160 | 2 | chr4 | 996182 | 996214 | 6 | | (145) | 105-248 |
| 161 | 3 | chr4 | 13524191 | 13524222 | 5 | | (147) | 3964-4206 |
| 162 | 4 | chr4 | 79642406 | 79642506 | 6 | | 154 | 416-551 |
| 163 | 5 | chr4 | 90757351 | 90757393 | 5 | SNCA | 156 | 321-472 |
| 164 | 6 | chr4 | 90757410 | 90757461 | 7 | | 157 | 321-472 |
| 165 | 7 | chr4 | 155411690 | 155411750 | 10 | CDHS2 | (162) | 726-869 |
| 166 | 8 | chr4 | 174459370 | 174459423 | 10 | HAND2 | 166 | 333-480 |
| 167 | 1 | chr5 | 40681077 | 40681141 | 7 | PTGER4 | 172 | 061-204 |
| 168 | 2 | chr5 | 40681185 | 40681231 | 4 | PTGER4 | (172) | 061-204 |
| 169 | 3 | chr5 | 40681782 | 40681867 | 15 | PTGER4 | (173) | 821-971 |
| 170 | 4 | chr5 | 42952292 | 42952369 | 6 | | 174 | 291-437 |
| 171 | 5 | chr5 | 132150034 | 132150105 | 8 | SOWAHA | (188) | 049-198 |
| 172 | 6 | chr5 | 169724753 | 169724803 | 4 | LCP2 | 195-197 (198) | 755-907 |
| 173 | 7 | chr5 | 176302865 | 176302976 | 8 | UNC5A | 200-201 | 837-990 |
| 174 | 8 | chr5 | 176784688 | 176784779 | 8 | | 202-204 | 641-801 |

(continued)

| # | # in chr | chr# | start | end-1 | # CpGs | gene | # CpG of Table 3 | Probe |
|---|---|---|---|---|---|---|---|---|
| 175 | 1 | chr6 | 475044 | 475083 | 4 | | 205 | 4960-5120 |
| 176 | 2 | chr6 | 7541036 | 7541138 | 8 | | 206 | 40994-41132 |
| 177 | 3 | chr6 | 10422332 | 10422371 | 6 | | 207 | 246-407 |
| 178 | 4 | chr6 | 17280987 | 17281073 | 12 | | 209 | 0936-1080 |
| 179 | 5 | chr6 | 27107097 | 27107354 | 27 | HIST1H4I/ HIST1H2BK | 211-212 | 077-221 |
| 180 | 6 | chr6 | 28457149 | 28457213 | 5 | | 213 | 170-312 |
| 181 | 7 | chr6 | 29943349 | 29943593 | 23 | | 219-223 | 408-537 |
| 182 | 8 | chr6 | 30653580 | 30653755 | 14 | | 230-233 (234) | 652-813 |
| 183 | 9 | chr6 | 30711555 | 30711586 | 5 | | 235 | 1578-2126 |
| 184 | 10 | chr6 | 31765567 | 31765634 | 5 | | 236-240 | 513-653 |
| 185 | 11 | chr6 | 41528674 | 41528874 | 17 | FOXP4 | (250) 251-252 | 547-820 |
| 186 | 12 | chr6 | 42738973 | 42739079 | 13 | | (253) 254-255 | 8890-9131 |
| 187 | 13 | chr6 | 105388681 | 105388718 | 4 | | (259) 260 (261) | 598-797 |
| 188 | 14 | chr6 | 105400878 | 105400929 | 5 | | (262-263) | 0906-1050 |
| 189 | 15 | chr6 | 105400978 | 105401004 | 6 | | 262-263 | 0906-1050 |
| 190 | 16 | chr6 | 106429294 | 106429307 | 4 | | (264) | 240-520 |
| 191 | 17 | chr6 | 106429378 | 106429414 | 6 | | (264) | 240-520 |
| 192 | 18 | chr6 | 134210790 | 134210868 | 7 | TCF21 | (267) | 0867-1015 |
| 193 | 19 | chr6 | 134211020 | 134211057 | 9 | TCF21 | (267) | 0867-1015 |
| 194 | 20 | chr6 | 170403214 | 170403350 | 14 | | 268 | 185-331 |
| 195 | 21 | chr6 | 170463378 | 170463499 | 7 | | 269 | 325-464 |
| 196 | 1 | chr7 | 26193003 | 26193052 | 4 | NFE2L3 | 270 | 030-169 |
| 197 | 2 | chr7 | 26193061 | 26193230 | 9 | NFE2L3 | (270) | 030-169 |
| 198 | 3 | chr7 | 27205200 | 27205280 | 11 | HOXA10-HOXA9 | 271-273 | 126-299 |
| 199 | 4 | chr7 | 42267684 | 42267710 | 6 | GLI3 | (276-277) | 645-827 |
| 200 | 5 | chr7 | 42267803 | 42267857 | 6 | GLI3 | (276-277) | 645-827 |
| 201 | 6 | chr7 | 45018734 | 45018964 | 13 | | (278-280) | 719-918 |
| 202 | 7 | chr7 | 96652218 | 96652231 | 4 | DLX5 | (284) 285 | 145-297 |
| 203 | 8 | chr7 | 121939881 | 121939919 | 4 | | (287) | 750-906 |
| 204 | 1 | chr8 | 23563925 | 23563940 | 4 | NKX2-6 | (293) | 3955-4099 |
| 205 | 2 | chr8 | 23563960 | 23564059 | 14 | | 293-294 | 3955-4099 |
| 206 | 3 | chr8 | 72754447 | 72754517 | 8 | MSC | 296 | 397-543 |
| 207 | 4 | chr8 | 145106219 | 145106353 | 17 | OPLAH | 300-301 | 220-364 |
| 208 | 1 | chr9 | 100616558 | 100616594 | 4 | FOXE1 | (302-303) | 392-679 |

(continued)

| # | # in chr | chr# | start | end-1 | # CpGs | gene | # CpG of Table 3 | Probe |
|---|---|---|---|---|---|---|---|---|
| 209 | 2 | chr9 | 100616626 | 100616638 | 4 | | (302-303) | 392-679 |
| 210 | 3 | chr9 | 122132044 | 122132054 | 4 | | (304) | 1976-2119 |
| 211 | 4 | chr9 | 139427882 | 139428039 | 13 | NOTCH1 | 305 | 7927-8069 |

By way of example, the construction of CpG cluster no. 5 of Table 4 is outlined hereafter. Reads in the region of CpG cluster #5 that are common between the 4 fold validation runs are listed in Table 5 by means of the start positions of the CpGs in the read. Combining the overlapping reads yields the cluster. The start position of the cluster is defined by the start position of the first occurring CpG, the end position of the cluster by the end position of the last occurring CpG (thus the start position of the last occurring CpG + 1). The full sequence of CpG cluster #5 is depicted in SEQ ID NO:2 wherein the CpGs are highlighted, and was obtained via https://www.ncbi.nlm.nih.gov/genome/ as explained in the detailed description.

**Table 5.** Construction of CpG cluster #5 (of Table 4). Indicated are chromosome number (all chromosome 1) and start positions of CpGs. Each row in the Table corresponds to an individual sequence read (characterized only by the positions of the CpGs present in the read). The overlap between the individual sequence reads is illustrated by the bold-faced CpG start positions or bold-italic CpG start positions.

| |
|---|
| chr1_16488977.Z_16488996.Z_16489000.Z_**16489002.Z** |
| chr1_16488996.Z_16489000.Z_**16489002.Z**_16489046.Z_16489048.Z |
| chr1_16488996.Z_16489000.Z_**16489002.Z_**16489046.Z_16489048.Z_16489061.Z |
| chr1_16488996.Z_16489000.Z_**16489002.Z_**16489046.Z_16489048.Z_16489061.Z_16489065.Z |
| chr1_16489000.Z_**16489002.Z**_16489046.Z_16489048.Z |
| chr1_16489000.Z_**16489002.Z**_16489046.Z_16489048.Z_16489061.Z |
| chr1_16489000.Z_**16489002.Z**_16489046.Z_16489048.Z_16489061.Z_16489065.Z |
| chr1_**16489002.Z**_16489046.Z_16489048.Z_***16489961.Z*** |
| chr1_**16489002.Z**_16489046.Z_16489048.Z_***16489961.Z***_16489065.Z |
| chr1_**16489002.Z**_16489046.Z_16489048.7_16489061.Z_***16489065.Z***_16489074.Z |
| chr1_16489046.Z_16489048.Z_***16489061.Z***_16489065.Z |
| chr1_16489046.Z_16489048.Z_***16489061.Z***_16489065.Z_16489074.Z |
| chr1_16489046.Z_16489048.Z_***16489061.Z***_16489065.Z_16489074.Z _16489083.Z |
| chr1_16489048Z_***16489061.Z***_16489065.Z_16489074.Z |
| chr1_16489048.Z_***16489061.Z***_16489065.Z_16489074.Z_16489083.Z |
| chr1_***16489061.Z***_16489065.Z_16489074.Z_**16489083.Z** |
| chr1_***16489061.Z***_16489065.Z_16489074.Z_**16489083.Z**_16489102.Z |
| chr1_16489065.Z_16489074.Z_**16489083.Z**_16489102.Z |
| chr1_16489065.Z_16489074.Z_**16489083.Z**_16489102.Z_16489115.Z |
| chr1_16489074.Z_**16489083.Z**_16489102.Z_***16489115.Z*** |
| chr1_16489102.Z_***16489115.Z***_16489123.Z_16489135.Z |
| chr1_16489102.Z_***16489115.Z***_16489123.Z_16489135.Z_16489142.Z |
| chr1_16489102.Z_16489115.Z_***16489123.Z***_16489135.Z_16489142.Z 16489153.Z |
| chr1_16489102.Z_16489115.Z_***16489123.Z***_16489135.Z_16489142.Z_16489153.Z_16489185.Z |
| chr1_16489102.Z_***16489115.Z***_16489123.Z_16489135.Z_16489142.Z_16489153.Z_16489185.Z_16489199.Z |
| chr1_***16489115.Z***_16489123.Z_16489135.Z_**16489142.Z** |
| chr1_***16489115.Z***_16489123.Z_16489135.Z_**16489142.Z**_16489153.Z |

(continued)

| |
|---|
| chr1_***16489115.Z_***16489123.Z_16489135.Z_16489142.Z_**16489153.Z_**16489185.Z |
| chr1_16489123.7_16489135.Z_**16489142.Z_**16489153.Z |
| chr1_16489123.Z_16489135.Z_**16489142.Z_**16489153.Z_16489185.Z |
| chr1_16489135.Z_**16489142.Z_**16489153.Z_16489185.Z |
| chr1_16489135.Z_**16489142.Z_**16489153.Z_16489185.Z_16489199.Z |
| chr1_16489135.Z_**16489142.Z_**16489153.Z_16489185.Z_16489199.Z_16489215.Z |
| chr1_**16489142.Z_**16489153.Z_16489185.Z_***16489199.Z*** |
| chr1_**16489142.Z_**16489153.Z_16489185.Z_***16489199.Z_***16489215.Z |
| chr1_16489153.Z_16489185.Z_***16489199.Z_***16489215.Z |

Full DNA sequence of CpG cluster #5 : chr1:16488977 (referred to as "8977" in the sequence below) - 16489216 (referred to as "9216" in the sequence below). The 19 CpGs comprised therein (see Table 4) are shadowed in black.

```
8977        CGCC ACTTCCTTTG GGGCTCGCTC GCGCTTCCTG GGAGCCTTTG ATGTGGGTTC
9031 TTGGTAGATC CTGCCCGCGG GGCCTGGATG CGGGCGGAGC CTCCGGGCAG GGCGGGGCCT
9091 CAGCCTGGGT CCGCACTGGG GAGGCGGCCA CCCGGTAGCT TGGGCGGGTT CCGCCCACCT
9151 CCCGCCTGTA TTTACAGCCC CCTTCCAACA TCCCCGACTC CCTGAGGGCG CCCCTCATTC
9211 TGCTCG 9216       (SEQ ID NO:2)
```

By way of example, CpG clusters 93, 94 and 95 are explained in more detail with respect to Table 4, and more specifically with respect to the columns "# CpG of Table 3" and "Probe". CpG clusters 93-95 (all of chromosome 17) all are built on the basis of an ovarian tumor specific probe spanning positions 80186190 to 80186405 (see Table 4, column "Probe"). This probe comprises the CpGs 541 (start position 80186266), 542 (start position 80186273), 543 (start position 80186275) and 544 (start position 80186336) of Table 3. CpG cluster 95 is spanning positions 80186266 to 80186311 (end = (end-1) +1)), and thus comprises CpGs 541, 542 and 543, but not 544. CpG clusters 93 and 94 do not comprise a CpG listed in Table 3 (none of CpGs 541-544, hence indication (541-544)) but were identified based on sequence reads obtained through the probe, as CpG clusters 93 and 94 do overlap partially or completely with the probe. The latter does not always need to be true as sequence reads obtained through a probe may reveal CpG clusters present in such sequence read but residing outside the probe region, examples thereof are CpG clusters #48, 53, 59, 66, 126, 156 and 193 of Table 4, and CpG cluster #192 of Table 4 is only overlapping by 2 nucleotides with the corresponding probe.

**Table 6.** Individual CpGs comprised in CpG clusters listed in Table 4 and defined by start position ("start") on indicated chromosome ("chr #"). The cross-reference to the numbering in Table 3 ("# in Table 3") is indicated as well as a new general number ("#").

| # | # in Table 3 | chr# | start |
|---|---|---|---|
| 1 | 5 | chr1 | 2222419 |
| 2 | 6 | chr1 | 2222440 |
| 3 | 7 | chr1 | 2232469 |
| 4 | 8 | chr1 | 2232481 |
| 5 | 12 | chr1 | 6526129 |
| 6 | 13 | chr1 | 9714397 |
| 7 | 14 | chr1 | 16489115 |
| 8 | 15 | chr1 | 25349006 |
| 9 | 27 | chr1 | 63790044 |
| 10 | 34 | chr1 | 91301484 |
| 11 | 35 | chr1 | 91301651 |
| 12 | 36 | chr1 | 91301731 |

(continued)

| # | # in Table 3 | chr# | start |
|---|---|---|---|
| 13 | 43 | chr1 | 119535693 |
| 14 | 48 | chr1 | 200842890 |
| 15 | 62 | chr2 | 19551750 |
| 16 | 63 | chr2 | 19551789 |
| 17 | 65 | chr2 | 25496390 |
| 18 | 66 | chr2 | 25500046 |
| 19 | 72 | chr2 | 63283939 |
| 20 | 73 | chr2 | 63283967 |
| 21 | 78 | chr2 | 86263224 |
| 22 | 83 | chr2 | 105459097 |
| 23 | 84 | chr2 | 105459164 |
| 24 | 85 | chr2 | 127413505 |
| 25 | 86 | chr2 | 127414108 |
| 26 | 89 | chr2 | 127839539 |
| 27 | 98 | chr2 | 219736549 |
| 28 | 99 | chr2 | 223154140 |
| 29 | 101 | chr2 | 228736258 |
| 30 | 104 | chr2 | 238600061 |
| 31 | 105 | chr2 | 242743214 |
| 32 | 111 | chr3 | 50312913 |
| 33 | 113 | chr3 | 51990575 |
| 34 | 117 | chr3 | 121379745 |
| 35 | 118 | chr3 | 121379777 |
| 36 | 119 | chr3 | 121379791 |
| 37 | 126 | chr3 | 147127662 |
| 38 | 127 | chr3 | 147128123 |
| 39 | 128 | chr3 | 147128157 |
| 40 | 129 | chr3 | 147130477 |
| 41 | 130 | chr3 | 147130536 |
| 42 | 131 | chr3 | 147140847 |
| 43 | 142 | chr3 | 184099432 |
| 44 | 145 | chr4 | 996175 |
| 45 | 154 | chr4 | 79642491 |
| 46 | 156 | chr4 | 90757351 |
| 47 | 157 | chr4 | 90757452 |
| 48 | 166 | chr4 | 174459412 |
| 49 | 172 | chr5 | 40681137 |
| 50 | 174 | chr5 | 42952369 |
| 51 | 195 | chr5 | 169724768 |

(continued)

| # | # in Table 3 | chr# | start |
|---|---|---|---|
| 52 | 196 | chr5 | 169724791 |
| 53 | 197 | chr5 | 169724803 |
| 54 | 200 | chr5 | 176302913 |
| 55 | 201 | chr5 | 176302976 |
| 56 | 202 | chr5 | 176784688 |
| 57 | 203 | chr5 | 176784715 |
| 58 | 204 | chr5 | 176784719 |
| 59 | 205 | chr6 | 475074 |
| 60 | 206 | chr6 | 7541066 |
| 61 | 207 | chr6 | 10422322 |
| 62 | 209 | chr6 | 17281015 |
| 63 | 211 | chr6 | 27107097 |
| 64 | 212 | chr6 | 27107145 |
| 65 | 213 | chr6 | 28457210 |
| 66 | 219 | chr6 | 29943408 |
| 67 | 220 | chr6 | 29943414 |
| 68 | 221 | chr6 | 29943425 |
| 69 | 222 | chr6 | 29943455 |
| 70 | 223 | chr6 | 29943480 |
| 71 | 230 | chr6 | 30653659 |
| 72 | 231 | chr6 | 30653732 |
| 73 | 232 | chr6 | 30653736 |
| 74 | 233 | chr6 | 30653755 |
| 75 | 235 | chr6 | 30711580 |
| 76 | 236 | chr6 | 31765567 |
| 77 | 237 | chr6 | 31765590 |
| 78 | 238 | chr6 | 31765614 |
| 79 | 239 | chr6 | 31765619 |
| 80 | 240 | chr6 | 31765634 |
| 81 | 251 | chr6 | 41528750 |
| 82 | 252 | chr6 | 41528785 |
| 83 | 254 | chr6 | 42739021 |
| 84 | 255 | chr6 | 42739049 |
| 85 | 260 | chr6 | 105388694 |
| 86 | 262 | chr6 | 105400985 |
| 87 | 263 | chr6 | 105400993 |
| 88 | 268 | chr6 | 170403264 |
| 89 | 269 | chr6 | 170463397 |
| 90 | 270 | chr7 | 26193032 |

(continued)

| # | # in Table 3 | chr# | start |
|---|---|---|---|
| 91 | 271 | chr7 | 27205224 |
| 92 | 272 | chr7 | 27205230 |
| 93 | 273 | chr7 | 27205262 |
| 94 | 285 | chr7 | 96652245 |
| 95 | 293 | chr8 | 23563970 |
| 96 | 294 | chr8 | 23564025 |
| 97 | 296 | chr8 | 72754469 |
| 98 | 300 | chr8 | 145106246 |
| 99 | 301 | chr8 | 145106299 |
| 100 | 305 | chr9 | 139428006 |
| 101 | 312 | chr10 | 22542024 |
| 102 | 313 | chr10 | 22634142 |
| 103 | 314 | chr10 | 22634199 |
| 104 | 320 | chr10 | 30316432 |
| 105 | 325 | chr10 | 100993553 |
| 106 | 332 | chr10 | 118891670 |
| 107 | 333 | chr10 | 118891706 |
| 108 | 343 | chr11 | 720859 |
| 109 | 346 | chr11 | 2322729 |
| 110 | 347 | chr11 | 2322741 |
| 111 | 348 | chr11 | 2322781 |
| 112 | 349 | chr11 | 2322802 |
| 113 | 350 | chr11 | 2322808 |
| 114 | 354 | chr11 | 31846844 |
| 115 | 355 | chr11 | 31846849 |
| 116 | 356 | chr11 | 44325823 |
| 117 | 357 | chr11 | 47416487 |
| 118 | 358 | chr11 | 47416535 |
| 119 | 364 | chr11 | 65647270 |
| 120 | 365 | chr11 | 65661516 |
| 121 | 377 | chr11 | 128565168 |
| 122 | 395 | chr12 | 46767665 |
| 123 | 396 | chr12 | 46767683 |
| 124 | 397 | chr12 | 46767747 |
| 125 | 399 | chr12 | 51717865 |
| 126 | 400 | chr12 | 51718088 |
| 127 | 401 | chr12 | 51718112 |
| 128 | 405 | chr12 | 54088934 |
| 129 | 411 | chr12 | 57618943 |

(continued)

| # | # in Table 3 | chr# | start |
|---|---|---|---|
| 130 | 412 | chr12 | 57618965 |
| 131 | 413 | chr12 | 58013414 |
| 132 | 414 | chr12 | 58013458 |
| 133 | 415 | chr12 | 58013475 |
| 134 | 416 | chr12 | 58013487 |
| 135 | 417 | chr12 | 58013517 |
| 136 | 418 | chr12 | 58013539 |
| 137 | 419 | chr12 | 58013569 |
| 138 | 420 | chr12 | 58013636 |
| 139 | 421 | chr12 | 58013645 |
| 140 | 422 | chr12 | 58013651 |
| 141 | 423 | chr12 | 58013687 |
| 142 | 424 | chr12 | 58021569 |
| 143 | 425 | chr12 | 58135973 |
| 144 | 426 | chr12 | 58135981 |
| 145 | 430 | chr12 | 85672623 |
| 146 | 433 | chr12 | 105114327 |
| 147 | 434 | chr12 | 105114399 |
| 148 | 435 | chr12 | 114847578 |
| 149 | 438 | chr12 | 128850696 |
| 150 | 441 | chr13 | 24844846 |
| 151 | 442 | chr13 | 24844852 |
| 152 | 443 | chr13 | 24844880 |
| 153 | 444 | chr13 | 24844896 |
| 154 | 450 | chr13 | 98794470 |
| 155 | 451 | chr13 | 112722719 |
| 156 | 453 | chr14 | 24045549 |
| 157 | 470 | chr15 | 51385891 |
| 158 | 473 | chr15 | 75081572 |
| 159 | 474 | chr15 | 81589248 |
| 160 | 479 | chr16 | 11327141 |
| 161 | 484 | chr16 | 50699167 |
| 162 | 485 | chr16 | 50699197 |
| 163 | 487 | chr16 | 55363058 |
| 164 | 489 | chr16 | 56669681 |
| 165 | 490 | chr16 | 56669726 |
| 166 | 491 | chr16 | 67197150 |
| 167 | 492 | chr16 | 67197186 |
| 168 | 494 | chr16 | 67199830 |

(continued)

| # | # in Table 3 | chr# | start |
|---|---|---|---|
| 169 | 495 | chr16 | 72821498 |
| 170 | 497 | chr16 | 89034778 |
| 171 | 500 | chr17 | 2699505 |
| 172 | 501 | chr17 | 2699542 |
| 173 | 502 | chr17 | 2699553 |
| 174 | 503 | chr17 | 2699689 |
| 175 | 504 | chr17 | 2699706 |
| 176 | 505 | chr17 | 2699718 |
| 177 | 518 | chr17 | 27942115 |
| 178 | 519 | chr17 | 32964596 |
| 179 | 520 | chr17 | 32964615 |
| 180 | 521 | chr17 | 35303330 |
| 181 | 526 | chr17 | 56408197 |
| 182 | 531 | chr17 | 59480674 |
| 183 | 532 | chr17 | 59480730 |
| 184 | 533 | chr17 | 59530104 |
| 185 | 536 | chr17 | 73749654 |
| 186 | 537 | chr17 | 73749694 |
| 187 | 541 | chr17 | 80186266 |
| 188 | 542 | chr17 | 80186273 |
| 189 | 543 | chr17 | 80186275 |
| 190 | 545 | chr17 | 80358829 |
| 191 | 546 | chr17 | 80358850 |
| 192 | 547 | chr17 | 80358876 |
| 193 | 548 | chr17 | 80407590 |
| 194 | 549 | chr18 | 501137 |
| 195 | 550 | chr18 | 3499253 |
| 196 | 554 | chr18 | 77277583 |
| 197 | 560 | chr19 | 3178844 |
| 198 | 561 | chr19 | 6481819 |
| 199 | 562 | chr19 | 6481826 |
| 200 | 565 | chr19 | 13124215 |
| 201 | 566 | chr19 | 14550997 |
| 202 | 567 | chr19 | 14550999 |
| 203 | 569 | chr19 | 17346507 |
| 204 | 572 | chr19 | 50004444 |
| 205 | 575 | chr19 | 55593580 |
| 206 | 578 | chr20 | 25129296 |
| 207 | 580 | chr20 | 35274595 |

(continued)

| # | # in Table 3 | chr# | start |
|---|---|---|---|
| 208 | 581 | chr20 | 35274627 |
| 209 | 582 | chr20 | 35274639 |
| 210 | 583 | chr20 | 35274655 |
| 211 | 591 | chr21 | 36399226 |
| 212 | 592 | chr21 | 36399258 |
| 213 | 593 | chr21 | 36421467 |
| 214 | 594 | chr21 | 36421472 |
| 215 | 595 | chr21 | 36421503 |
| 216 | 597 | chr22 | 24823455 |
| 217 | 598 | chr22 | 24823509 |
| 218 | 599 | chr22 | 24823514 |
| 219 | 600 | chr22 | 24823519 |
| 220 | 601 | chr22 | 24823554 |
| 221 | 602 | chr22 | 30662972 |
| 222 | 603 | chr22 | 30662987 |
| 223 | 604 | chr22 | 30662994 |
| 224 | 605 | chr22 | 30663007 |
| 225 | 606 | chr22 | 30663034 |
| 226 | 607 | chr22 | 30663041 |
| 227 | 608 | chr22 | 30663316 |
| 228 | 610 | chr22 | 38477085 |

**Table 7.** Individual CpGs not comprised in CpG clusters or probes listed in Table 4 and defined by start position ("start") on indicated chromosome ("chr #"). A general number ("#") is given to each CpG. "#3": CpG number ("#") as used in Table 3; H: percentage methylation of the CpG in healthy reference cfDNA samples (n=49); T: percentage methylation of the CpG in ovarian cancer cfDNA samples (HGSOC; n=25). Grey-shaded CpGs are also listed in Table 8; black-shaded CpGs are also listed in Table 6. Missing from Table 3 are CpG # 3, 9, 11, 24, 28, 29, 32, 36, 187, 467, 556, and 571 (methylation status to be determined). Not included in this Table are CpGs from Table 3 with # 282, 341, 342, 499, 510, 538, and 559 because of no difference between H and T values.

| # | #3 | chr# | start | H | T |
|---|---|---|---|---|---|
| 1 | 1 | chr1 | 969254 | 0 | 22.43 |
| 2 | 19 | chr1 | 36043002 | 0.99 | 46.24 |
| 3 | 143 | chr3 | 187458630 | 2.51 | 29.94 |
| 4 | 144 | chr3 | 194408901 | 0 | 15.02 |
| 5 | 146 | chr4 | 4867164 | 1.47 | 10.35 |
| **6** | **147** | **chr4** | **13524143** | **0.35** | **14.06** |
| 7 | 148 | chr4 | 25656865 | 0.95 | 5.44 |
| 8 | 149 | chr4 | 38807337 | 0 | 2.45 |
| 9 | 150 | chr4 | 38807382 | 0.17 | 2.56 |
| 10 | 151 | chr4 | 40198392 | 0.82 | 5.5 |
| 11 | 152 | chr4 | 40858965 | 7.21 | 11.39 |
| 12 | 153 | chr4 | 56686117 | 1.08 | 23.1 |
| 13 | 20 | chr1 | 36043014 | 0.82 | 47.54 |
| 14 | 155 | chr4 | 85402497 | 1.2 | 24.51 |
| 15 | 158 | chr4 | 120061211 | 2.02 | 60.74 |
| 16 | 159 | chr4 | 140656926 | 0.95 | 47.44 |
| 17 | 160 | chr4 | 153601329 | 2.42 | 13.92 |
| 18 | 161 | chr4 | 154713748 | 1.49 | 14.98 |
| **19** | **162** | **chr4** | **155411806** | **2.76** | **20.79** |
| 20 | 163 | chr4 | 169799087 | 0.2 | 46.4 |
| 21 | 164 | chr4 | 174430487 | 2.94 | 18.4 |
| 22 | 165 | chr4 | 174452835 | 9.27 | 35.5 |
| 23 | 167 | chr4 | 175133103 | 0.52 | 5.81 |
| 24 | 21 | chr1 | 36043084 | 2.31 | 39.61 |
| 25 | 168 | chr4 | 175133151 | 1.22 | 13.44 |
| 26 | 169 | chr4 | 183062460 | 0.32 | 21.87 |
| 27 | 170 | chr5 | 2038528 | 0.75 | 9.51 |
| 28 | 171 | chr5 | 14810180 | 0.73 | 54.08 |
| **29** | **173** | **chr5** | **40681893** | **0.21** | **25.52** |
| 30 | 175 | chr5 | 42994123 | 1.22 | 47.5 |
| 31 | 176 | chr5 | 42994709 | 0.58 | 16.39 |
| 32 | 177 | chr5 | 42994776 | 0.23 | 27.57 |
| 33 | 178 | chr5 | 43019660 | 0.45 | 34.27 |
| 34 | 179 | chr5 | 54398555 | 4.8 | 11.75 |
| 35 | 22 | chr1 | 42217077 | 2 | 25.6 |
| 36 | 180 | chr5 | 94982330 | 6.47 | 17.19 |
| 37 | 181 | chr5 | 115152413 | 0.26 | 15.6 |
| 38 | 182 | chr5 | 115152420 | 0.57 | 21.28 |
| 39 | 183 | chr5 | 115152431 | 0.52 | 20.75 |
| 40 | 184 | chr5 | 115152485 | 0.98 | 26.27 |
| 41 | 185 | chr5 | 115152492 | 1.14 | 29.73 |
| 42 | 186 | chr5 | 115152494 | 1.36 | 29.52 |

| # | #3 | chr# | start | H | T |
|---|---|---|---|---|---|
| **43** | **188** | **chr5** | **132150117** | **3.94** | **26.74** |
| 44 | 189 | chr5 | 134363516 | 2.58 | 32.07 |
| 45 | 23 | chr1 | 47701206 | 2.81 | 51.21 |
| 46 | 190 | chr5 | 134363562 | 11.61 | 40.92 |
| 47 | 191 | chr5 | 138677027 | 3.54 | 4.37 |
| 48 | 192 | chr5 | 140797234 | 0.8 | 20.56 |
| 49 | 193 | chr5 | 142814934 | 0.74 | 1.87 |
| 50 | 194 | chr5 | 159894937 | 0.2 | 11.9 |
| **51** | **198** | **chr5** | **169724831** | **0.37** | **13.9** |
| 52 | 199 | chr5 | 172672959 | 0.8 | 15.51 |
| 53 | 208 | chr6 | 13274180 | 9.54 | 11.73 |
| 54 | 210 | chr6 | 26044405 | 3.44 | 22.96 |
| 55 | 214 | chr6 | 29617549 | 2.48 | 24.46 |
| 56 | 215 | chr6 | 29894315 | 1.33 | 37.09 |
| 57 | 216 | chr6 | 29894619 | 0.39 | 22.2 |
| 58 | 217 | chr6 | 29894679 | 0.25 | 23.68 |
| 59 | 218 | chr6 | 29895116 | 6.7 | 14.72 |
| 60 | 224 | chr6 | 30458161 | 3.93 | 19.02 |
| 61 | 225 | chr6 | 30646949 | 0 | 18.46 |
| 62 | 226 | chr6 | 30652347 | 0 | 18.69 |
| 63 | 227 | chr6 | 30652376 | 0 | 11.18 |
| 64 | 228 | chr6 | 30652396 | 0 | 3.27 |
| 65 | 229 | chr6 | 30652399 | 0 | 4 |
| **66** | **26** | **chr1** | **50513870** | **1.5** | **14.06** |
| **67** | **234** | **chr6** | **30653799** | **1.66** | **34.18** |
| 68 | 241 | chr6 | 31783468 | 0 | 18.16 |
| 69 | 242 | chr6 | 31866072 | 0.18 | 0.49 |
| 70 | 243 | chr6 | 33245488 | 7.68 | 36.09 |
| 71 | 244 | chr6 | 33245490 | 9.76 | 36.1 |
| 72 | 245 | chr6 | 33245537 | 4.39 | 22.26 |
| 73 | 246 | chr6 | 33245541 | 3.6 | 22.09 |
| 74 | 247 | chr6 | 34203153 | 0 | 11.93 |
| 75 | 248 | chr6 | 35108921 | 1.78 | 10.76 |
| 76 | 249 | chr6 | 35109121 | 2.04 | 7.57 |
| 77 | 28 | chr1 | 67217950 | 1.35 | 23.41 |
| **78** | **250** | **chr6** | **41528623** | **1.9** | **31.06** |
| **79** | **253** | **chr6** | **42738967** | **1.06** | **48.85** |
| 80 | 256 | chr6 | 43337775 | 16.34 | 29.59 |
| 81 | 257 | chr6 | 85482570 | 3.98 | 21.58 |
| 82 | 258 | chr6 | 85482612 | 7.44 | 33.76 |
| **83** | **259** | **chr6** | **105388668** | **4.49** | **28.3** |
| **84** | **261** | **chr6** | **105388731** | **0.98** | **22.77** |
| **85** | **264** | **chr6** | **106429316** | **1.18** | **15.52** |

| # | #3 | chr# | start | H | T |
|---|---|---|---|---|---|
| 86 | 265 | chr6 | 106960491 | 0.29 | 17.71 |
| 87 | 266 | chr6 | 108495678 | 2.31 | 23.19 |
| 88 | 29 | chr1 | 67217984 | 1.85 | 23.79 |
| **89** | **267** | **chr6** | **134210946** | **4.94** | **22.15** |
| 90 | 274 | chr7 | 27213971 | 0.58 | 16.64 |
| 91 | 275 | chr7 | 39649290 | 1.05 | 29.63 |
| **92** | **276** | **chr7** | **42267719** | **0.81** | **24.58** |
| **93** | **278** | **chr7** | **45018757** | **1.17** | **46.04** |
| **94** | **279** | **chr7** | **45018789** | **7.74** | **78.95** |
| 95 | 281 | chr7 | 45961537 | 9.8 | 25.76 |
| 96 | 283 | chr7 | 73021662 | 6.65 | 33.87 |
| **97** | **284** | **chr7** | **96652153** | **1.57** | **21.3** |
| 98 | 30 | chr1 | 86081680 | 7.14 | 32.75 |
| 99 | 285 | chr7 | 96652245 | 0.43 | 9.69 |
| 100 | 286 | chr7 | 102067162 | 4.35 | 24.65 |
| **101** | **287** | **chr7** | **121939827** | **3.92** | **22.51** |
| 102 | 288 | chr7 | 150211761 | 28.96 | 67.01 |
| 103 | 289 | chr7 | 150211811 | 1.17 | 19.51 |
| 104 | 290 | chr7 | 150211820 | 3.34 | 45.24 |
| 105 | 291 | chr7 | 150211855 | 3.41 | 32.15 |
| 106 | 292 | chr8 | 22961088 | 1.79 | 12.74 |
| 107 | 295 | chr8 | 70982285 | 0.76 | 9.79 |
| 108 | 297 | chr8 | 97157856 | 0.66 | 29.18 |
| 109 | 2 | chr1 | 969257 | 0 | 22.67 |
| 110 | 31 | chr1 | 90309410 | 0.74 | 16 |
| 111 | 298 | chr8 | 97157878 | 0.49 | 33.8 |
| 112 | 299 | chr8 | 120651398 | 0.57 | 20.97 |
| **113** | **302** | **chr9** | **100616469** | **1.73** | **5.81** |
| **114** | **303** | **chr9** | **100616607** | **1.05** | **7.98** |
| **115** | **306** | **chr10** | **8097331** | **1.29** | **42.14** |
| **116** | **307** | **chr10** | **8097354** | **0.34** | **31.91** |
| 117 | 308 | chr10 | 11207527 | 0.75 | 12.74 |
| **118** | **309** | **chr10** | **11207969** | **2.15** | **16.37** |
| 119 | 310 | chr10 | 21807252 | 1.37 | 11.68 |
| **120** | **311** | **chr10** | **22541995** | **0.9** | **25.52** |
| **121** | **315** | **chr10** | **22634218** | **1.28** | **28.19** |
| **122** | **316** | **chr10** | **22634224** | **3.6** | **33.92** |
| **123** | **317** | **chr10** | **22634226** | **1.89** | **31.5** |
| **124** | **318** | **chr10** | **22634432** | **1.27** | **27.62** |
| **125** | **319** | **chr10** | **22634439** | **0.53** | **27.74** |
| 126 | 321 | chr10 | 43893015 | 0.16 | 22.36 |
| 127 | 322 | chr10 | 70847350 | 2.95 | 26.27 |
| 128 | 323 | chr10 | 70847399 | 4.35 | 22.73 |

| # | #3 | chr# | start | H | T |
|---|---|---|---|---|---|
| 129 | 324 | chr10 | 70847430 | 4.3 | 18.83 |
| 130 | 326 | chr10 | 102587110 | 0.43 | 24.21 |
| 131 | 33 | chr1 | 91301461 | 4.12 | 29.43 |
| 132 | 327 | chr10 | 102998662 | 1.24 | 16.46 |
| 133 | 328 | chr10 | 102998762 | 3.16 | 12.23 |
| 134 | 329 | chr10 | 103536348 | 0.37 | 20.39 |
| 135 | 330 | chr10 | 105036645 | 2.12 | 19.96 |
| 136 | 331 | chr10 | 118891601 | 6.45 | 41.04 |
| 137 | 334 | chr11 | 268950 | 6.5 | 18.52 |
| 138 | 335 | chr11 | 415080 | 0.2 | 11.83 |
| 139 | 336 | chr11 | 415088 | 0.47 | 12.61 |
| 140 | 337 | chr11 | 415111 | 0.42 | 14.24 |
| 141 | 338 | chr11 | 627152 | 0 | 3.05 |
| 142 | 37 | chr1 | 110186027 | 5.48 | 23.28 |
| 143 | 339 | chr11 | 627175 | 0.57 | 14.93 |
| 144 | 340 | chr11 | 637170 | 0 | 0.8 |
| 145 | 344 | chr11 | 2292691 | 0.49 | 25.63 |
| 146 | 345 | chr11 | 2292751 | 2.87 | 13.5 |
| 147 | 351 | chr11 | 2889809 | 0.19 | 1.45 |
| 148 | 352 | chr11 | 2889875 | 0.11 | 1.45 |
| 149 | 353 | chr11 | 31825226 | 1.28 | 33.47 |
| 150 | 359 | chr11 | 61062962 | 0.86 | 21.74 |
| 151 | 38 | chr1 | 110186044 | 3.05 | 16.98 |
| 152 | 360 | chr11 | 63974123 | 2.85 | 58.85 |
| 153 | 361 | chr11 | 63974131 | 4.02 | 64.89 |
| 154 | 362 | chr11 | 63974153 | 3.94 | 65.75 |
| 155 | 363 | chr11 | 63974162 | 2.66 | 51.41 |
| 156 | 366 | chr11 | 66885276 | 2.45 | 16.54 |
| 157 | 367 | chr11 | 71952131 | 2.43 | 49.4 |
| 158 | 368 | chr11 | 76382128 | 0.98 | 23.44 |
| 159 | 369 | chr11 | 76382149 | 0.87 | 32.83 |
| 160 | 370 | chr11 | 78673073 | 0.56 | 49.39 |
| 161 | 371 | chr11 | 78673100 | 1.69 | 50.28 |
| 162 | 39 | chr1 | 110611465 | 0.19 | 3.22 |
| 163 | 372 | chr11 | 82443614 | 3.55 | 11.74 |
| 164 | 373 | chr11 | 94884121 | 0.13 | 20.18 |
| 165 | 374 | chr11 | 119293861 | 0 | 20.49 |
| 166 | 375 | chr11 | 119293863 | 0.23 | 20.89 |
| 167 | 376 | chr11 | 119293869 | 0.39 | 18.73 |
| 168 | 378 | chr11 | 129243219 | 0.21 | 41.37 |
| 169 | 379 | chr12 | 3861567 | 0.47 | 3.48 |
| 170 | 380 | chr12 | 6184355 | 2.77 | 25.19 |
| 171 | 381 | chr12 | 6665288 | 0.35 | 49.12 |

| # | #3 | chr# | start | H | T |
|---|---|---|---|---|---|
| 172 | 382 | chr12 | 6665330 | 0.29 | 52.23 |
| 173 | 40 | chr1 | 113051846 | 2.01 | 19.45 |
| 174 | 383 | chr12 | 6665335 | 0.85 | 52.3 |
| 175 | 384 | chr12 | 6665370 | 0.89 | 54.93 |
| 176 | 385 | chr12 | 6665424 | 0.47 | 52.38 |
| 177 | 386 | chr12 | 6665447 | 4.75 | 53.24 |
| 178 | 387 | chr12 | 6881590 | 2.16 | 43.74 |
| 179 | 388 | chr12 | 6881595 | 3.88 | 42.43 |
| 180 | 389 | chr12 | 6881601 | 1.77 | 30.44 |
| 181 | 390 | chr12 | 6881624 | 0.31 | 18.37 |
| 182 | 391 | chr12 | 6881629 | 0.2 | 14.1 |
| 183 | 392 | chr12 | 12867753 | 2.96 | 9.64 |
| 184 | 41 | chr1 | 113051925 | 1.14 | 17.33 |
| 185 | 393 | chr12 | 14135228 | 6.61 | 31.96 |
| 186 | 394 | chr12 | 25055967 | 0.47 | 7.74 |
| 187 | 398 | chr12 | 51640305 | 1.56 | 20.84 |
| 188 | 400 | chr12 | 51718088 | 1 | 70.77 |
| 189 | 402 | chr12 | 52652220 | 1.45 | 24.75 |
| 190 | 403 | chr12 | 52652462 | 0.78 | 31.67 |
| 191 | 404 | chr12 | 53718427 | 2.26 | 17.91 |
| 192 | 406 | chr12 | 54389934 | 1.27 | 8.53 |
| 193 | 407 | chr12 | 54398765 | 0.6 | 15.75 |
| 194 | 408 | chr12 | 54398809 | 0.13 | 19.8 |
| 195 | 42 | chr1 | 119535619 | 1.36 | 4.76 |
| 196 | 409 | chr12 | 54812000 | 0.33 | 12.69 |
| 197 | 410 | chr12 | 54812085 | 0.55 | 22.61 |
| 198 | 427 | chr12 | 63207179 | 0.59 | 30.72 |
| 199 | 428 | chr12 | 66582780 | 2.03 | 23.86 |
| 200 | 429 | chr12 | 85667616 | 6.86 | 31.84 |
| 201 | 431 | chr12 | 85673200 | 3.23 | 6.73 |
| 202 | 432 | chr12 | 85673221 | 2.37 | 11.66 |
| 203 | 436 | chr12 | 128850550 | 0.82 | 26.65 |
| 204 | 437 | chr12 | 128850557 | 0.89 | 24.52 |
| 205 | 439 | chr13 | 20806730 | 0 | 0.62 |
| 206 | 44 | chr1 | 151811364 | 2.99 | 38.71 |
| 207 | 440 | chr13 | 20875915 | 1.63 | 15.29 |
| 208 | 445 | chr13 | 28527255 | 2.23 | 32.36 |
| 209 | 446 | chr13 | 30945876 | 1.78 | 32.07 |
| 210 | 447 | chr13 | 41556559 | 3.53 | 6.02 |
| 211 | 448 | chr13 | 53313174 | 0.54 | 11.39 |
| 212 | 449 | chr13 | 95360278 | 6.83 | 31.42 |
| 213 | 452 | chr14 | 22362410 | 3.73 | 21.66 |
| 214 | 454 | chr14 | 29254853 | 5.47 | 25.32 |

| # | #3 | chr# | start | H | T |
|---|---|---|---|---|---|
| 215 | 455 | chr14 | 38724648 | 0.79 | 16.24 |
| 216 | 456 | chr14 | 65565498 | 0.9 | 12.65 |
| 217 | 4 | chr1 | 1566687 | 2.15 | 33.83 |
| 218 | 45 | chr1 | 160983767 | 0 | 30.64 |
| 219 | 457 | chr14 | 70653964 | 0 | 0.33 |
| 220 | 458 | chr14 | 102027797 | 5.66 | 18.57 |
| 221 | 459 | chr14 | 102172296 | 0 | 6.38 |
| 222 | 460 | chr14 | 105512071 | 1.43 | 35.45 |
| 223 | 461 | chr14 | 105512213 | 0 | 25.81 |
| 224 | 462 | chr14 | 105512268 | 0.12 | 31.96 |
| 225 | 463 | chr14 | 105512306 | 0.2 | 34.1 |
| 226 | 464 | chr15 | 40600265 | 1.28 | 43.57 |
| 227 | 465 | chr15 | 40600279 | 3.1 | 51.4 |
| 228 | 466 | chr15 | 40600284 | 4.54 | 53.2 |
| 229 | 46 | chr1 | 192544716 | 0.32 | 3.64 |
| 230 | 468 | chr15 | 41787780 | 0.68 | 36.7 |
| 231 | 469 | chr15 | 44092606 | 3.33 | 6.88 |
| 232 | 471 | chr15 | 57592222 | 4.03 | 20.91 |
| 233 | 472 | chr15 | 65689152 | 2.78 | 14.99 |
| 234 | 475 | chr15 | 82555336 | 0.53 | 0.59 |
| 235 | 476 | chr15 | 85360319 | 10.63 | 43.56 |
| 236 | 477 | chr16 | 3265396 | 3.97 | 3.35 |
| 237 | 478 | chr16 | 3265467 | 1.54 | 7.11 |
| 238 | 480 | chr16 | 28996329 | 0 | 3.27 |
| 239 | 47 | chr1 | 197888880 | 1.86 | 17.77 |
| 240 | 481 | chr16 | 28996358 | 0 | 3.33 |
| 241 | 482 | chr16 | 28996362 | 0 | 6.87 |
| 242 | 483 | chr16 | 30103978 | 1.36 | 9.85 |
| 243 | 486 | chr16 | 54970523 | 1.71 | 32.21 |
| 244 | 488 | chr16 | 55544136 | 3.04 | 28.47 |
| 245 | 493 | chr16 | 67199463 | 2.12 | 26.74 |
| 246 | 496 | chr16 | 89006335 | 3.84 | 12.83 |
| 247 | 498 | chr16 | 89034797 | 10.88 | 13.32 |
| 248 | 499 | chr16 | 89778247 | 0 | 0 |
| 249 | 506 | chr17 | 4648566 | 4.33 | 23.78 |
| 250 | 49 | chr1 | 202129865 | 0.61 | 30.86 |
| 251 | 507 | chr17 | 4648580 | 4.42 | 24.32 |
| 252 | 508 | chr17 | 6358928 | 0.76 | 36.34 |
| 253 | 509 | chr17 | 7341641 | 0 | 26.72 |
| 254 | 511 | chr17 | 8770954 | 1.26 | 16.09 |
| 255 | 512 | chr17 | 8771003 | 1.85 | 24.03 |
| 256 | 513 | chr17 | 8771055 | 0 | 4.86 |
| 257 | 514 | chr17 | 8771114 | 0.8 | 3.15 |

| # | #3 | chr# | start | H | T |
|---|---|---|---|---|---|
| 258 | 515 | chr17 | 9143754 | 10.61 | 8.31 |
| 259 | 516 | chr17 | 16284307 | 0.8 | 10.46 |
| 260 | 50 | chr1 | 219347279 | 0.49 | 32.67 |
| 261 | 517 | chr17 | 27940509 | 0.72 | 14.2 |
| 262 | 522 | chr17 | 40440904 | 4.3 | 18.21 |
| 263 | 523 | chr17 | 40440916 | 7.73 | 40.29 |
| 264 | 524 | chr17 | 42287971 | 0 | 36.08 |
| 265 | 525 | chr17 | 53341243 | 0.2 | 10.66 |
| 266 | 527 | chr17 | 56408804 | 0.57 | 23.09 |
| 267 | 528 | chr17 | 56409518 | 10.26 | 76.78 |
| 268 | 529 | chr17 | 56409534 | 7.18 | 72.58 |
| 269 | 530 | chr17 | 58498977 | 0.45 | 26.77 |
| 270 | 534 | chr17 | 61778515 | 0.82 | 30.16 |
| 271 | 51 | chr1 | 219347340 | 0.18 | 34.85 |
| 272 | 535 | chr17 | 61778550 | 1.62 | 27.35 |
| 273 | 539 | chr17 | 76128481 | 1.36 | 14.8 |
| 274 | 540 | chr17 | 76128556 | 6.13 | 14.73 |
| 275 | 544 | chr17 | 80186336 | 1.72 | 15.89 |
| 276 | 551 | chr18 | 29265840 | 2.15 | 24.24 |
| 277 | 552 | chr18 | 29265842 | 1.52 | 23.57 |
| 278 | 553 | chr18 | 46461202 | 0 | 1.23 |
| 279 | 555 | chr19 | 1069320 | 2.47 | 8.81 |
| 280 | 52 | chr1 | 219347410 | 0.21 | 39.14 |
| 281 | 557 | chr19 | 1851882 | 3.61 | 22.35 |
| 282 | 558 | chr19 | 1851995 | 1.74 | 20.71 |
| 283 | 563 | chr19 | 6481857 | 1.16 | 28.61 |
| 284 | 564 | chr19 | 11354132 | 0.15 | 25.24 |
| 285 | 568 | chr19 | 15342915 | 1.08 | 9.49 |
| 286 | 570 | chr19 | 17462455 | 4.29 | 17.78 |
| 287 | 576 | chr20 | 3052692 | 0 | 14.63 |
| 288 | 577 | chr20 | 25129126 | 0.12 | 19.5 |
| 289 | 53 | chr1 | 219347458 | 0.24 | 37.1 |
| 290 | 579 | chr20 | 30639305 | 0.53 | 32.16 |
| 291 | 582 | chr20 | 35274639 | 5.89 | 70.65 |
| 292 | 583 | chr20 | 35274655 | 3.02 | 61.55 |
| 293 | 584 | chr20 | 44879700 | 4.15 | 41.73 |
| 294 | 585 | chr20 | 55200146 | 0 | 18.13 |
| 295 | 586 | chr20 | 55965245 | 3.91 | 14.37 |
| 296 | 587 | chr20 | 56025557 | 2.14 | 37.42 |
| 297 | 588 | chr20 | 57224919 | 0.93 | 26.04 |
| 298 | 589 | chr20 | 61560627 | 0.09 | 8.7 |
| 299 | 590 | chr20 | 62369462 | 0 | 10.41 |
| 300 | 54 | chr1 | 219834537 | 2.56 | 24.14 |

| # | #3 | chr# | start | H | T |
|---|---|---|---|---|---|
| 301 | 596 | chr22 | 19711051 | 1.96 | 10.75 |
| 302 | 609 | chr22 | 37813098 | 1.91 | 2.15 |
| 303 | 611 | chr22 | 44577211 | 3.27 | 11.48 |
| 304 | 612 | chr22 | 44577222 | 3 | 7.5 |
| 305 | 613 | chr22 | 44577265 | 3.74 | 14.02 |
| 306 | 614 | chr22 | 50623687 | 0.78 | 13.31 |
| 307 | 615 | chr22 | 50623692 | 1.26 | 18.16 |
| 308 | 616 | chr22 | 50986962 | 0.8 | 17.25 |
| 309 | 55 | chr1 | 219834581 | 1.25 | 23.9 |
| 310 | 4 | chr1 | 1566699 | 0 | 20.64 |
| 311 | 56 | chr1 | 221050459 | 1.83 | 25.63 |
| 312 | 57 | chr1 | 221050491 | 3.56 | 42.07 |
| 313 | 58 | chr1 | 224805753 | 0.85 | 20.41 |
| 314 | 59 | chr1 | 228463634 | 1.29 | 26.06 |
| 315 | 60 | chr1 | 231115871 | 1.47 | 7.79 |
| 316 | 61 | chr1 | 235099151 | 3.05 | 5.83 |
| 317 | 64 | chr2 | 25439110 | 0.29 | 6.45 |
| 318 | 67 | chr2 | 38302230 | 4.77 | 34.9 |
| 319 | 68 | chr2 | 43019854 | 1.02 | 17.85 |
| 320 | 69 | chr2 | 45231382 | 0.31 | 5.95 |
| 321 | 9 | chr1 | 6086254 | 1.01 | 14.87 |
| 322 | 70 | chr2 | 63281069 | 1.53 | 32.47 |
| 323 | 71 | chr2 | 63281139 | 0.77 | 27.22 |
| 324 | 74 | chr2 | 63284066 | 0.92 | 24.99 |
| 325 | 75 | chr2 | 63284132 | 5.8 | 29.72 |
| 326 | 76 | chr2 | 71192445 | 1.22 | 25.39 |
| 327 | 77 | chr2 | 85804732 | 1.26 | 11.5 |
| 328 | 79 | chr2 | 86263270 | 2.08 | 40.97 |
| 329 | 80 | chr2 | 96990861 | 4.04 | 16.76 |
| 330 | 81 | chr2 | 99439533 | 0.87 | 22.16 |
| 331 | 82 | chr2 | 99439883 | 13.28 | 45.03 |
| 332 | 10 | chr1 | 6086275 | 0.58 | 20.72 |
| 333 | 87 | chr2 | 127414381 | 1.68 | 28.89 |
| 334 | 88 | chr2 | 127414455 | 1.08 | 30.37 |
| 335 | 90 | chr2 | 157177008 | 0.76 | 29.54 |
| 336 | 91 | chr2 | 162279964 | 2.5 | 24.4 |
| 337 | 92 | chr2 | 162281111 | 6.51 | 35.41 |
| 338 | 93 | chr2 | 171573419 | 0.75 | 35.77 |

| # | #3 | chr# | start | H | T |
|---|---|---|---|---|---|
| 339 | 94 | chr2 | 200329872 | 6.78 | 17.81 |
| 340 | 95 | chr2 | 200334001 | 1.36 | 15.95 |
| 341 | 96 | chr2 | 202125212 | 2.08 | 76.15 |
| 342 | 97 | chr2 | 219736250 | 0.64 | 38.49 |
| 343 | 16 | chr1 | 27687232 | 1.05 | 32.89 |
| 344 | 100 | chr2 | 228736253 | 0.97 | 15.16 |
| 345 | 102 | chr2 | 228736449 | 0.61 | 12.07 |
| 346 | 103 | chr2 | 233925001 | 0.41 | 1.33 |
| 347 | 106 | chr3 | 4910524 | 0.23 | 25.53 |
| 348 | 107 | chr3 | 9904411 | 1.01 | 23.97 |
| 349 | 108 | chr3 | 9904557 | 0.08 | 22.86 |
| 350 | 109 | chr3 | 43431343 | 1.56 | 54.27 |
| 351 | 110 | chr3 | 50310766 | 0.56 | 12.85 |
| 352 | 112 | chr3 | 50378529 | 1.19 | 25.89 |
| 353 | 114 | chr3 | 61550379 | 0.77 | 36.01 |
| 354 | 17 | chr1 | 35351340 | 1.7 | 20.42 |
| 355 | 115 | chr3 | 62861055 | 4.62 | 26.93 |
| 356 | 116 | chr3 | 113252289 | 0.14 | 15.57 |
| 357 | 118 | chr3 | 121379777 | 1.34 | 53.64 |
| 358 | 119 | chr3 | 121379791 | 1.58 | 53.27 |
| 359 | 120 | chr3 | 129693489 | 0.13 | 22.65 |
| 360 | 121 | chr3 | 141516232 | 0.38 | 46.87 |
| 361 | 122 | chr3 | 147126753 | 1.29 | 14.31 |
| 362 | 123 | chr3 | 147126961 | 14.76 | 30.1 |
| 363 | 124 | chr3 | 147127010 | 8.13 | 25.35 |
| 364 | 125 | chr3 | 147127012 | 10.26 | 27.52 |
| 365 | 18 | chr1 | 36042986 | 0.56 | 48.88 |
| 366 | 132 | chr3 | 147140880 | 7.17 | 28.22 |
| 367 | 133 | chr3 | 147140930 | 1.46 | 18.05 |
| 368 | 134 | chr3 | 150996297 | 5.39 | 53.15 |
| 369 | 135 | chr3 | 157261086 | 0.9 | 27.8 |
| 370 | 136 | chr3 | 157812475 | 7.28 | 37.42 |
| 371 | 137 | chr3 | 160167990 | 8.2 | 37.16 |
| 372 | 138 | chr3 | 171175950 | 0.16 | 12.57 |
| 373 | 139 | chr3 | 171176016 | 0.34 | 19.08 |
| 374 | 140 | chr3 | 181444870 | 0.24 | 14.67 |
| 375 | 141 | chr3 | 183273557 | 1.77 | 13.62 |

**Table 8.** Individual CpGs located in a probe corresponding to a CpG cluster but not comprised in the CpG cluster (CpGs listed in Table 4 as bracketed CpGs). Individual CpGs are defined by start position ("start") on indicated chromosome ("chr #"). A general number ("#") is given to each CpG. #CpG Table 3: CpG number ("#") as used in Table 3; H: percentage co-methylation of the listed CpG with methylation of the CpG cluster corresponding to the probe in healthy reference cfDNA samples (n=49); T: percentage co-methylation of the listed CpG with methylation of the CpG cluster corresponding to the probe in ovarian cancer cfDNA samples (HGSOC; n=25). As threshold for co-methylation were taken reads comprising the listed CpG with at least 80% of the CpGs in these reads being methylated. Empty cells: to be determined.

| # | #CpG Table 3 | chr# | start | H | T |
|---|---|---|---|---|---|
| 1 | 17 | chr1 | 35351340 | 0 | 0.11 |
| 2 | 75 | chr2 | 63284132 | 0 | 0.13 |
| 3 | 91 | chr2 | 162279964 | 0 | 0.11 |
| 4 | 100 | chr2 | 228736253 | 0 | 0.11 |
| 5 | 103 | chr2 | 233925001 | 0.02 | 0.19 |
| 6 | 112 | chr3 | 50378529 | 0.02 | 0.08 |
| 7 | 122 | chr3 | 147126753 | 0.01 | 0.1 |
| 8 | 124 | chr3 | 147127010 | 0.01 | 0.23 |
| 9 | 125 | chr3 | 147127012 | 0.01 | 0.23 |
| 10 | 132 | chr3 | 147140880 | 0 | 0.12 |
| 11 | 25 | chr1 | 50513766 | | |
| 12 | 26 | chr1 | 50513870 | | |
| 13 | 33 | chr1 | 91301461 | 0.01 | 0.13 |
| 14 | 133 | chr3 | 147140930 | 0 | 0.12 |
| 15 | 147 | chr4 | 13524143 | 0 | 0.11 |
| 16 | 162 | chr4 | 155411806 | 0 | 0.15 |
| 17 | 173 | chr5 | 40681893 | 0 | 0.19 |
| 18 | 188 | chr5 | 132150117 | 0.01 | 0.13 |
| 19 | 198 | chr5 | 169724831 | 0.03 | 0.2 |
| 20 | 234 | chr6 | 30653799 | 0.01 | 0.33 |
| 21 | 250 | chr6 | 41528623 | 0.01 | 0.31 |
| 22 | 42 | chr1 | 119535619 | 0 | 0.07 |
| 23 | 253 | chr6 | 42738967 | 0.02 | 0.36 |
| 24 | 259 | chr6 | 105388668 | 0 | 0.19 |
| 25 | 261 | chr6 | 105388731 | 0 | 0.17 |
| 26 | 264 | chr6 | 106429316 | 0 | 0.1 |
| 27 | 267 | chr6 | 134210946 | 0 | 0.14 |
| 28 | 276 | chr7 | 42267719 | 0 | 0.25 |
| 29 | 277 | chr7 | 42267747 | | |
| 30 | 278 | chr7 | 45018757 | 0.05 | 0.41 |
| 31 | 279 | chr7 | 45018789 | 0.06 | 0.42 |
| 32 | 280 | chr7 | 45018849 | | |
| 33 | 284 | chr7 | 96652153 | 0 | 0.11 |
| 34 | 44 | chr1 | 151811364 | 0.01 | 0.28 |

(continued)

| # | #CpG Table 3 | chr# | start | H | T |
|---|---|---|---|---|---|
| 35 | 287 | chr7 | 121939827 | 0.01 | 0.14 |
| 36 | 302 | chr9 | 100616469 | 0 | 0.04 |
| 37 | 303 | chr9 | 100616607 | 0 | 0.09 |
| 38 | 304 | chr9 | 122132103 | | |
| 39 | 306 | chr10 | 8097331 | 0 | 0.18 |
| 40 | 307 | chr10 | 8097354 | 0 | 0.18 |
| 41 | 309 | chr10 | 11207969 | 0.01 | 0.1 |
| 42 | 311 | chr10 | 22541995 | 0 | 0.11 |
| 43 | 315 | chr10 | 22634218 | 0 | 0.22 |
| 44 | 316 | chr10 | 22634224 | 0 | 0.22 |
| 45 | 317 | chr10 | 22634226 | 0 | 0.22 |
| 46 | 318 | chr10 | 22634432 | | |
| 47 | 319 | chr10 | 22634439 | | |
| 48 | 329 | chr10 | 103536348 | 0 | 0.09 |
| 49 | 331 | chr10 | 118891601 | 0 | 0.07 |
| 50 | 366 | chr11 | 66885276 | 0 | 0.06 |
| 51 | 368 | chr11 | 76382128 | 0 | 0.12 |
| 52 | 369 | chr11 | 76382149 | 0 | 0.14 |
| 53 | 379 | chr12 | 3861567 | 0.02 | 0.06 |
| 54 | 380 | chr12 | 6184355 | 0.02 | 0.09 |
| 55 | 393 | chr12 | 14135228 | 0 | 0.09 |
| 56 | 428 | chr12 | 66582780 | 0 | 0.05 |
| 57 | 431 | chr12 | 85673200 | 0 | 0.1 |
| 58 | 432 | chr12 | 85673221 | 0 | 0.1 |
| 59 | 436 | chr12 | 128850550 | 0 | 0.14 |
| 60 | 437 | chr12 | 128850557 | 0 | 0.15 |
| 61 | 445 | chr13 | 28527255 | 0 | 0.08 |
| 62 | 455 | chr14 | 38724648 | 0 | 0.09 |
| 63 | 458 | chr14 | 102027797 | 0 | 0.14 |
| 64 | 472 | chr15 | 65689152 | 0.02 | 0.14 |
| 65 | 486 | chr16 | 54970523 | 0 | 0.11 |
| 66 | 493 | chr16 | 67199463 | 0.01 | 0.18 |
| 67 | 498 | chr16 | 89034797 | 0.03 | 0.1 |
| 68 | 515 | chr17 | 9143754 | 0.01 | 0.15 |
| 69 | 544 | chr17 | 80186336 | 0.01 | 0.09 |
| 70 | 551 | chr18 | 29265840 | 0 | 0.06 |
| 71 | 552 | chr18 | 29265842 | 0 | 0.07 |
| 72 | 563 | chr19 | 6481857 | 0 | 0.13 |
| 73 | 568 | chr19 | 15342915 | 0 | 0.13 |

(continued)

| # | #CpG Table 3 | chr# | start | H | T |
|---|---|---|---|---|---|
| 74 | 573 | chr19 | 51232007 | | |
| 75 | 574 | chr19 | 52104632 | | |
| 76 | 577 | chr20 | 25129126 | 0 | 0.18 |
| 77 | 614 | chr22 | 50623687 | 0 | 0.08 |
| 78 | 615 | chr22 | 50623692 | 0 | 0.07 |
| 79 | 74 | chr2 | 63284066 | 0 | 0.17 |

### 2.5. Diagnostic performance

We studied a cohort of cfDNA samples from 108 patients with adnexal masses and 43 samples from healthy individuals (control samples). Of these samples, low-coverage whole-genome-sequencing (LC-WGS) data was available and genome-wide z-scores and nucleosome scores have been previously calculated for each sample. In addition, we sequenced 25 HGSOC tumor tissue samples to use as a positive reference set in the training set. We randomly partitioned the 43 control samples into 4 folds to enable 4-fold cross-validation of our results (see Figure 7). In each fold, we extracted all methylation patterns from the tumor tissue and control plasma samples on a read by read basis and determined pattern beta distributions in a tumor and healthy class, respectively. These beta distributions were subsequently used in the cohort of test set samples to determine individual methylation scores. An overview of the methylation pipeline is illustrated in Figure 8.

The results of the test set methylation scores are shown in Figure 9. We noticed that samples from controls and patients with benign adnexal masses displayed the lowest methylation score. In contrast, elevated methylation scores were found for borderline ovarian carcinoma (BOT) samples, and the highest scores were observed for invasive ovarium cancer patients. Since ovarian carcinomas may contain heterogeneous methylation patterns, it is not unexpected that we also find invasive plasma samples with a low methylation score. Moreover, not all filtered patterns are covered across all test samples due to coverage differences, i.e. sometimes the pattern coverage is below 10X in a particular sample region of interest. We observed an elevated methylation score in 2 of the 4 metastatic plasma samples.

We next assessed which metric, either genome-wide z-score, nucleosome score or methylation score, is able to distinguish, in the same set of samples (not fully the same as in Example 1) healthy plasma samples from ovarium cancer plasma samples most effectively (see Table 7). The genome-wide z-score is the most predictive metric for invasive and HGSOC cases, with AUC values of 0.82 and 0.92 respectively. However, for non-HGSOC cases the AUC value of genome-wide z-score is only 0.69, while nucleosome score and methylation score become more favorable with AUC values of 0.79 and 0.77 respectively. When BOT & invasive or BOT samples are assessed, the methylation score has the highest predictivity with AUC values of 0.78 and 0.73 respectively.

### EXAMPLE 3. Combinations of genome-wide copy number alteration, nucleosome footprints and/or methylation of cfDNA of patients with different stages of ovarian cancer

To assess if the methylation score acts complementary with the other metrics obtained from LC-WGS (genome wide Z-score and genome-wide nucleosome score), the scores were combined into a single predictor and corresponding ROC curve by fitting a logistic regression model with ranks of the scores. Optimism of the AUC value of the combined predictor was estimated using 500 non-parametric bootstrap iterations and subtracted to obtain an unbiased estimate of performance.

We also investigated whether the combination of two or three scores results in an improved predictivity. As shown in Table 9, we obtain an AUC-value of 0.92 when combining genome-wide z-score and methylation score for the prediction of HGSOC cases. In contrast, the combination of nucleosome score and methylation score is preferred for non-HGSOC sample and BOT samples. Combining the three methods does not increase the predictivity even further for HGSOC samples, but we find improved AUC values for the group of invasive samples (AUC of 0.87) and BOT & invasive (AUC of 0.81). The latter improvements are of course of importance in the context of early or earlier detection of ovarian cancer. These results show that the different scores can act complementary in distinguishing healthy from cancer plasma samples. Moreover, we observed that a combination with the methylation score gave the best predictivity in each comparison of the different sample types. As an illustration, the ROC curves of invasive samples versus benign samples are shown in Figure 10.

**Table 9.** Diagnostic power (AUC values) of single methodologies, and of double and triple combinations of methodologies.

| Test set | | single metric | | |
|---|---|---|---|---|
| | | AUC meth | AUC gw-z | AUC nucl |
| benign (n=26) | invasive (n=55) | 0.79 | 0.82 | 0.79 |
| benign (n=26) | HGSOC (n=32) | 0.81 | 0.92 | 0.79 |
| benign (n=26) | non-HGSOC (n=23) | 0.77 | 0.68 | 0.78 |
| benign (n=26) | BOT & invasive (n=78) | 0.77 | 0.72 | 0.76 |
| benign (n=26) | BOT (n=23) | 0.7 | 0.49 | 0.65 |

| Test set | | combination 2 metrics | | |
|---|---|---|---|---|
| | | AUC gw-z + meth | AUC nucl + meth | AUC gw-z + nucl |
| benign (n=26) | invasive (n=55) | 0.87 | 0.83 | 0.83 |
| benign (n=26) | HGSOC (n=32) | 0.92 | 0.84 | 0.91 |
| benign (n=26) | non-HGSOC (n=23) | 0.77 | 0.81 | 0.77 |
| benign (n=26) | BOT & invasive (n=78) | 0.8 | 0.83 | 0.77 |
| benign (n=26) | BOT (n=23) | 0.67 | 0.69 | 0.61 |

| Test set | | combination 3 metrics |
|---|---|---|
| | | AUC gw-z + nucl + meth |
| benign (n=26) | invasive (n=55) | 0.87 |
| benign (n=26) | HGSOC (n=32) | 0.91 |
| benign (n=26) | non-HGSOC (n=23) | 0.78 |
| benign (n=26) | BOT & invasive (n=78) | 0.81 |
| benign (n=26) | BOT (n=23) | 0.67 |

AUC meth: AUC value obtained by DNA methylation analysis
AUC gw-z: AUC value obtained by genome-wide CNA analysis
AUC nucl: AUC value obtained by genome-wide nucleosome footprint analysis
AUC gw-z + meth: AUC value obtained by genome-wide CNA analysis combined with DNA methylation analysis
AUC nucl + meth: AUC value obtained by genome-wide nucleosome footprint analysis combined with DNA methylation analysis
AUC gw-z + nucl: AUC value obtained by genome-wide CNA analysis combined with genome-wide nucleosome footprint analysis
AUC gw-z + nucl + meth: AUC value obtained by genome-wide CNA analysis combined with genome-wide nucleosome footprint analysis and combined with DNA methylation analysis

**EXAMPLE 4. Selection of CpG methylation patterns**

Although requiring further optimization, an initial attempt to further reduce the amount of CpG clusters that needs to be analyzed without losing reliable predictive power was performed. PAM (prediction analysis for microarrays) analysis (nearest shrunken centroid classification) was applied to the CpG clusters, and this specifically for HGSOC cancer samples. From this initial, non-optimized attempt, it appeared that coverage of 12 CpG clusters is not decreasing the diagnostic power as indicated by unchanged or even increased AUC values. This illustrates that DNA methylation analysis of all 211 CpG clusters is not required and that DNA methylation analysis of a limited number of CpG clusters is sufficient.
The 12 CpG clusters resulting from this initial attempt are: chr1:9714299-9714408, chr2: 25496257-25496390, chr3:50312802-50312975, chr3: 50312992-50313009, chr7:45018734-45018964, chr11:47416442-47416635, chr11:65661461-65661584, chr12:51717740-51717947, chr12:51718011-51718224, chr12: 85672699-85672731, chr17:32964454-32964735, and chr17:80358752-80358932.

The resulting AUC values are included hereafter in Table 10.

**Table 10.** Diagnostic power (AUC values) of single methodologies, and of double and triple combinations of methodologies relying on 12 CpG clusters for DNA methylation analysis.

| Test set | | single metric | | |
|---|---|---|---|---|
| | | AUC meth | AUC gw-z | AUC nucl |
| benign (n=26) | HGSOC (n=32) | 0.82 | 0.92 | 0.79 |

| Test set | | combination 2 metrics | | |
|---|---|---|---|---|
| | | AUC gw-z + meth | AUC nucl + meth | AUC gw-z + nucl |
| benign (n=26) | HGSOC (n=32) | 0.93 | 0.84 | 0.91 |

| Test set | | combination 3 metrics |
|---|---|---|
| | | AUC gw-z + nucl + meth |
| benign (n=26) | HGSOC (n=32) | 0.92 |

**EXAMPLE 5. Analysis of larger number of ovarian cancer cfDNA samples.**

In expanding the number of individual ovarian cancer cfDNA samples, a cohort of cfDNA samples from 265 patients with adnexal masses and 115 samples from healthy individuals (control samples) were studied (see Figure 11). Of these samples, low-coverage whole-genome-sequencing (LC-WGS) data was available and genome-wide z-scores and nucleosome scores have been previously calculated for each sample. Genome-wide z-scores, nucleosome scores and cfDNA methylation profiling was performed as described in Examples 1 and 2. We randomly partitioned 80 invasive and 94 control samples into 10 folds to enable 10-fold cross-validation of our results (see Figure 12). In each fold, we calculated the mean methylation rate of all CpGs in the probe regions from the invasive and control plasma samples. A random forest model based on the mean methylation rates was subsequently used in the cohort of test set samples to determine individual methylation scores. Of note, the methylation score in this analysis was thus determined in a slightly different way as compared to the methodology outlined in Example 2.4; this in an effort to demonstrate robustness of the cfDNA methylation profile as obtained with the selected CpGs/selected probes covering these CpGs.

After the training step, we quantified the methylation score in cfDNA collected from plasma samples in a test set using the random forest probability, ranging from 0 (no tumor content) to 1 (only tumor content). To assess the predictivity of the methylation score, we constructed receiver operation characteristic (ROC) curve and calculated the corresponding area under the curve (AUC) values using the pROC package in R (Robin et al. 2011, BMC Bioinformatics 12:77). We used the GNU Parallels tool for simulation of each fold in the cross validation (Tange 2011, USENIX Mag 36:42-47). The caret package was used for creation of the random forest models.

The results of the test set methylation scores are shown in Figure 13. We noticed that samples from controls and patients with benign adnexal masses displayed the lowest methylation score. In contrast, elevated methylation scores were found for borderline ovarian carcinoma (BOT) samples, and the highest scores were observed for invasive ovarium cancer patients. Since ovarian carcinomas may contain heterogenous methylation patterns, it is not fully unexpected that we also find invasive plasma samples with a low methylation score. Moreover, not all probe regions are covered across all test samples due to coverage differences, i.e. sometimes the probe coverage is below 10X in a particular sample region of interest.

We next assessed which metric, either genome-wide z-score, nucleosome score or methylation score, is able to distinguish healthy plasma samples from ovarium cancer plasma samples most effectively (see Table 11). The genome-wide z-score is the most predictive metric for invasive and HGSOC cases, with AUC values of 0.81 and 0.90 respectively. However, for non-HGSOC cases the AUC value of genome-wide z-score is only 0.61, while nucleosome score and methylation score became more favorable with AUC values of 0.76 and 0.75 respectively. When BOT & invasive or BOT samples were assessed, the methylation score has the highest predictivity with AUC values of 0.78 and 0.68 respectively.

We also investigated whether the combination of two or three scores results in an improved predictivity. The scores were combined into a single predictor and corresponding ROC curve by fitting a logistic regression model with ranks of the scores. Optimism of the AUC value of the combined predictor was estimated using 500 non-parametric bootstrap iterations and subtracted to obtain an unbiased estimate of performance. As shown in Table 11, we obtained an AUC-value of 0.91 when combining genome-wide z-score and methylation score for the prediction of HGSOC cases. In contrast, the combination of nucleosome score and methylations score was preferred for non-HGSOC sample and BOT samples. By combining the three methods, we found improved AUC values for the group of invasive samples (AUC of 0.86). These results show that the different scores can act complementary in distinguishing healthy from cancer plasma samples. Moreover, we observed that a combination including the methylation score gave the best predictivity in each comparison of the different sample types. As an illustration, the ROC curves of invasive samples versus benign samples are shown in Figure 14.

**Table 11. Summary of the predictivity of each metric: methylation score (meth), genome-wide z-score (gw-z) and nucleosome score (nucl).**

| **Test set** | | Single metric | | | Combination 2 metrics | | | Combination 3 metrics |
|---|---|---|---|---|---|---|---|---|
| | | AUC gw-z | AUC nucl | AUC meth | AUC gw-z + meth | AUC nucl + meth | AUC gw-z + nucl | AUC gw-z + nucl + meth |
| benign (n = 91) | BOT & invasive (n=118) | 0.73 | 0.74 | 0.78 | 0.78 | 0.8 | 0.76 | 0.79 |
| | invasive (n=80) | 0.81 | 0.79 | 0.83 | 0.85 | 0.85 | 0.83 | 0.86 |
| | BOT (n=38) | 0.56 | 0.63 | 0.68 | 0.68 | 0.69 | 0.63 | 0.68 |
| | HGSOC (n = 54) | 0.9 | 0.81 | 0.87 | 0.91 | 0.88 | 0.9 | 0.91 |
| | non-HGSOC (n=26) | 0.61 | 0.76 | 0.75 | 0.73 | 0.79 | 0.75 | 0.79 |

**EXAMPLE 6. Analysis of cfDNA samples of cancers metastasized to ovaries.**

To assess the efficacy of our model to distinguish tumor types different from ovarian cancer, we investigated eight samples with metastatic disease to the ovaries in more detail (Table 12). The metastatic samples represented a different tumor origin than ovarian cancer, yet five samples demonstrated elevated methylation scores (above 0.5) and were hence detected by methylation cfDNA profiling. On the other hand, two samples showed elevated genome wide z-scores (above 100), while wonly four samples exhibited elevated nucleosome scores (above 0.5). Only the colon and melanoma samples had elevated scores for all 3 scores, while a combination of scores was necessary for the other samples, indicating that our approach was applicable to detect tumor types other that ovarian cancer.

**Table 12. Summary of methylation score, genome-wide z-score and nucleosome score for metastasis samples.**

| metastasis sample | origin | methylation score | genome-wide z-score | nucleosome score |
|---|---|---|---|---|
| 1 | colon | 0.8 | 174 | 1 |
| 2 | gastric | 0.85 | -0.62 | 0 |
| 3 | gastric | 0.65 | 0.22 | 1 |
| 4 | thyroid | 0.41 | 1.22 | 0 |
| 5 | gastric | 0.38 | 0.37 | 0 |
| 6 | melanoma | 0.72 | 293 | 0.99 |
| 7 | peritoneum | 0.62 | 0.07 | 0.57 |
| 8 | anal canal | 0.29 | 0.81 | 0 |

## Claims

**1.** A method for analyzing cell free DNA (cfDNA), the method comprising:

obtaining cell-free DNA (cfDNA) from a biological sample obtained from a subject and analyzing the obtained cfDNA, wherein the analysis of the cfDNA consists of:

- analyzing the presence of DNA methylation in the obtained cfDNA on a disease-specific set of cfDNA CpGs or on a disease-specific set of cfDNA CpG clusters with a mean average methylation ß-value of less than 0.03 in cfDNA of healthy subjects; and
- analyzing a nucleosome footprint of the obtained cfDNA;

or wherein the analysis of the cfDNA consists of:

- analyzing the presence of DNA methylation in the obtained cfDNA on a disease-specific set of cfDNA CpGs or on a disease-specific set of cfDNA CpG clusters with a mean average methylation ß-value of less than 0.03 in cfDNA of healthy subjects;
- analyzing a nucleosome footprint of the obtained cfDNA; and
- analyzing copy number alteration (CNA) in the obtained cfDNA.

**2.** The method according to claim 1 wherein the nucleosome footprint is a genome-wide nucleosome footprint and/or wherein the copy number alteration analysis is a genome-wide copy number alteration analysis.

**3.** The method according to any of claims 1 to 2 further comprising calculating a sample cfDNA methylation score which is the DNA methylation score for the cfDNA on which the presence of DNA methylation has been analyzed and calculating the sample cfDNA nucleosome score which is the nucleosome score for the obtained cfDNA; or further comprising calculating a sample cfDNA methylation score which is the DNA methylation score for the cfDNA on which the presence of DNA methylation has been analyzed, calculating the sample cfDNA nucleosome score which is the nucleosome score for the obtained cfDNA, and calculating the sample cfDNA CNA score which is the CNA score for the obtained cfDNA.

**4.** The method according to any one of claims 1 to 3 further comprising determination of a disease or disorder likely to be present in the subject when the DNA methylation in the sample cfDNA is deviating from the DNA methylation of reference cfDNA or when the sample cfDNA methylation score is deviating from the reference cfDNA methylation score; and when the sample cfDNA nucleosome score is deviating from the reference cfDNA nucleosome score; or further comprising determination of a disease or disorder likely to be present in the subject when the DNA methylation in the sample cfDNA is deviating from the DNA methylation of reference cfDNA or when the sample cfDNA methylation score is deviating from the reference cfDNA methylation score; when the sample cfDNA nucleosome score is deviating from the reference cfDNA nucleosome score; and when the sample cfDNA CNA score is deviating from the reference cfDNA CNA score.

**5.** The method according to claim 3 wherein the sample cfDNA methylation score, the sample cfDNA nucleosome score and the sample cfDNA CNA score are combined in a sample cfDNA single score.

**6.** The method according to claim 5 further comprising determination of a disease or disorder likely to be present in the subject when the sample cfDNA single score is deviating from the reference cfDNA single score.

**7.** The method according to any one of claims 1 to 6 wherein the nucleosome footprint or CNA is analyzed via low-coverage sequencing, ultralow-pas sequencing, shallow sequencing, or by random non-targeted sequencing.

**8.** The method according to any one of the foregoing claims wherein the biological sample is blood, serum or plasma.

**9.** The method according to any one of the foregoing claims wherein at least one analysis or calculation step is performed by a computer system or via a computer program product.

**10.** The method according to any one of the preceding claims which is a method for diagnosing a disease or disorder in a subject, for detecting the presence of a disease or disorder in a subject, for early detection of a disease or disorder in a subject, for early diagnosis of a disease or disorder in a subject, for screening for the presence of a disease or disorder in a subject, for determining an increased likelihood for a disease or disorder to be present in a subject, for monitoring a disease or disorder in a subject, for determining a response of a disease or disorder to therapy, for monitoring a disease or disorder after therapy, or for predicting a response of a disease or disorder to therapy.

**11.** The method according to any one of the preceding claims wherein the disease or disorder is cancer or a tumor, acute or chronic tissue damage, an inflammatory disorder or an autoimmune disease.

**12.** The method according to any one of the preceding claims which is a computer-implemented method.

**13.** A computer product comprising a computer readable medium storing instructions for operating a computer system to perform the analyses and/or calculations according to a method of any one of claims 1 to 11.

**Patentansprüche**

**1.** Verfahren zur Analyse zellfreier DNA (cfDNA), wobei das Verfahren umfasst:

Beziehen von zellfreier DNA (cfDNA) aus einer biologischen Probe, die von einem Probanden bezogen wird, und Analysieren der bezogenen cfDNA, wobei die Analyse der cfDNA besteht aus:

- Analysieren des Vorliegens von DNA-Methylierung in der bezogenen cfDNA an einem krankheitsspezifischen Satz von cfDNA-CpG oder an einem krankheitsspezifischen Satz von cfDNA-CpG-Clustern mit einem mittleren durchschnittlichen Methylierungs-ß-Wert von weniger als 0,03 in cfDNA von gesunden Probanden; und
- Analysieren eines nukleosomalen Fußabdrucks der bezogenen cfDNA;

oder wobei die Analyse der cfDNA besteht aus:

- Analysieren des Vorliegens von DNA-Methylierung in der bezogenen cfDNA an einem krankheitsspezifischen Satz von cfDNA-CpG oder an einem krankheitsspezifischen Satz von cfDNA-CpG-Clustern mit einem mittleren durchschnittlichen Methylierungs-ß-Wert von weniger als 0,03 in cfDNA von gesunden Probanden;
- Analysieren eines nukleosomalen Fußabdrucks der bezogenen cfDNA; und
- Analysieren der Kopienzahlveränderung (CNA) in der bezogenen cfDNA.

**2.** Verfahren nach Anspruch 1, wobei der nukleosomale Fußabdruck ein genomweiter nukleosomaler Fußabdruck ist und/oder wobei die Kopienzahlveränderungsanalyse eine genomweite Kopienzahlveränderungsanalyse ist.

**3.** Verfahren nach einem der Ansprüche 1 bis 2, ferner umfassend ein Berechnen eines Proben-cfDNA-Methylierungsscores, der der DNA-Methylierungsscore für die cfDNA ist, an der das Vorliegen von DNA-Methylierung analysiert wurde, und ein Berechnen des Proben-cfDNA-Nukleosomscores, der der Nukleosomscore für die bezogene cfDNA ist; oder ferner umfassend ein Berechnen eines Proben-cfDNA-Methylierungsscores, der der DNA-Methylierungsscore für die cfDNA ist, an der das Vorliegen von DNA-Methylierung analysiert wurde, ein Berechnen des Proben-cfDNA-Nukleosomscores, der der Nukleosomscore für die bezogene cfDNA ist, und ein Berechnen des Proben-cfDNA-CNA-Scores, der der CNA-Score für die bezogene cfDNA ist.

**4.** Verfahren nach einem der Ansprüche 1 bis 3, ferner umfassend eine Bestimmung einer Krankheit oder Erkrankung, die wahrscheinlich bei dem Probanden vorliegt, wenn die DNA-Methylierung in der Proben-cfDNA von der DNA-Methylierung von Referenz-cfDNA abweicht oder wenn der Proben-cfDNA-Methylierungsscore von dem Referenz-cfDNA-Methylierungsscore abweicht; und wenn der Proben-cfDNA-Nukleosomscore von dem Referenz-cfDNA-Nukleosomscore abweicht; oder ferner umfassend eine Bestimmung einer Krankheit oder Erkrankung, die wahrscheinlich bei dem Probanden vorliegt, wenn die DNA-Methylierung in der Proben-cfDNA von der DNA-Methylierung von Referenz-cfDNA abweicht oder wenn der Proben-cfDNA-Methylierungsscore von dem Referenz-cfDNA-Methylierungsscore abweicht; wenn der Proben-cfDNA-Nukleosomscore von dem Referenz-cfDNA-Nukleosomscore abweicht; und wenn der Proben-cfDNA-CNA-Score von dem Referenz-cfDNA-CNA-Score abweicht.

**5.** Verfahren nach Anspruch 3, wobei der Proben-cfDNA-Methylierungsscore, der Proben-cfDNA-Nukleosomscore und der Proben-cfDNA-CNA-Score in einem einzigen Proben-cfDNA-Score kombiniert werden.

**6.** Verfahren nach Anspruch 5, ferner umfassend eine Bestimmung einer Krankheit oder Erkrankung, die wahrscheinlich bei dem Probanden vorliegt, wenn der einzige Proben-cfDNA-Score von dem einzigen Referenz-cfDNA-Score abweicht.

**7.** Verfahren nach einem der Ansprüche 1 bis 6, wobei der nukleosomale Fußabdruck oder die CNA mittels Low-Coverage-Sequenzierung, Ultralow-Pass-Sequenzierung, flacher Sequenzierung oder durch zufällige nicht-targetierte Sequenzierung analysiert wird.

**8.** Verfahren nach einem der vorhergehenden Ansprüche, wobei die biologische Probe Blut, Serum oder Plasma ist.

**9.** Verfahren nach einem der vorhergehenden Ansprüche, wobei mindestens ein Analyse- oder Berechnungsschritt durch ein Computersystem oder mittels eines Computerprogrammprodukts durchgeführt wird.

**10.** Verfahren nach einem der vorhergehenden Ansprüche, das ein Verfahren zur Diagnose einer Krankheit oder Erkrankung bei einem Probanden, zum Nachweis des Vorliegens einer Krankheit oder Erkrankung bei einem Probanden, zur Früherkennung einer Krankheit oder Erkrankung bei einem Probanden, zur Frühdiagnose einer Krankheit oder Erkrankung bei einem Probanden, zum Screening auf das Vorliegen einer Krankheit oder Erkrankung bei einem Probanden, zur Bestimmung einer erhöhten Wahrscheinlichkeit, dass eine Krankheit oder Erkrankung bei einem Probanden vorliegt, zur Überwachung einer Krankheit oder Erkrankung bei einem Probanden, zur Bestimmung eines Ansprechens einer Krankheit oder Erkrankung auf eine Therapie, zur Überwachung einer Krankheit oder Erkrankung nach einer Therapie oder zur Vorhersage eines Ansprechens einer Krankheit oder Erkrankung auf eine Therapie ist.

**11.** Verfahren nach einem der vorhergehenden Ansprüche, wobei die Krankheit oder Erkrankung Krebs oder ein Tumor, akuter oder chronischer Gewebeschaden, eine Entzündungserkrankung oder eine Autoimmunkrankheit ist.

**12.** Verfahren nach einem der vorhergehenden Ansprüche, das ein computerimplementiertes Verfahren ist.

**13.** Computerprodukt, umfassend ein computerlesbares Medium, das Anweisungen zum Betreiben eines Computersystems speichert, um die Analysen und/oder die Berechnungen gemäß einem Verfahren nach einem der Ansprüche 1 bis 11 durchzuführen.

## Revendications

**1.** Procédé d'analyse d'ADN acellulaire (ADNcf), le procédé comprenant :

obtenir de l'ADN acellulaire (ADNcf) à partir d'un échantillon biologique obtenu auprès d'un sujet et analyser l'ADNcf obtenu, dans lequel l'analyse de l'ADNcf consiste en :

- l'analyse de la présence de la méthylation de l'ADN dans l'ADNcf obtenu sur un ensemble spécifique à la maladie de CpG d'ADNcf ou sur un ensemble spécifique à la maladie de groupes de CpG d'ADNcf avec une valeur ß de méthylation moyenne de moins de 0,03 dans de l'ADNcf de sujets sains ; et
- l'analyse d'une empreinte nucléosome de l'ADNcf obtenu ;

ou dans lequel l'analyse de l'ADNcf consiste en :

- l'analyse de la présence de la méthylation de l'ADN dans l'ADNcf obtenu sur un ensemble spécifique à la maladie de CpG d'ADNcf ou sur un ensemble spécifique à la maladie de groupes de CpG d'ADNcf avec une valeur ß de méthylation moyenne de moins de 0,03 dans de l'ADNcf de sujets sains ;
- l'analyse d'une empreinte nucléosome de l'ADNcf obtenu ; et
- l'analyse de l'altération du nombre de copies (CNA) dans l'ADNcf obtenu.

**2.** Procédé selon la revendication 1, dans lequel l'empreinte nucléosome est une empreinte nucléosome à l'échelle du génome et/ou dans lequel l'analyse de l'altération du nombre de copies est une analyse de l'altération du nombre de copies à l'échelle du génome.

**3.** Procédé selon l'une quelconque des revendications 1 à 2, comprenant en outre calculer un score de méthylation d'ADNcf d'échantillon qui est le score de méthylation de l'ADN pour l'ADNcf sur lequel la présence de méthylation de l'ADN a été analysée et calculer le score de nucléosome d'ADNcf d'échantillon qui est le score de nucléosome pour l'ADNcf obtenu ; ou comprenant en outre calculer un score de méthylation d'ADNcf d'échantillon qui est le score de méthylation de l'ADN pour l'ADNcf sur lequel la présence de méthylation de l'ADN a été analysée, calculer le score de nucléosome d'ADNcf d'échantillon qui est le score de nucléosome pour l'ADNcf obtenu, et calculer le score de CNA d'ADNcf d'échantillon qui est le score de CNA pour l'ADNcf obtenu.

4. Procédé selon l'une quelconque des revendications 1 à 3, comprenant en outre la détermination d'une maladie ou d'un trouble susceptible d'être présent chez le sujet lorsque la méthylation de l'ADN dans l'ADNcf d'échantillon s'écarte de la méthylation de l'ADN d'ADNcf de référence ou lorsque le score de méthylation d'ADNcf d'échantillon s'écarte du score de méthylation d'ADNcf de référence ; et lorsque le score de nucléosome d'ADNcf d'échantillon s'écarte du score de nucléosome d'ADNcf de référence ; ou comprenant en outre la détermination d'une maladie ou d'un trouble susceptible d'être présent chez le sujet lorsque la méthylation de l'ADN dans l'ADNcf d'échantillon s'écarte de la méthylation de l'ADN d'ADNcf de référence ou lorsque le score de méthylation d'ADNcf d'échantillon s'écarte du score de méthylation d'ADNcf de référence ; lorsque le score de nucléosome d'ADNcf d'échantillon s'écarte du score de nucléosome d'ADNcf de référence ; et lorsque le score de CNA d'ADNcf d'échantillon s'écarte du score de CNA d'ADNcf de référence.

5. Procédé selon la revendication 3, dans lequel le score de méthylation d'ADNcf d'échantillon, le score de nucléosome d'ADNcf d'échantillon et le score de CNA d'ADNcf d'échantillon sont combinés en un seul score d'ADNcf d'échantillon.

6. Procédé selon la revendication 5, comprenant en outre la détermination d'une maladie ou d'un trouble susceptible d'être présent chez le sujet lorsque le score unique d'ADNcf d'échantillon s'écarte du score unique d'ADNcf de référence.

7. Procédé selon l'une quelconque des revendications 1 à 6, dans lequel l'empreinte nucléosome ou la CNA est analysée par le biais d'un séquençage faible couverture, d'un séquençage à ultra-faible passage, d'un séquençage peu profond ou par séquençage aléatoire non ciblé.

8. Procédé selon l'une quelconque des revendications précédentes, dans lequel l'échantillon biologique est du sang, du sérum ou du plasma.

9. Procédé selon l'une quelconque des revendications précédentes, dans lequel au moins une analyse ou étape de calcul est réalisée par un système informatique ou par le biais d'un produit de programme informatique.

10. Procédé selon l'une quelconque des revendications précédentes, qui est un procédé de diagnostic d'une maladie ou d'un trouble chez un sujet, de détection de la présence d'une maladie ou d'un trouble chez un sujet, de détection précoce d'une maladie ou d'un trouble chez un sujet, de diagnostic précoce d'une maladie ou d'un trouble chez un sujet, de dépistage de la présence d'une maladie ou d'un trouble chez un sujet, de détermination d'une probabilité accrue de la présence d'une maladie ou d'un trouble chez un sujet, de surveillance d'une maladie ou d'un trouble chez un sujet, de détermination d'une réponse d'une maladie ou d'un trouble à une thérapie, de surveillance d'une maladie ou d'un trouble après une thérapie, ou de prédiction d'une réponse d'une maladie ou d'un trouble à une thérapie.

11. Procédé selon l'une quelconque des revendications précédentes, dans lequel la maladie ou le trouble est un cancer ou une tumeur, une lésion de tissu aigüe ou chronique, un trouble inflammatoire ou une maladie auto-immune.

12. Procédé selon l'une quelconque des revendications précédentes, qui est un procédé mis en œuvre par ordinateur.

13. Produit informatique comprenant un support lisible par ordinateur stockant des instructions pour opérer un système informatique à réaliser les analyses et/ou calculs conformément à un procédé selon l'une quelconque des revendications 1 à 11.

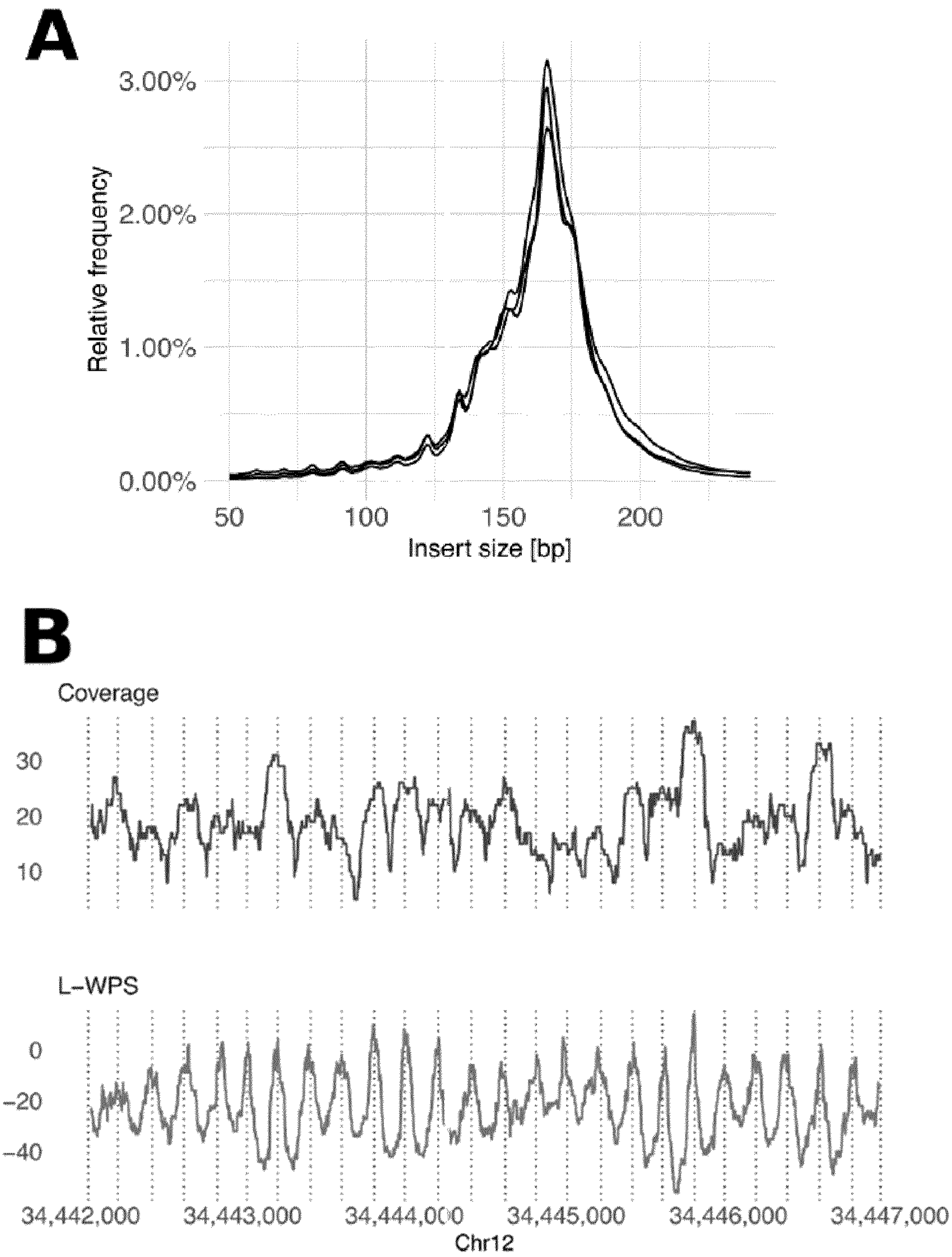
A
3.00%
2.00%
1.00%
0.00%
Relative frequency
50
100
150
200
Insert size [bp]
B
Coverage
30
20
10
L-WPS
0
-20
-40
34,442,000
34,443,000
34,444,000
34,445,000
34,446,000
34,447,000
Chr12

FIGURE 1

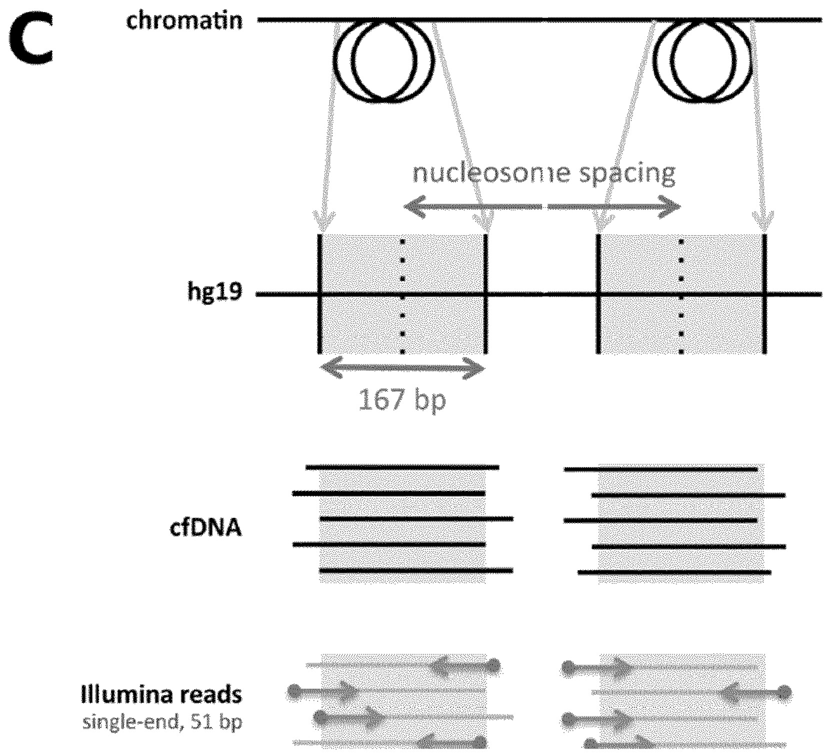


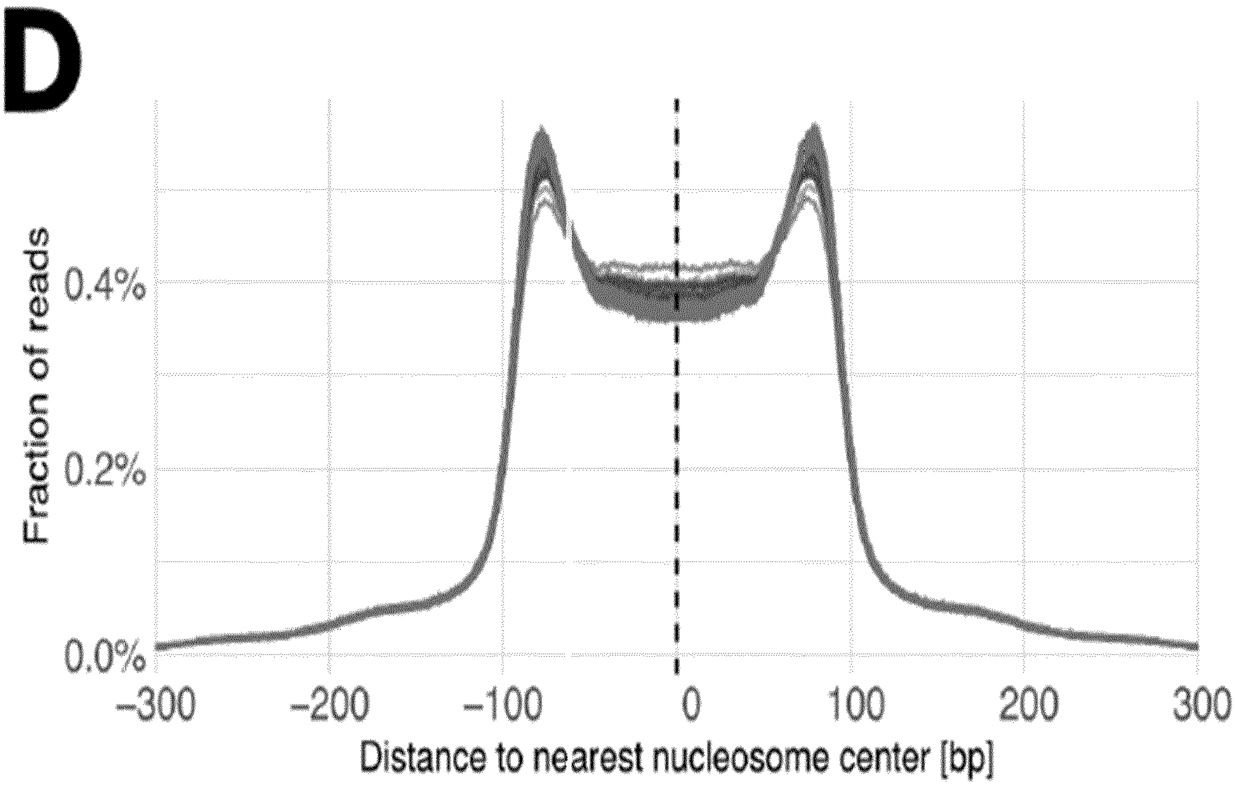


FIGURE 1 - continued

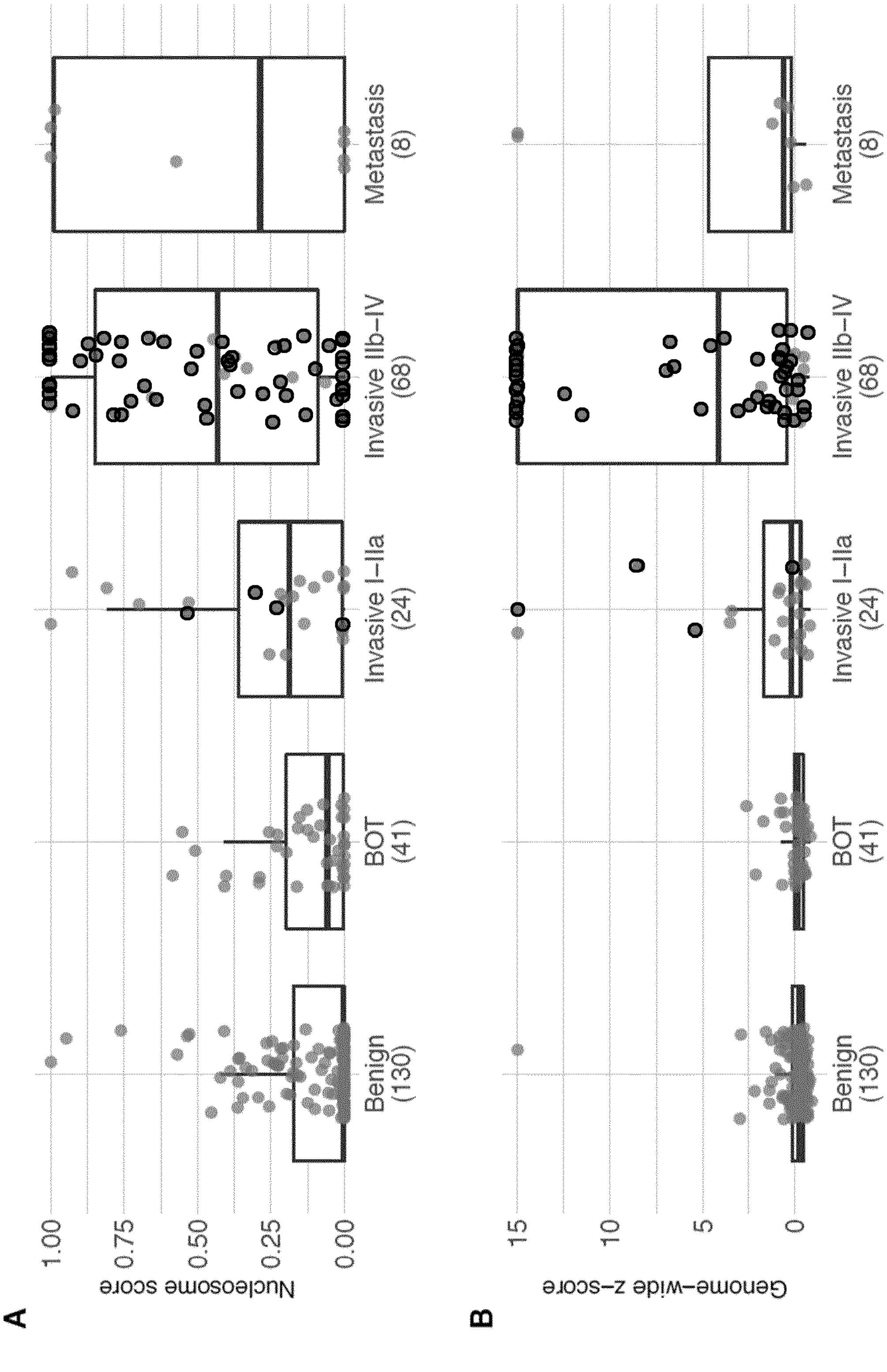
A
Nucleosome score
1.00
0.75
0.50
0.25
0.00
Benign
(130)
BOT
(41)
Invasive I–IIa
(24)
Invasive IIb–IV
(68)
Metastasis
(8)
B
Genome–wide z–score
15
10
5
0
Benign
(130)
BOT
(41)
Invasive I–IIa
(24)
Invasive IIb–IV
(68)
Metastasis
(8)

FIGURE 2

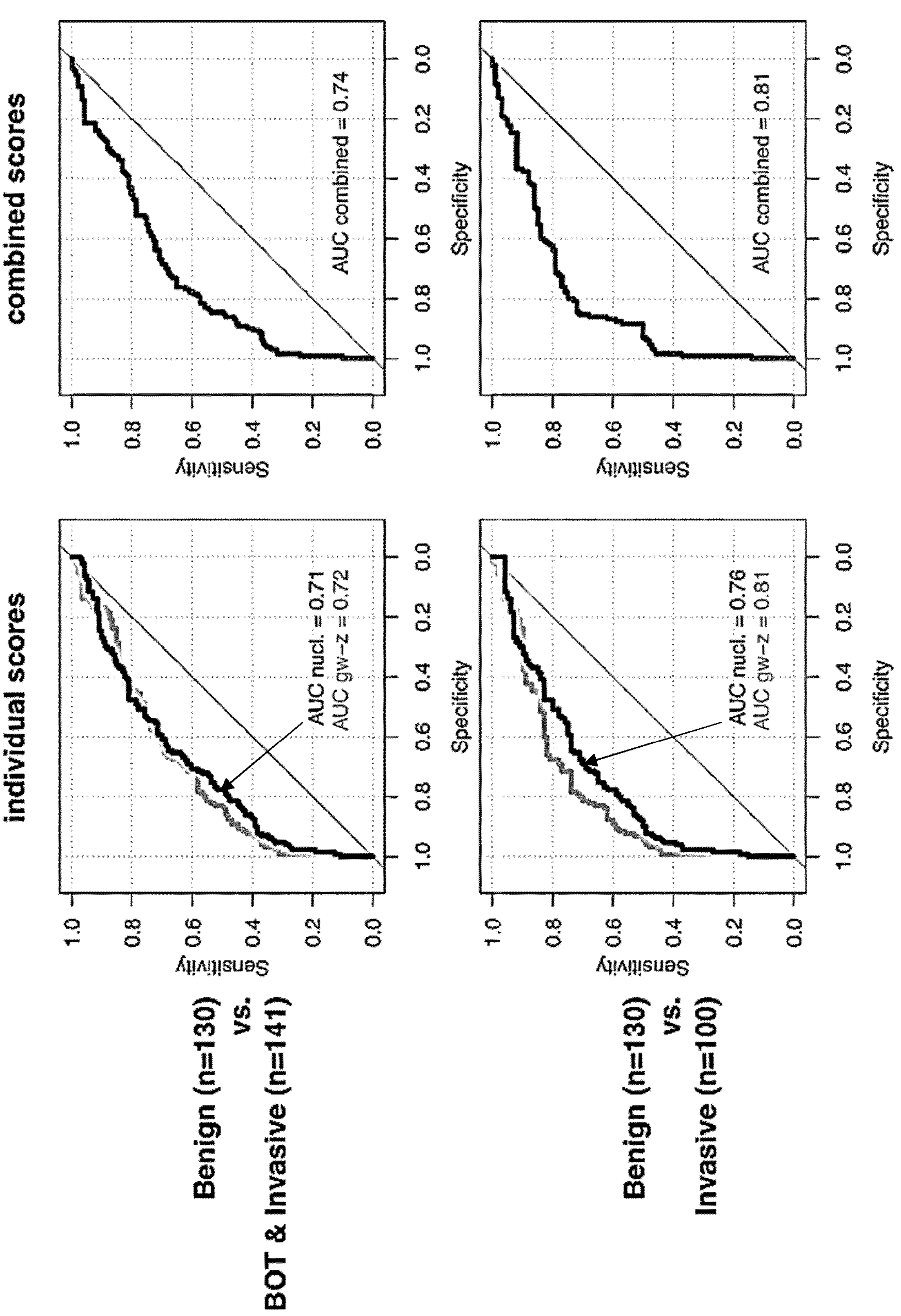
individual scores
combined scores
Benign (n=130) vs. BOT & Invasive (n=141)
Benign (n=130) vs. Invasive (n=100)
AUC nucl. = 0.71
AUC gw−z = 0.72
AUC combined = 0.74
AUC nucl. = 0.76
AUC gw−z = 0.81
AUC combined = 0.81
Sensitivity
Specificity

FIGURE 3

**Benign (n=130)**
**vs.**
**HGSOC (n=62)**

**Benign (n=130)**
**vs.**
**non-HGSOC (n=30)**

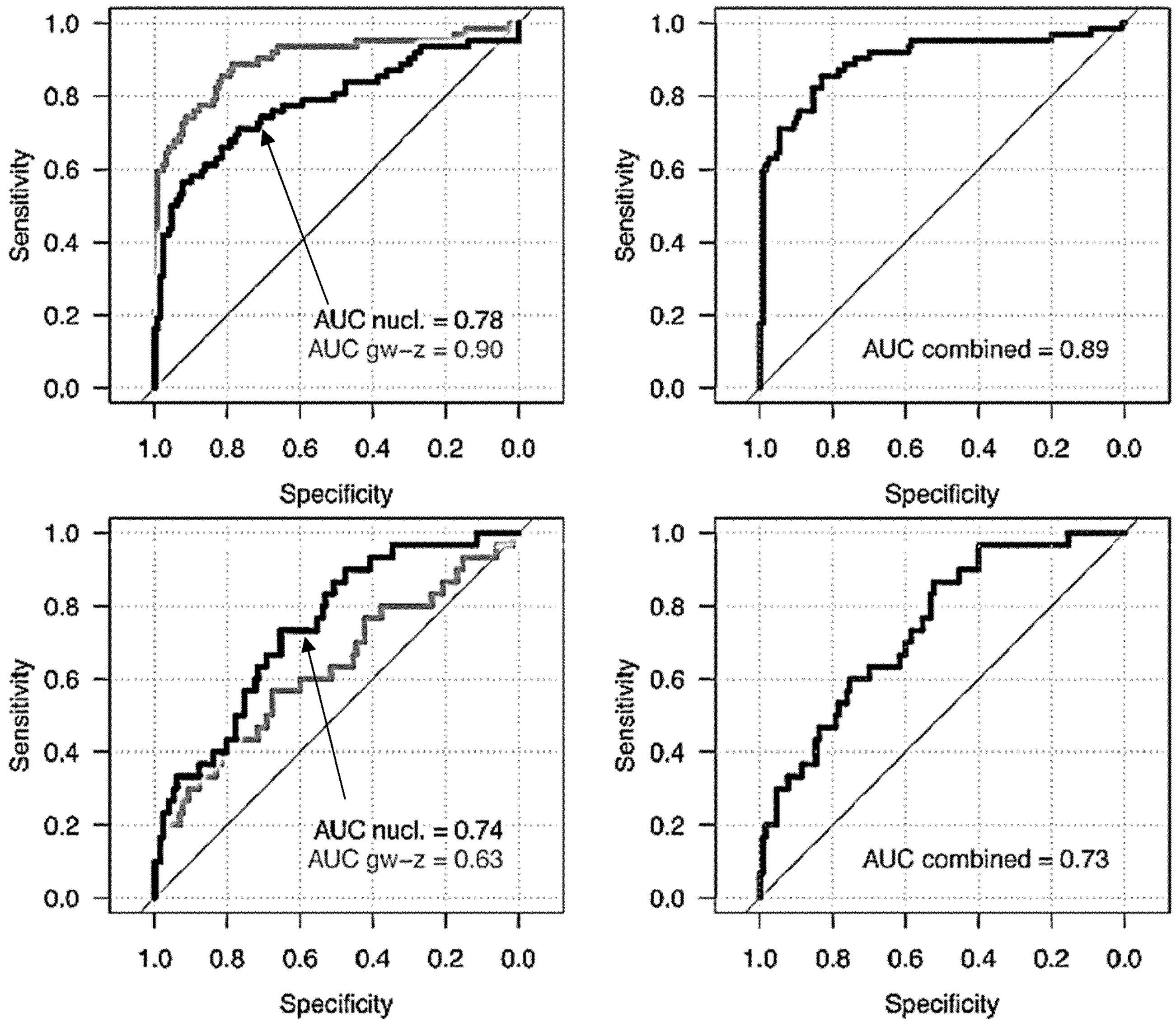


**FIGURE 3 - continued**

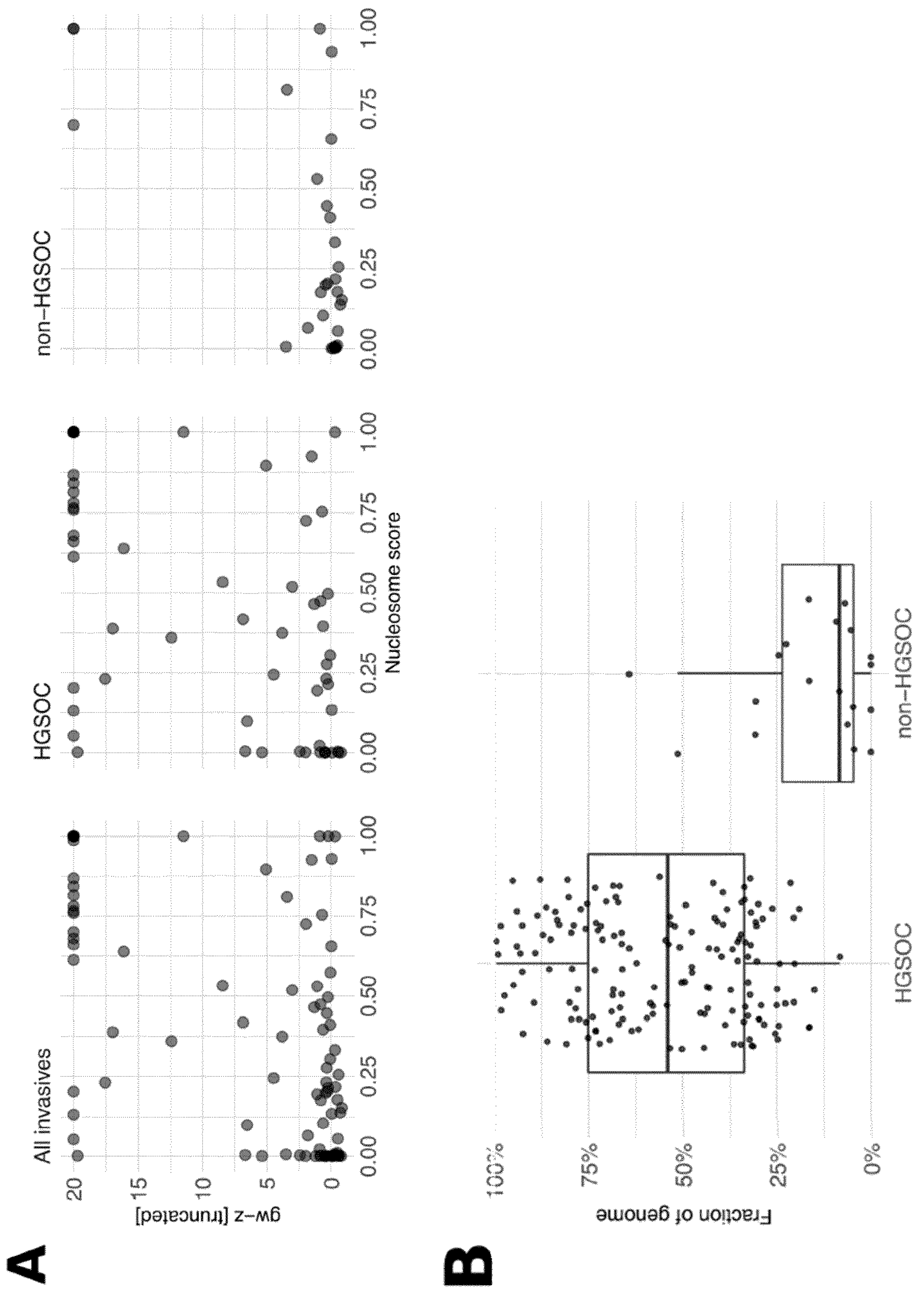
A
All invasives
HGSOC
non−HGSOC
gw−z [truncated]
20
15
10
5
0
0.00
0.25
0.50
0.75
1.00
Nucleosome score
B
Fraction of genome
100%
75%
50%
25%
0%
HGSOC
non−HGSOC

FIGURE 4

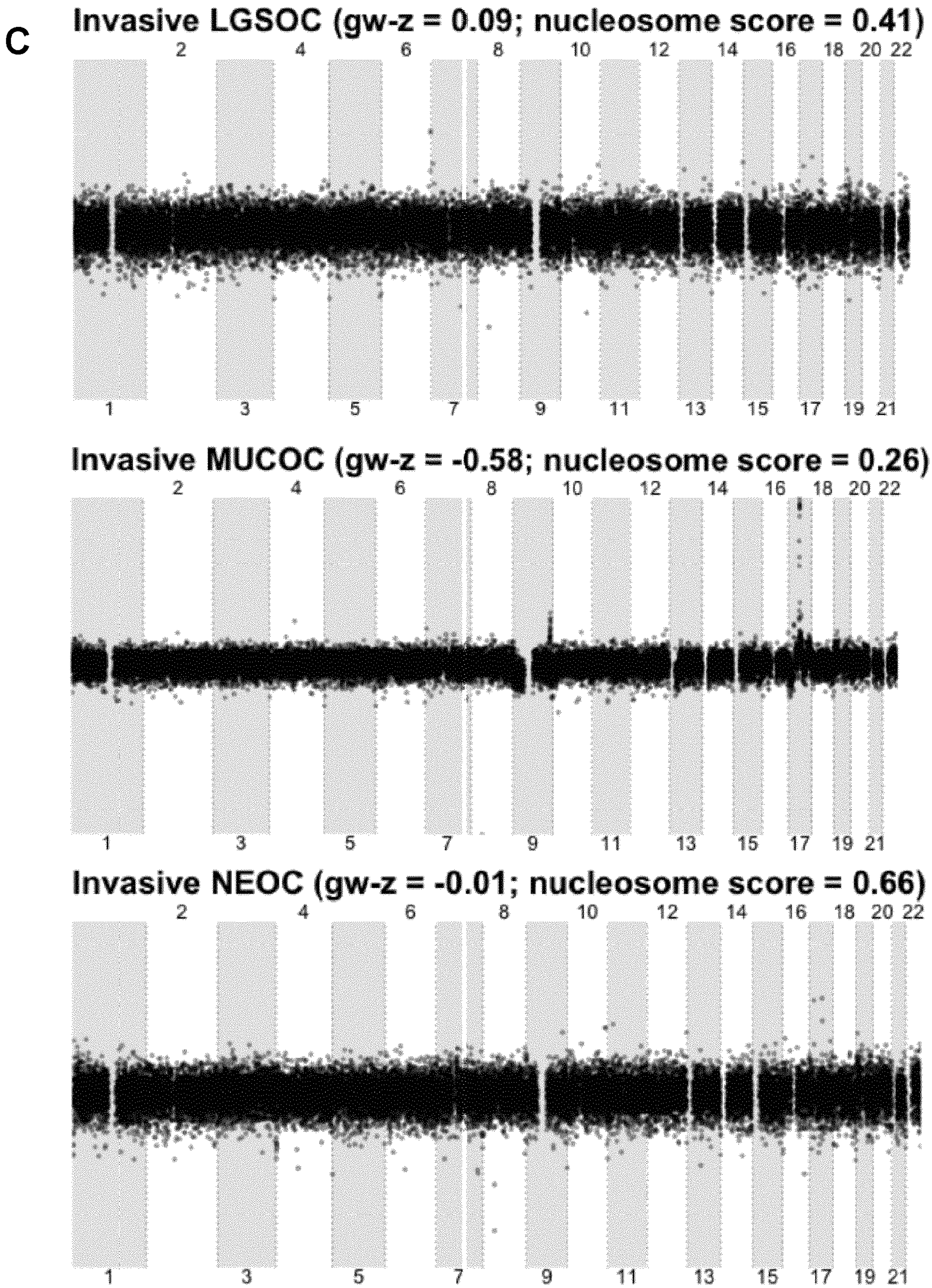


FIGURE 4 – continued

D

Invasive HGSOC (gw-z = 8.45; nucleosome score = 0.53)

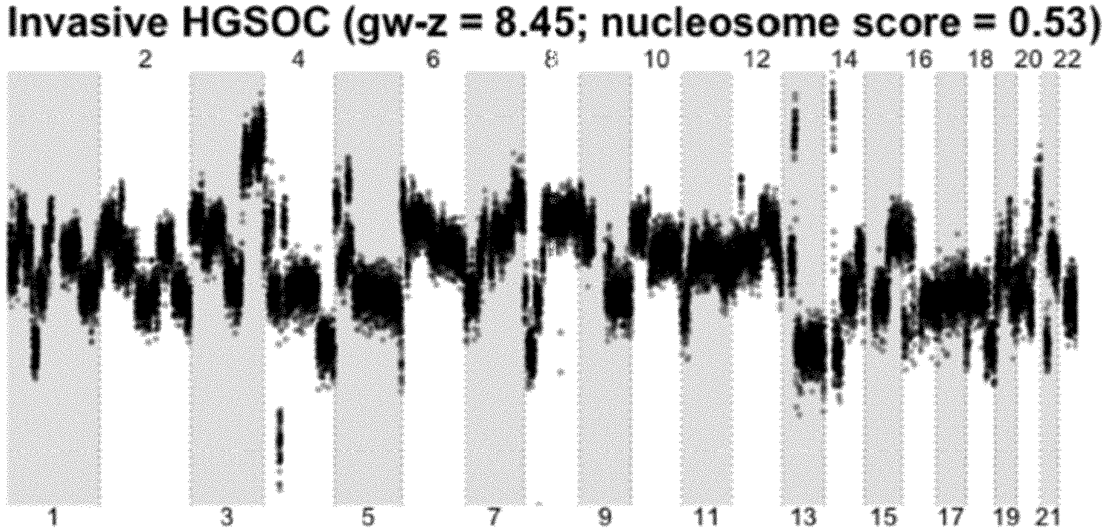


Invasive HGSOC (gw-z = 5.07; nucleosome score = 0.89)

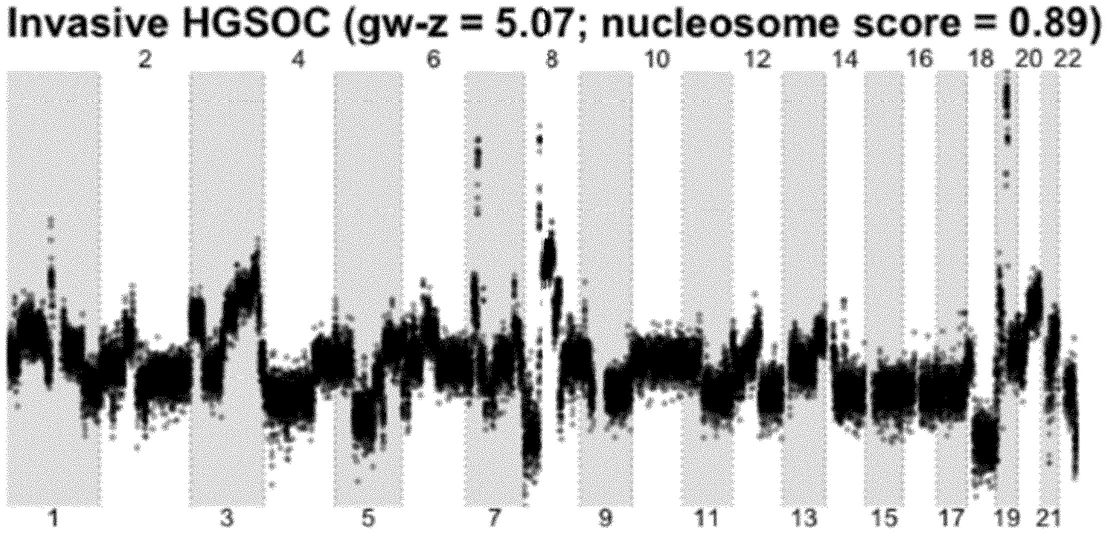


Invasive HGSOC (gw-z = 1.96; nucleosome score = 0.72)

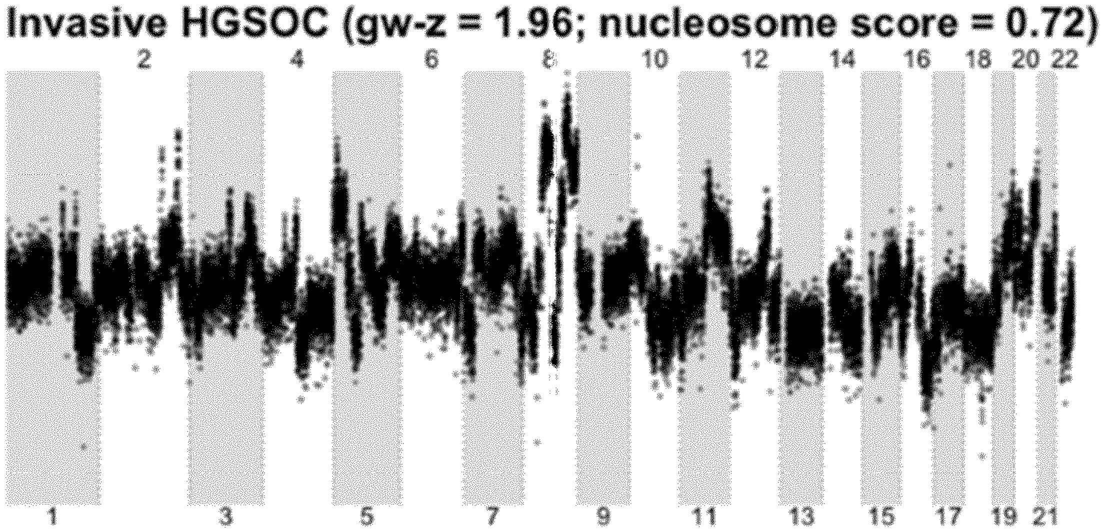


Figure 4 continued

| | All stages | Stage I-II | Stage IIIA/B | Stage IIIC | Stage IV |
|---|---|---|---|---|---|
| Benign mass | (n=130)<br>**median: -0.3**<br>IQR: -0.5-0.1<br>range: -0.9-93.1 | -<br>-<br>-<br>- | -<br>-<br>-<br>- | -<br>-<br>-<br>- | -<br>-<br>-<br>- |
| Carcinoma | (n=141)<br>**median: 0.5**<br>IQR: -0.2-6.7<br>range: -0.9-3513.6 | (n=64)<br>**median: -0.1**<br>IQR: -0.4-0.6<br>range: -0.9-69.5 | (n=15)<br>**median: 0.5**<br>IQR: -0.0-77.7<br>range: -0.6-3513.6 | (n=22)<br>**median: 1.6**<br>IQR: 0.3-18.9<br>range: -0.8-811.0 | (n=32)<br>**median: 16.5**<br>IQR: 1.9-68.4<br>range: -0.5-681.7 |
| Borderline carcinoma | (n=41)<br>**median: -0.2**<br>IQR: -0.5-0.0<br>range: -0.9-2.6 | (n=38)<br>**median: -0.2**<br>IQR: -0.5-0.0<br>range: -0.9-2.6 | (n=3)<br>**median: -0.2**<br>IQR: -0.4--0.1<br>range: -0.6--0.1 | -<br>-<br>-<br>- | -<br>-<br>-<br>- |
| Invasive carcinoma | (n=92)<br>**median: 1.7**<br>IQR: 0.1-23.1<br>range: -0.8-3513.6 | (n=26)<br>**median: 0.2**<br>IQR: -0.3-1.0<br>range: -0.8-69.5 | (n=12)<br>**median: 0.9**<br>IQR: 0.3-394.4<br>range: -0.6-3513.6 | (n=22)<br>**median: 1.6**<br>IQR: 0.3-18.9<br>range: -0.8-811.0 | (n=32)<br>**median: 16.5**<br>IQR: 1.9-68.4<br>range: -0.5-681.7 |
| Invasive carcinoma HGSOC | (n=62)<br>**median: 6.6**<br>IQR: 0.7-38.3<br>range: -0.8-3513.6 | (n=5)<br>**median: 5.4**<br>IQR: 0.5-8.4<br>range: 0.1-17.5 | (n=11)<br>**median: 1.1**<br>IQR: 0.4-636.3<br>range: -0.6-3513.6 | (n=17)<br>**median: 2.0**<br>IQR: 0.5-21.4<br>range: -0.8-787.3 | (n=29)<br>**median: 17.0**<br>IQR: 3.8-60.1<br>range: -0.5-681.7 |
| Invasive carcinoma non-HGSOC | (n=30)<br>**median: -0.0**<br>IQR: -0.3-0.9<br>range: -0.8-811.0 | (n=21)<br>**median: -0.0**<br>IQR: -0.3-0.8<br>range: -0.8-69.5 | (n=1)<br>**median: 0.0**<br>-<br>- | (n=5)<br>**median: 0.1**<br>IQR: -0.5-1.8<br>range: -0.5-811.0 | (n=3)<br>**median: 0.3**<br>IQR: 0.1-76.2<br>range: -0.1-152.1 |
| non-HGSOC: Clear cell | (n=3)<br>**median: 1.8**<br>IQR: 0.9-406.4<br>range: -0.0-811.0 | (n=1)<br>**median: 0.0**<br>-<br>- | -<br>-<br>-<br>- | (n=2)<br>**median: 406.4**<br>IQR: 204.1-608.7<br>range: 1.8-811.0 | -<br>-<br>-<br>- |
| non-HGSOC: Endometrioid | (n=9)<br>**median: 0.4**<br>IQR: -0.3-0.9<br>range: -0.4-69.5 | (n=9)<br>**median: 0.4**<br>IQR: -0.3-0.9<br>range: -0.4-69.5 | -<br>-<br>-<br>- | -<br>-<br>-<br>- | -<br>-<br>-<br>- |
| non-HGSOC: LGSOC | (n=6)<br>**median: -0.2**<br>IQR: -0.4-0.0<br>range: -0.5-0.3 | (n=1)<br>**median: -0.3**<br>-<br>- | -<br>-<br>-<br>- | (n=3)<br>**median: -0.5**<br>IQR: -0.5--0.2<br>range: -0.5-0.1 | (n=2)<br>**median: 0.1**<br>IQR: -0.0-0.2<br>range: -0.1-0.3 |
| non-HGSOC: Mucinous | (n=8)<br>**median: -0.0**<br>IQR: -0.5-0.9<br>range: -0.8-152.1 | (n=7)<br>**median: -0.3**<br>IQR: -0.6-0.5<br>range: -0.8-1.1 | -<br>-<br>-<br>- | -<br>-<br>-<br>- | (n=1)<br>**median: 152.1**<br>-<br>- |
| non-HGSOC: Non-epithelial | (n=4)<br>**median: -0.1**<br>IQR: -0.4-0.9<br>range: -0.7-3.5 | (n=3)<br>**median: -0.3**<br>IQR: -0.5-1.6<br>range: -0.7-3.5 | (n=1)<br>**median: 0.0**<br>-<br>- | -<br>-<br>-<br>- | -<br>-<br>-<br>- |
| Metastatic | (n=8)<br>**median: 0.6**<br>IQR: 0.2-44.5<br>range: -0.6-292.9 | -<br>-<br>-<br>- | -<br>-<br>-<br>- | -<br>-<br>-<br>- | -<br>-<br>-<br>- |

# FIGURE 5

| | All patients | Stage I-II | Stage IIIA/B | Stage IIIC | Stage IV |
|---|---|---|---|---|---|
| Benign mass | (n=130) | - | - | - | - |
| | median: 0.00 | - | - | - | - |
| | IQR: 0.00-0.17 | - | - | - | - |
| | range: 0.00-1.00 | - | - | - | - |
| Carcinoma | (n=141) | (n=64) | (n=15) | (n=22) | (n=32) |
| | median: 0.20 | median: 0.09 | median: 0.40 | median: 0.16 | median: 0.65 |
| | IQR: 0.01-0.61 | IQR: 0.00-0.24 | IQR: 0.20-0.83 | IQR: 0.02-0.69 | IQR: 0.33-0.87 |
| | range: 0.00-1.00 | range: 0.00-1.00 | range: 0.00-1.00 | range: 0.00-1.00 | range: 0.00-1.00 |
| Borderline carcinoma | (n=41) | (n=38) | (n=3) | - | - |
| | median: 0.06 | median: 0.06 | median: 0.23 | - | - |
| | IQR: 0.00-0.20 | IQR: 0.00-0.16 | IQR: 0.11-0.41 | - | - |
| | range: 0.00-0.58 | range: 0.00-0.55 | range: 0.00-0.58 | - | - |
| Invasive carcinoma | (n=92) | (n=26) | (n=12) | (n=22) | (n=32) |
| | median: 0.35 | median: 0.19 | median: 0.46 | median: 0.16 | median: 0.65 |
| | IQR: 0.05-0.77 | IQR: 0.01-0.33 | IQR: 0.21-1.00 | IQR: 0.02-0.69 | IQR: 0.33-0.87 |
| | range: 0.00-1.00 | range: 0.00-1.00 | range: 0.00-1.00 | range: 0.00-1.00 | range: 0.00-1.00 |
| Invasive carcinoma HGSOC | (n=62) | (n=5) | (n=11) | (n=17) | (n=29) |
| | median: 0.44 | median: 0.23 | median: 0.40 | median: 0.13 | median: 0.66 |
| | IQR: 0.11-0.84 | IQR: 0.00-0.30 | IQR: 0.20-1.00 | IQR: 0.00-0.75 | IQR: 0.36-0.87 |
| | range: 0.00-1.00 | range: 0.00-0.53 | range: 0.00-1.00 | range: 0.00-1.00 | range: 0.00-1.00 |
| Invasive carcinoma non-HGSOC | (n=30) | (n=21) | (n=1) | (n=5) | (n=3) |
| | median: 0.19 | median: 0.18 | median: 0.66 | median: 0.18 | median: 0.45 |
| | IQR: 0.02-0.51 | IQR: 0.01-0.33 | - | IQR: 0.07-0.41 | IQR: 0.22-0.72 |
| | range: 0.00-1.00 | range: 0.00-1.00 | - | range: 0.01-1.00 | range: 0.00-1.00 |
| non-HGSOC: Clear cell | (n=3) | (n=1) | - | (n=2) | - |
| | median: 0.07 | median: 0.00 | - | median: 0.53 | - |
| | IQR: 0.03-0.53 | - | - | IQR: 0.30-0.77 | - |
| | range: 0.00-1.00 | - | - | range: 0.07-1.00 | - |
| non-HGSOC: Endometrioid | (n=9) | (n=9) | - | - | - |
| | median: 0.33 | median: 0.33 | - | - | - |
| | IQR: 0.20-0.81 | IQR: 0.20-0.81 | - | - | - |
| | range: 0.00-1.00 | range: 0.00-1.00 | - | - | - |
| non-HGSOC: LGSOC | (n=6) | (n=1) | - | (n=3) | (n=2) |
| | median: 0.09 | median: 0.01 | - | median: 0.18 | median: 0.22 |
| | IQR: 0.01-0.35 | - | - | IQR: 0.09-0.29 | IQR: 0.11-0.33 |
| | range: 0.00-0.45 | - | - | range: 0.01-0.41 | range: 0.00-0.45 |
| non-HGSOC: Mucinous | (n=8) | (n=7) | - | - | (n=1) |
| | median: 0.19 | median: 0.18 | - | - | median: 1.00 |
| | IQR: 0.13-0.32 | IQR: 0.10-0.23 | - | - | - |
| | range: 0.00-1.00 | range: 0.00-0.53 | - | - | - |
| non-HGSOC: Non-epithelial | (n=4) | (n=3) | (n=1) | - | - |
| | median: 0.07 | median: 0.01 | median: 0.66 | - | - |
| | IQR: 0.01-0.27 | IQR: 0.01-0.07 | - | - | - |
| | range: 0.00-0.66 | range: 0.00-0.14 | - | - | - |
| Metastatic | (n=8) | - | - | - | - |
| | median: 0.29 | - | - | - | - |
| | IQR: 0.00-0.99 | - | - | - | - |
| | range: 0.00-1.00 | - | - | - | - |

**FIGURE 6**

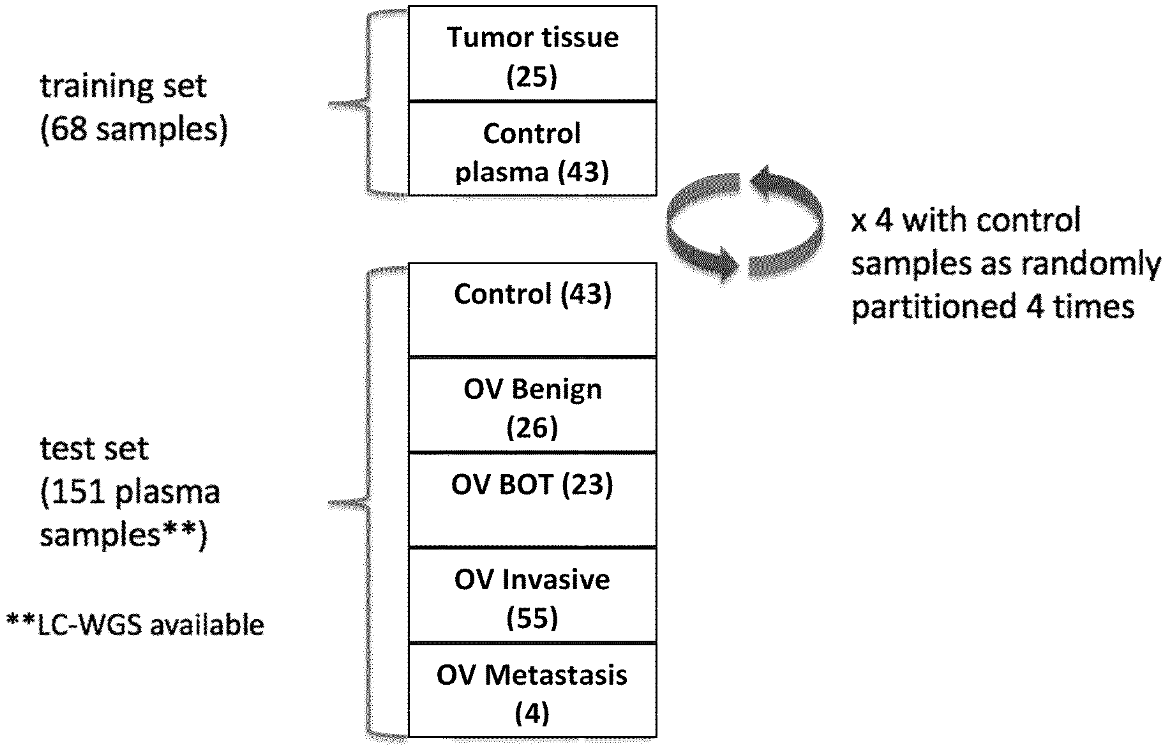


FIGURE 7

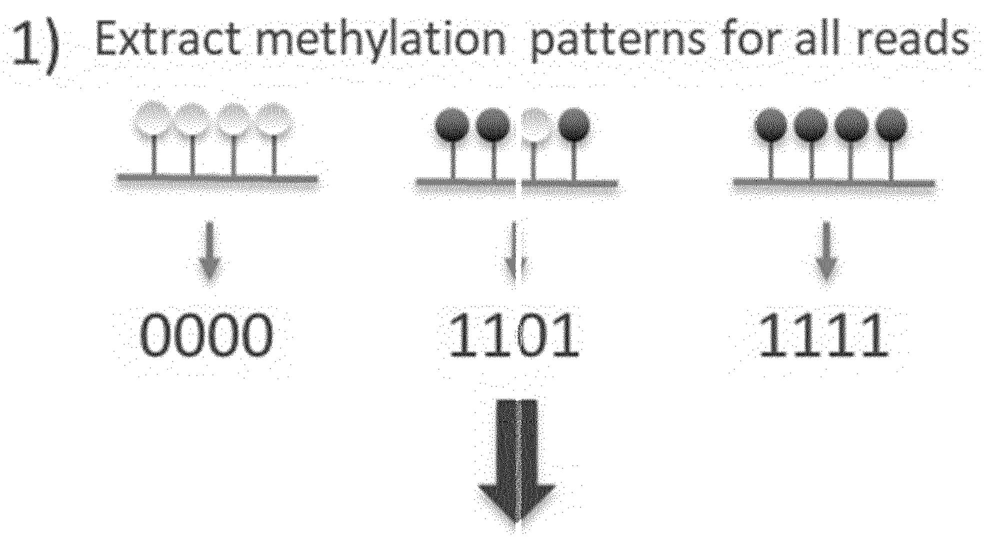


FIGURE 8

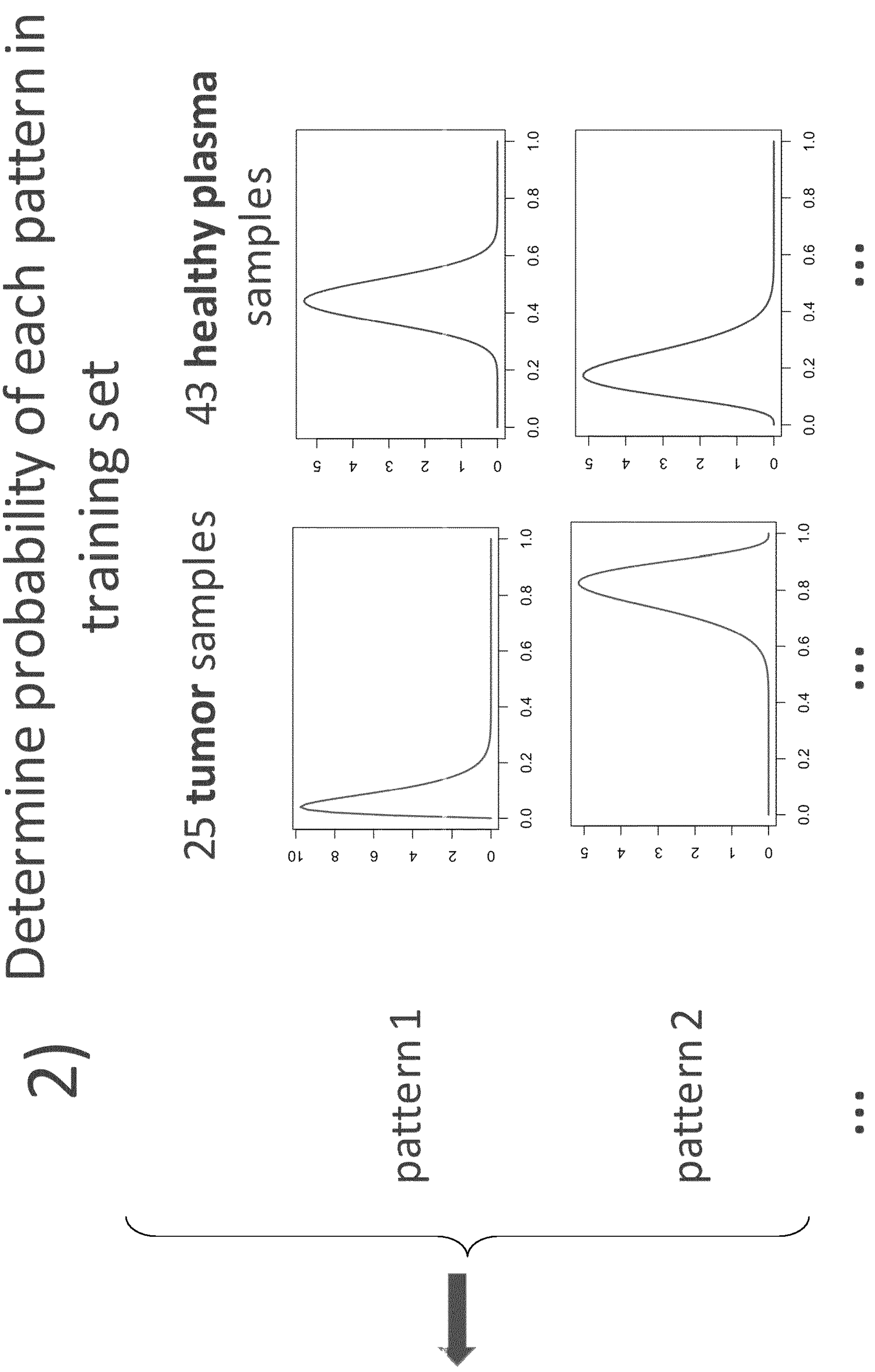


Figure 8 continued

3) Apply probabilities to determine maximum likelihood in test set via Monte Carlo simulation

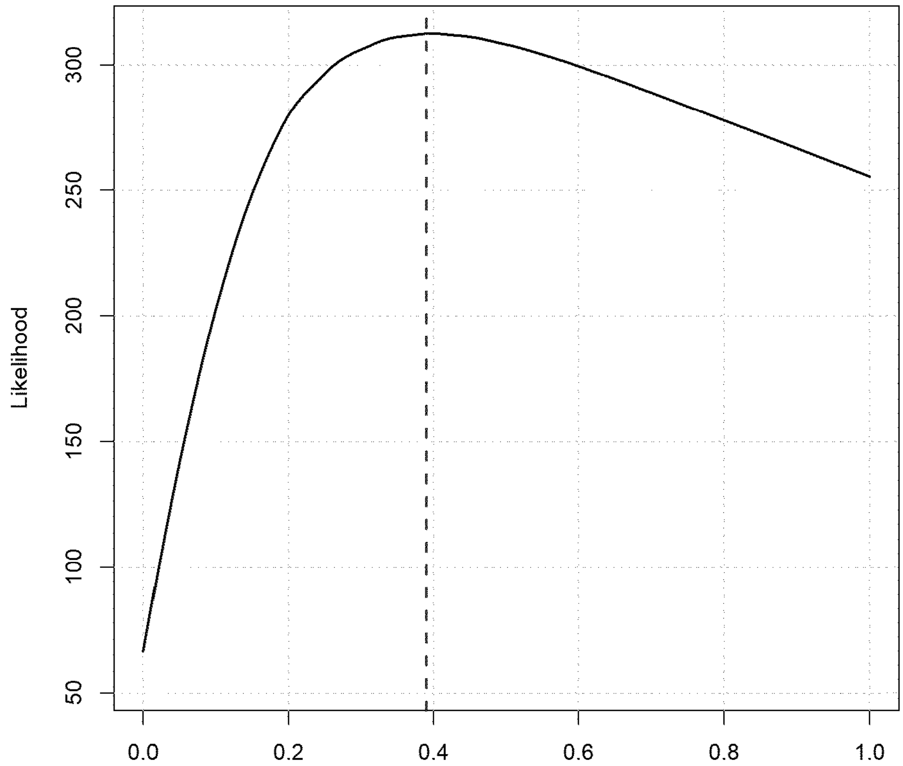


Estimated cfDNA tumor load based on methylation patterns = **methylation score**

**Figure 8 continued**

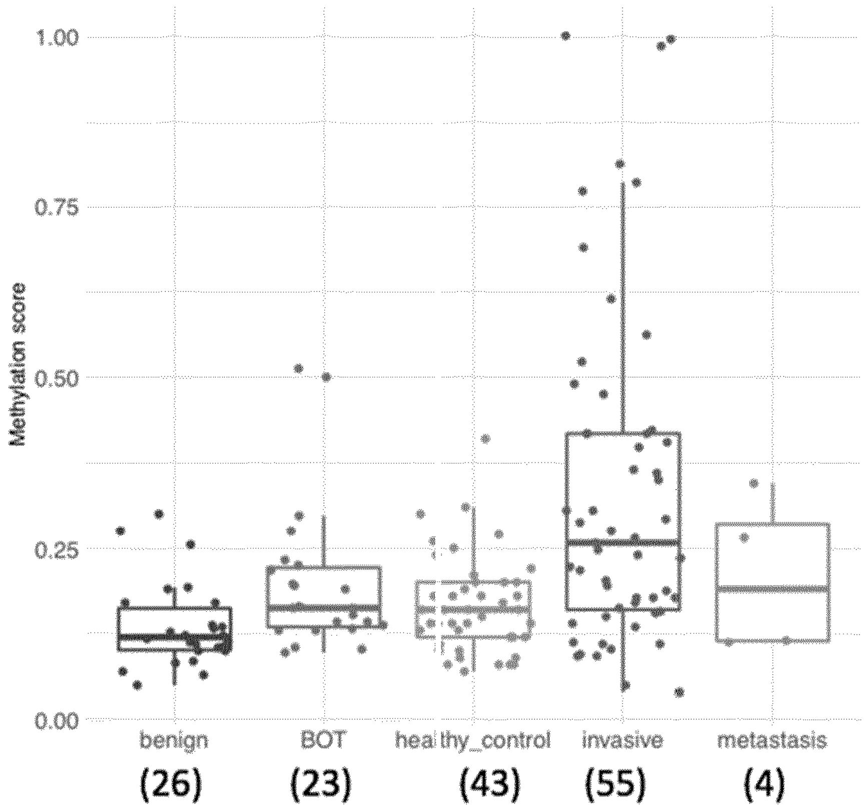


FIGURE 9

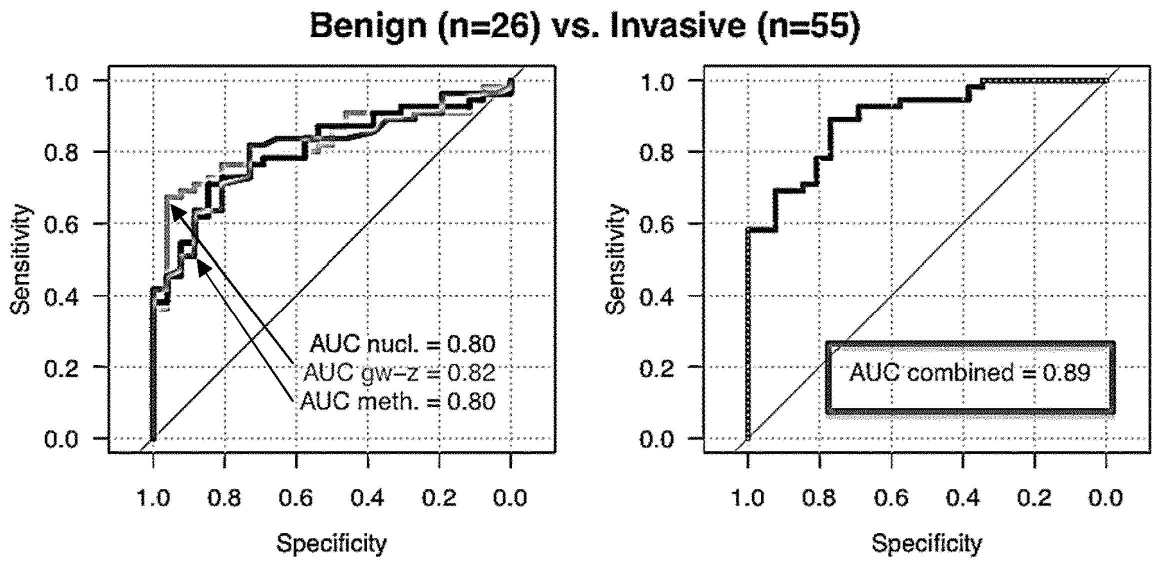


FIGURE 10

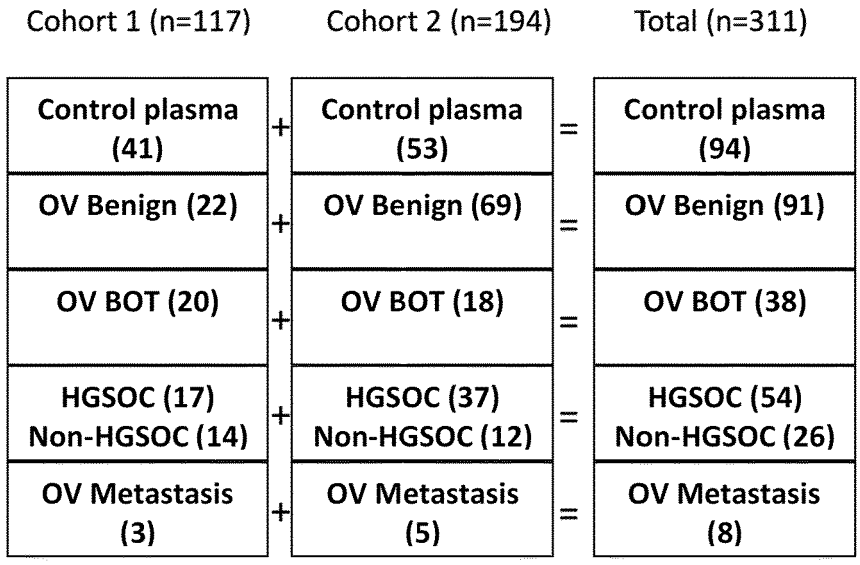


FIGURE 11

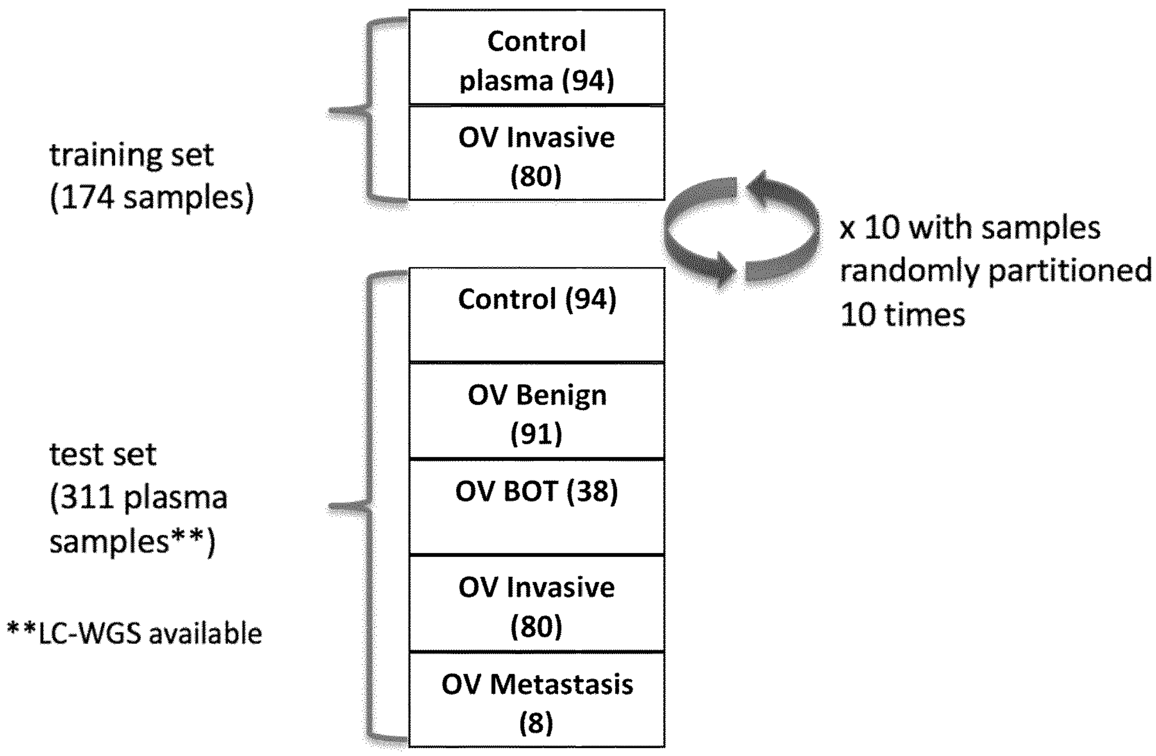


FIGURE 12

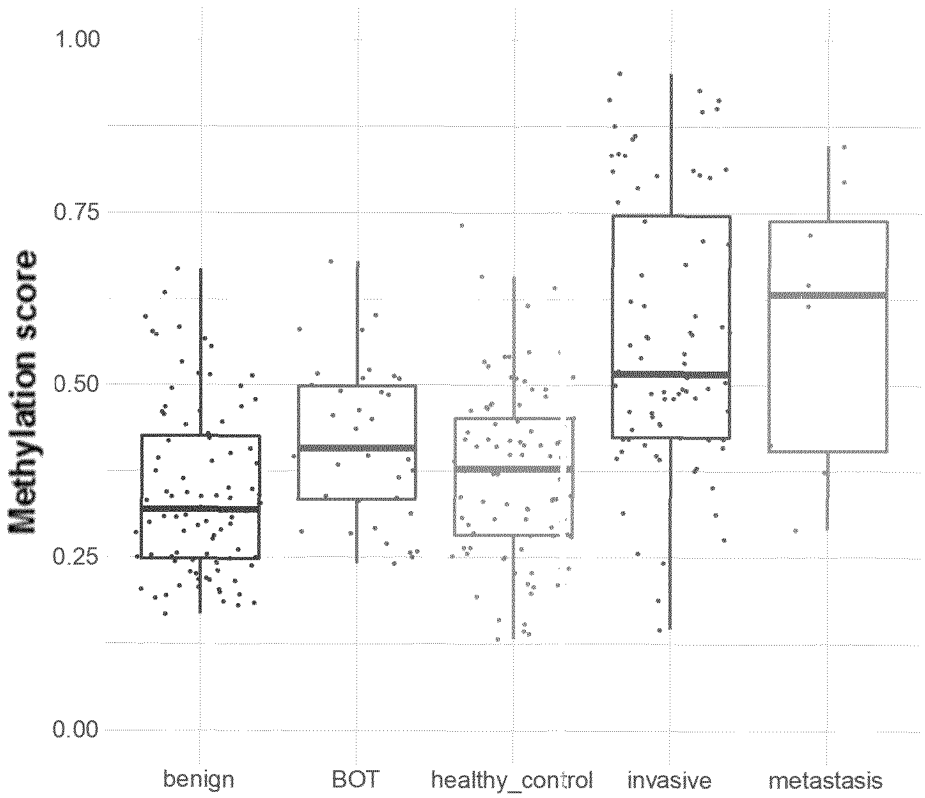


FIGURE 13

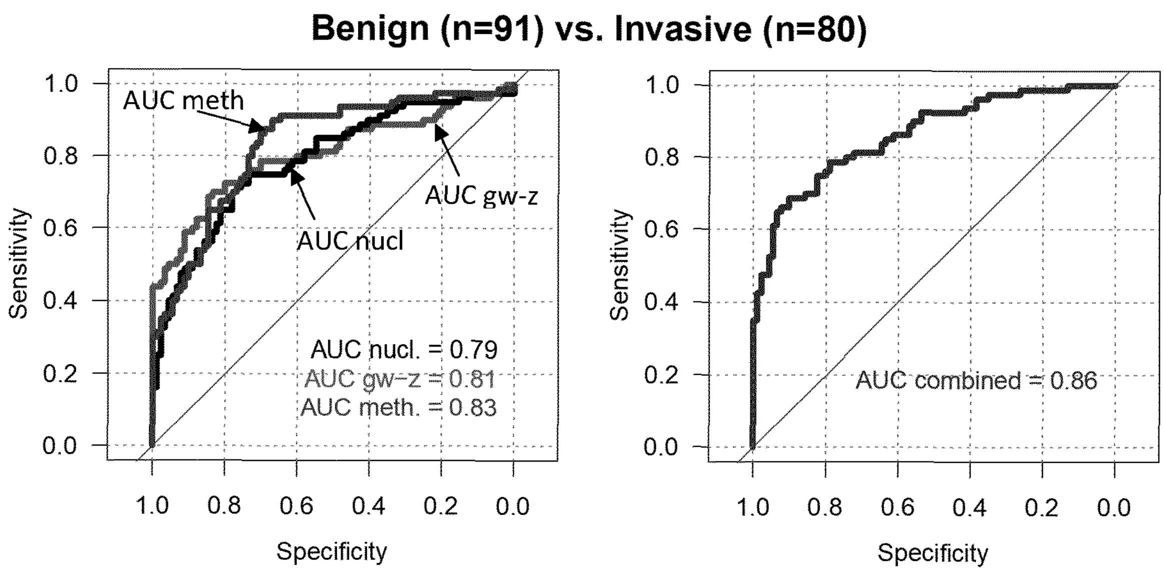


FIGURE 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017009372 A
- WO 2018204657 A
- WO 2019006269 A
- WO 2018109212 A
- WO 2018109217 A
- WO 2017048932 A
- WO 2019068082 A
- WO 2019071161 A
- WO 2016015058 A
- WO 2017012592 A
- WO 2017181146 A
- US 2018149636 A
- US 5786146 A
- WO 2004113567 A
- WO 0026401 A
- WO 2012067830 A
- WO 2017162754 A
- US 20100240549 A1


### Non-patent literature cited in the description


- *World Cancer Day 2019: Emphasis on Early Detection*, https://ascopost.com/News/59711
- **NEBGEN et al.** *Curr Oncol Rep*, 2019, vol. 21, 75
- **TUAEVA et al.** *Cells*, 2019, vol. 8, 1251
- **SUN et al.** *PNAS*, 2015, vol. 112, E5503-E5512
- **VANDERSTICHELE et al.** *Clin Cancer Res*, 2016, vol. 23, 2223-2231
- **GUO et al.** *Nat Genet*, 2017, vol. 49, 635-642
- **LI et al.** *Nucleic Acids Res*, 2018, vol. 46, e89
- **KANG et al.** *Genome Biol*, 2017, vol. 18, 53
- **WIDSCHWENDTER et al.** *Genome Medicine*, 2017, vol. 9, 116
- **LO et al.** *Sci Transl Med*, 2010, vol. 2, 61-91
- **JIANG et al.** *Trends Genet*, 2016, vol. 32, 360-371
- **SNYDER et al.** *Cell*, 2016, vol. 164, 57-68
- **CRISTIANO et al.** *Nature*, 2019, vol. 570, 385-389
- **ULZ et al.** *Nat Genet*, 2016, vol. 48, 1273-1278
- **STRAVER et al.** *Prenat Diagn*, 2016, vol. 36, 614-621
- **JIANG** ; **LO**. *Trends Genet*, 2016, vol. 32, 360-371
- **GARDINER-GARDEN et al.** *J Mol Biol*, 1987, vol. 196, 261-282
- **SAXONOV et al.** *PNAS*, 2006, vol. 103, 1412-1417
- **BACHMAN et al.** *Nat Chem*, 2014, vol. 6, 1049-1055
- **WILLIAMS et al.** *Nature*, 2011, vol. 473, 343-348
- **THIENPONT et al.** *Nature*, 2016, vol. 537, 63-68
- **CIRIELLO et al.** *Nat Genet*, 2013, vol. 45, 1127-1133
- **BOWTELL et al.** *Nat Rev Cancer*, 2015, vol. 15, 668-679
- **LEPAGE et al.** *Cancers*, 2019, vol. 11, 226
- **LEARY et al.** *Sci Transl Med*, 2012, vol. 4, 162-154
- **VANDERSTICHELE et al.** *Clin Cancer Res*, 2017, vol. 23, 2223-2231
- **XIA et al.** *Lung Cancer*, 2015, vol. 90, 78-84
- **SCHUTZ et al.** *Clin Chem*, 2015, vol. 61, 239-248
- **LAIRD**. *Nat Rev Genet*, 2010, vol. 11, 191-203
- **EADS et al.** *Nucleic Acids Res*, 2000, vol. 28, e32
- **HERMAN et al.** *Proc Natl Acad Sci USA*, 1996, vol. 93, 9821-9826
- **GONZALGO** ; **JONES**. *Nucleic Acids Res*, 1997, vol. 25, 2529-2531
- **GONZALGO et al.** *Cancer Research*, 1997, vol. 57, 594-599
- **XIONG** ; **LAIRD**. *Nucleic Acids Res*, 1997, vol. 25, 2532-2534
- **FROMMER et al.** *Proc Natl Acad Sci USA*, 1992, vol. 89, 1827-1831
- **MOORE**. *Methods Mol Biol*, 2001, vol. 181, 193-201
- **COTTRELL et al.** *Nucleic Acids Res*, 2004, vol. 32, e10
- **TOYOTA et al.** *Cancer Res*, 1999, vol. 59, 2307-12
- **MEISSNER et al.** *Nucleic Acids Res*, 2005, vol. 33, 5868-5877

- **LAIRD et al.** *Nat Rev Genet*, 2010, vol. 11, 191-203
- **KURDYUKOV** ; **BULLOCK**. *Biology*, 2016, vol. 5 (1), E3
- **DE KOKER et al.** *bioRxiv:663195*, 2019, http://dx.doi.org/10.1101/663195
- **VAN PAEMEL et al.** *bioRxiv:795047*, 2019, https://doi.org/10.1101/795047
- **SAMBROOK et al.** Molecular Cloning: A Laboratory Manual. Cold Spring Harbor Press, 2012
- **AUSUBEL et al.** current Protocols in Molecular Biology. John Wiley & Sons, 2012
- **CONCIN et al.** *J Clin Oncol*, 2018, vol. 36 (15), 5567-5567
- **LI** ; **DURBIN**. *Bioinformatics*, 2009, vol. 25, 1754-1760
- **LI et al.** *Bioinformatics*, 2009, vol. 25, 2078-2079
- **HEITZER et al.** *Genome Med*, 2013, vol. 5, 30
- **SNYDER et al.** *Cell*, 2016, vol. 164, 57-86
- **VAN LOO et al.** *PNAS*, 2010, vol. 107, 16910-16915
- **DESPIERRE et al.** *Gynecol Oncol*, 2014, vol. 135, 415-422
- **ROBIN et al.** *BMC Bioinformatics*, 2011, vol. 12, 77
- **HARRELL**. Regression modeling strategies. Springer International Publishing, 2015
- **TANGE**. *login USENIX Mag*, 2011, vol. 36, 42-47
- **WANG et al.** *Clin Cancer Res*, 2013, vol. 23, 2223-2231
- **BHATLA et al.** *Int J Gynecol Obstet*, 2019, vol. 145, 129-135
- **CURTIN**. *Gynecol Oncol*, 1994, vol. 55, S42-S46
- **VAN CALSTER et al.** *BMJ*, 2014, vol. 349, 1-14
- **JIANG et al.** *PNAS*, 2015, vol. 112, E1317-E1325
- **MOULIERE et al.** *BioRxiv*, 2017
- **CORCES et al.** *Science*, 2018, vol. 362, 1898
- **SCOTT-BROWNE et al.** *Immunity*, 2016, vol. 45, 1327-1340
- **VANDERSTICHELE et al.** *Clin Cancer Res*, 2014, vol. 23, 2223-2231
- **BAERT et al.** *Gynecol Oncol Reports*, 2017, vol. 19, 57-58
- **MELNIKOV et al.** *J Mol Diagnostics*, 2009, vol. 11, 60-65
- **LÖVKVIST et al.** *Nucleic Acids Res*, 2016, vol. 44, 5123-5132
- **TANGE**. *USENIX Mag*, 2011, vol. 36, 42-47